# EUROPEAN PATENT APPLICATION

(11) **EP 3 974 525 A2**
(43) Date of publication of application: **30.03.2022**
(21) Application number: 20810376.2
(22) Date of filing: 22.05.2020
(51) Int. Cl.: C12N 9/22, C12N 9/78, C12N 9/24, C12N 15/52, C12N 15/85, C12Q 1/6827

(54) **SINGLE BASE SUBSTITUTION PROTEIN, AND COMPOSITION COMPRISING SAME**

(30) Priority: 22.05.2019 US 201962851372 P; 10.09.2019 US 201962898094 P
(71) Applicant: Toolgen Incorporated, Seoul 08501 (KR)
(72) Inventor: BAE, Sung Min, Seoul 08208 (KR); KIM, Young Hoon, Seoul 04127 (KR); LEE, Jeong Joon, Seongnam-si Gyeonggi-do 13532 (KR)
(74) Representative: dompatent von Kreisler Selting Werner - Partnerschaft von Patent- und Rechtsanwälten mbB
(86) International application number: PCT/KR2020/006731
(87) International publication number: WO 2020/235974

(57) **Abstract**

The present application relates to: a single base substitution protein; a composition comprising same; and a use thereof.

## Description

### [Technical Field]

The present application relates to technology of substituting cytosine (C) or adenine (A) with any base using a protein for single base substitution using a CRISPR enzyme, a deaminase and a DNA glycosylase.

### [Background Art]

A CRISPR enzyme-linked deaminase has been used to treat genetic disorders by editing a genetic locus where a point mutation has occurred, or induce a targeted single nucleotide polymorphism (SNP) in a gene of a human or eukaryotic cell.

The currently-reported CRISPR enzyme-linked deaminases include:
1) base editors (BEs) including (i) catalytically-deficient Cas9 (dCas9) derived from S. *pyogenes* or D10A Cas9 nickase (nCas9), and (ii) rAPOBEC1, which is a cytidine deaminase of a rat;
2) target-AID including (i) dCas9 or nCas9 and (ii) PmCDA1, which is an activation-induced cytidine deaminase (AID) ortholog of a sea lamprey, or human AID;
3) CRISPR-X including MS2 RNA hairpin-linked sgRNAs and dCas9 to recruit a hyperactive AID variant fused to an MS2-binding protein; and
4) zinc-finger proteins or transcription activator-like effectors (TALEs) that are fused to a cytidine deaminase.

A CRISPR enzyme-linked deaminase used along with a conventional DNA glycosylase may substitute cytosine (C) with only thymine (T), or adenine (A) with only guanine (G) in nucleotides. In one example, a material in which Cas9, cytidine deaminase, and uracil DNA glycosylase inhibitor (UGI) are fused is used to substitute cytosine (C) with thymine (T). The materials serve to substitute uracil (U) with thymine (T) using a mechanism of inducing uracil (U) to not be removed by a DNA glycosylase. Likewise, recently, it has been reported that adenine (A) can be substituted with only guanine (G) using adenosine deaminase instead of cytidine deaminase.

Therefore, the inventors of the present application intend to substitute cytosine (C) or adenine (A) with any base by developing a protein for single base substitution using a CRISPR enzyme, a deaminase and a DNA glycosylase. The development of this technology can be used for identification of a genetic disease caused by a mutation, and drug development and therapeutic agents by analyzing a nucleic acid sequence affecting disease susceptibility by SNPs or having resistance to a drug, and will be more effective in developing drugs in the future and improving a therapeutic effect.

### [Disclosure]

### [Technical Problem]

Conventional CRISPR enzyme-linked deaminases have limitations in that cytosine (C) or adenine (A) can be converted to a specific base (A or G). Due to these limitations, the scope of research such as identification of genetic diseases caused by mutations, disease susceptibility by SNPs, and development of related therapeutic agents is limited.

Therefore, the development of means capable of substituting cytidine (C) or adenine (A) with any base (A, T, C, G or U), not a specific base, is urgently needed.

The present application is directed to providing a protein for single base substitution or a complex for single base substitution, or a composition for single base substitution, which includes the same, and a use thereof.

The present application is directed to providing a nucleic acid sequence encoding the protein for single base substitution or a vector including the same.

The present application is directed to providing a method for single base substitution.

The present application is directed to providing various uses for the protein for single base substitution or the complex for single base substitution, or the composition for single base substitution, which includes the same.

### [Technical Solution]

The present application provides that a fusion protein for single base substitution or a nucleic acid encoding thereof.

The present application provides that a vector comprising a nucleic acid encoding the fusion protein for single base substitution.

The present application provides that a complex for single base substitution.

The present application provides that a composition for single base substitution.

The present application provides that a method for single base substitution.

The present application provides that a use of epitope screening, drug resistance gene or protein screening, drug sensitization screening, or viral resistance gene or protein screening using the fusion protein for single base substitution, the complex for single base substitution, the composition for single base substitution of the present application.

The present application provides a fusion protein for single base substitution or a nucleic acid encoding the same, which includes (a) a CRISPR enzyme or a variant thereof, (b) a deaminase, and (c) a DNA glycosylase or a variant thereof. Wherein, the fusion protein for single base substitution induces substitution of cytidine or adenine included in one or more nucleotides in a target nucleic acid sequence with any base.

The present application provides a fusion protein for single base substitution or a nucleic acid encoding the same, which includes any one component of (i) N terminus-[CRISPR enzyme]-[deaminase]-[DNA glycosylase]-C terminus; (ii) N terminus-[CRISPR enzyme]-[DNA glycosylase]-[deaminase]-C terminus; (iii) N terminus-[deaminase]-[CRISPR enzyme]-[DNA glycosylase]-C terminus; (iv) N terminus-[deaminase]-[DNA glycosylase]-[CRISPR enzyme]-C terminus; (v) N terminus-[DNA glycosylase]-[CRISPR enzyme]-[deaminase]-C terminus; and (vi) N terminus-[DNA glycosylase]-[deaminase]-[CRISPR enzyme]-C terminus.

The present application provides a complex for single base substitution, which includes (a) a CRISPR enzyme or a variant thereof; (b) a deaminase; (c) a DNA glycosylase; and (d) two or more binding domains. Wherein, the fusion protein for single base substitution induces substitution of cytidine or adenine included in one or more nucleotides in a target nucleic acid sequence with any base.

According to the present application, in the complex for single base substitution, each of the CRISPR enzyme, the deaminase and the DNA glycosylase are linked to one or more binding domains. Wherein, the CRISPR enzyme, the deaminase and the DNA glycosylase form the complex by interaction between the binding domains.

According to the present application, in the complex for single base substitution, any one selected from the CRISPR enzyme, the deaminase, and the DNA glycosylase is linked to a first binding domain and a second binding domain. Wherein, the first binding domain and a binding domain of another component are an interactive pair, and the second binding domain and binding domain of the other binding domain are an interactive pair. Wherein, the complex is formed by the pairs.

According to the present application, the complex for single base substitution includes (i) a first fusion protein including two components selected from the CRISPR enzyme, the deaminase, and the DNA glycosylase, and a first binding domain, and (ii) a second fusion protein including the other component which is not selected above and a second binding domain. Wherein, the first binding domain and the second binding domain are an interactive pair, and the complex is formed by the pair.

According to the present application, the complex for single base substitution includes (i) a first fusion protein including the deaminase, the DNA glycosylase, and a first binding domain, and (ii) a second fusion protein including the CRISPR enzyme and a second binding domain.

Wherein, the first binding domain is a single chain variable fragment (scFv), and the second fusion protein further includes at least one or more binding domains, in which the further included binding domain is a GCN4 peptide. Wherein, two or more of the first fusion proteins may form the complex by interaction with any one of the GCN4 peptides.

The present application may provide a composition for single base substitution, which includes (a) a guide RNA or a nucleic acid encoding the same, and (b) i) the fusion protein for single base substitution of claim 1 or a nucleic acid encoding the same or ii) the complex for single base substitution of claim 13. Wherein, the guide RNA complementarily binds to a target nucleic acid sequence, wherein the target nucleic acid sequence binding to the guide RNA is 15 to 25 bp. Wherein, the fusion protein for single base substitution or the complex for single base substitution induces substitution of one or more cytosine or adenine present in a target region including the target nucleic acid sequence with any base.

According to the present application, the composition for single base substitution may include one or more vectors.

The present application may provide a method for single base substitution, which includes bringing (i) and (ii) into contact with the target region including the target nucleic acid sequence *in vitro* or *ex vivo,* wherein the (i) is a guide RNA and the (ii) is the fusion protein for single base substitution of claim 1 or the complex for single base substitution of claim 12. Wherein, the guide RNA complementarily binds to the target nucleic acid sequence, wherein the target nucleic acid sequence binding to the guide RNA is 15 to 25 bp., and wherein the fusion protein for single base substitution or the complex for single base substitution induces substitution of one or more cytosines or adenines present in a target region including the target nucleic acid sequence with any base.

Wherein, the deaminase is a cytidine deaminase, and the DNA glycosylase is Uracil-DNA glycosylase or a variant thereof. Wherein the fusion protein for single base substitution induces substitution of C (cytosine) included in one or more nucleotides in the target nucleic acid sequence with any base(s).

Wherein, the cytidine deaminase may be APOBEC, an activation-induced cytidine deaminase (AID) or a variant thereof.

Wherein, the deaminase may be an adenosine deaminase, and the DNA glycosylase may be alkyladenine DNA glycosylase or a variant thereof. Wherein, the fusion protein for single base substitution may induce substitution of adenine(s) included in one or more nucleotides in a target nucleic acid sequence with any base(s).

Wherein, the adenosine deaminase may be TadA, Tad2p, ADA, ADA1, ADA2, ADAR2, ADAT2, ADAT3 or a variant thereof.

Wherein, the binding domain may be any one selected from FRB domain, FKBP dimerization domain, intein, ERT domain, VPR domain, GCN4 peptide, single chain variable fragment (scFv), or any one of a domain forming a heterodimer.

Wherein, in the complex for single base substitution, the pair may be any one selected from the following (i) to (vi): (i) FRB and FKBP dimerization domains; (ii) a first intein and a second intein; (iii) ERT and VPR domains; (iv) a GCN4 peptide and a single chain variable fragment (scFv); and (v) first and second domains for forming a heterodimer.

### [Advantageous Effects]

The present application provides that a protein for single base substitution and/or a nucleic acid encoding thereof.

The present application provides that a composition for single base substitution comprising a protein for single base substitution and/or a nucleic acid encoding thereof.

The present application provides various uses of a protein for single base substitution or a composition for single base substitution comprising the same.

### [Description of Drawings]

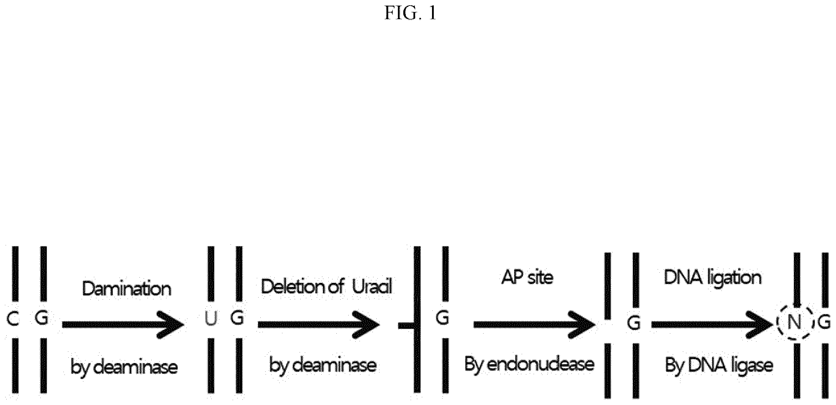

FIG. 1 is a diagram illustrating a process of substituting cytosine (C) with N (A, T or G) in a target nucleic acid region using a protein for single base substitution.
FIG. 2 is a diagram illustrating a process of substituting adenine (A) with N (C, T or G) in a target nucleic acid region using a protein for single base substitution.
FIG. 3 is a diagram illustrating various designs of fusion proteins for single base substitution inducing substitution of cytosine with any base.
FIG. 4 is a diagram illustrating various designs of fusion proteins for single base substitution inducing substitution of adenine with any base.
FIG. 5(a) is nCas9 having 10 identical GCN4 peptides fused to a carboxyl end; and FIGS. 5(b) and 5(c) are various designs of complexes (scFv-Apobec-UNG and scfv-UNG-Apobec) in which a single chain variable fragment (scFv) is fused to Apobec and UNG, respectively.

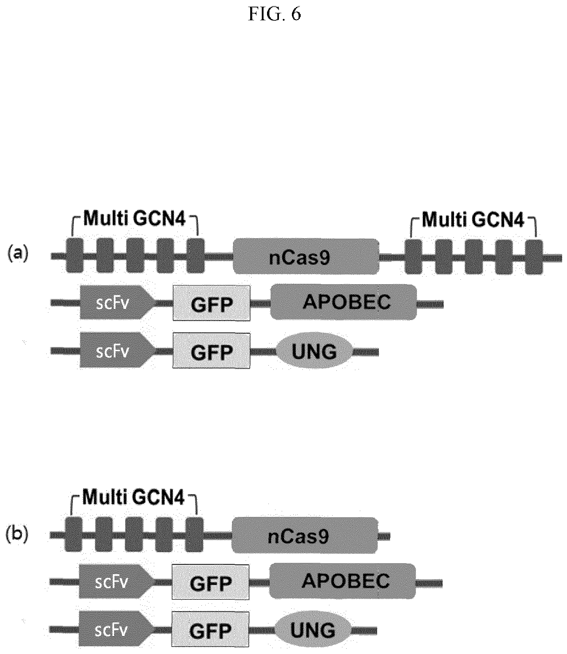

FIG. 6(a) is a diagram illustrating the design of a complex in which 5 identical GCN4 peptides are fused to each of the N terminus and the C terminus of nCas9, one scFv is fused to APOBEC, and the other scFv is fused to UNG. FIG. 6(b) is a diagram illustrating the design of a complex in which 5 identical GCN4 peptides are fused to the C-terminus of nCas9, one scFv is fused to APOBEC, and the other scFv is fused to UNG.
FIG. 7(a) shows the designs of BE3 WT and bpNLS BE3; and FIG. 7(b) is a graph showing single base substitution efficiency using BE3 WT and bpNLS BE3 in HEK cells.
FIG. 8 is a graph showing a substitution rate of C to G, C to T, or C to A using BE3 WT, ncas-delta UGI, UNG-ncas and ncas-UNG in Hela cells. ncas-delta UGI is a protein in which uracil DNA-glycosylase inhibitor (UGI) is removed from BE3 WT.
FIG. 9 shows a nucleic acid sequence (SEQ ID No: 1) in which base substitution is induced in a target region. In addition, FIG. 9 also shows base substitution rates of cytosine at position 15 and cytosine at position 16 in the nucleic acid sequence (SEQ ID NO: 1) using BE3 WT, bpNLS BE3, ncas-delta UGI, UNG-ncas and ncas-UNG in hela cells.
FIG. 10 is a graph confirming cytosine substitution in a hEMX1 target nucleic acid sequence targeted to GX20 sgRNA in HEK cells.
FIG. 11 is a set of graphs showing single base substitution efficiency using UNG-ncas and ncas-UNG in HEK cells. The left graph shows the C-to-N substitution rate in a hEMX1 target nucleic acid sequence targeted by GX20 sgRNA. The right graph shows the C-to-G or C-to-A substitution rate at positions 13C, 15C, 16C and 17C in a hEMX1 target nucleic acid sequence targeted by GX20 sgRNA.
FIG. 12 is a set of graphs confirming whether Nureki nCas9 have C-to-N base substitution at NG PAM in HEK cells.
FIG. 13 is a graph confirming whether C-to-N base substitution occurs using the complex for single base substitution of FIG. 5.

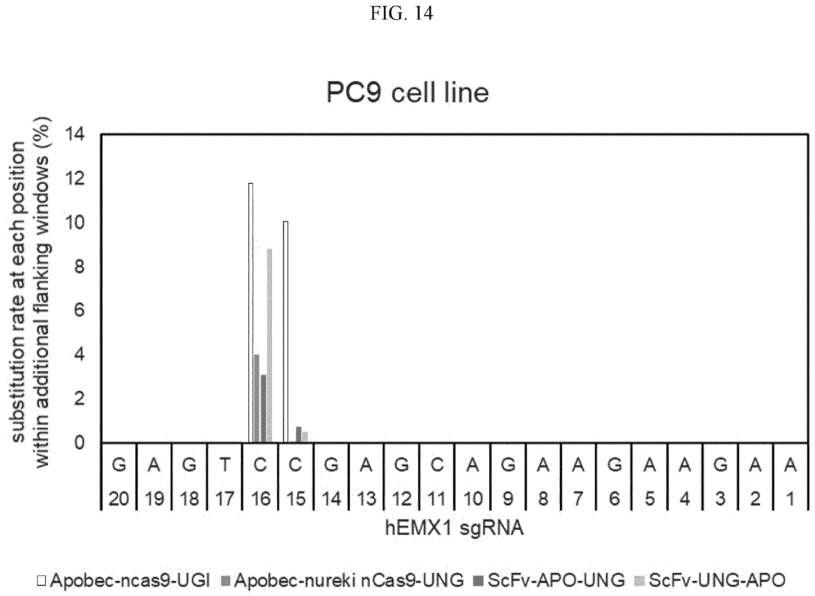

FIG. 14 is a graph identifying C at which substitution occurs in a nucleic acid sequence targeted to hEMX1 GX19 sgRNA in PC9 cells using the complex for single base substitution of FIG. 5.

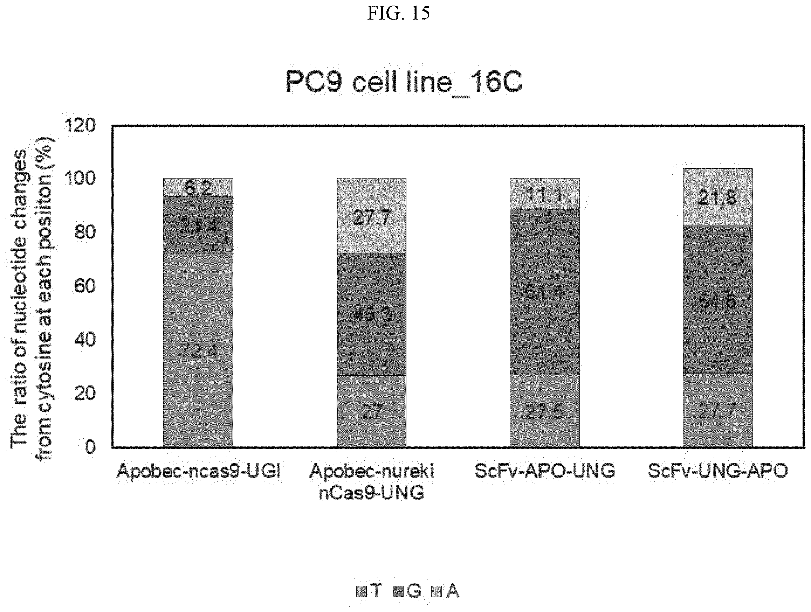

FIG. 15 is a graph showing a C-to-G, C-to-T or C-to-A substitution rate at position 16C in a sequence targeted to hEMX1 sgRNA in PC9 cells using the complex for single base substitution of FIG. 5.

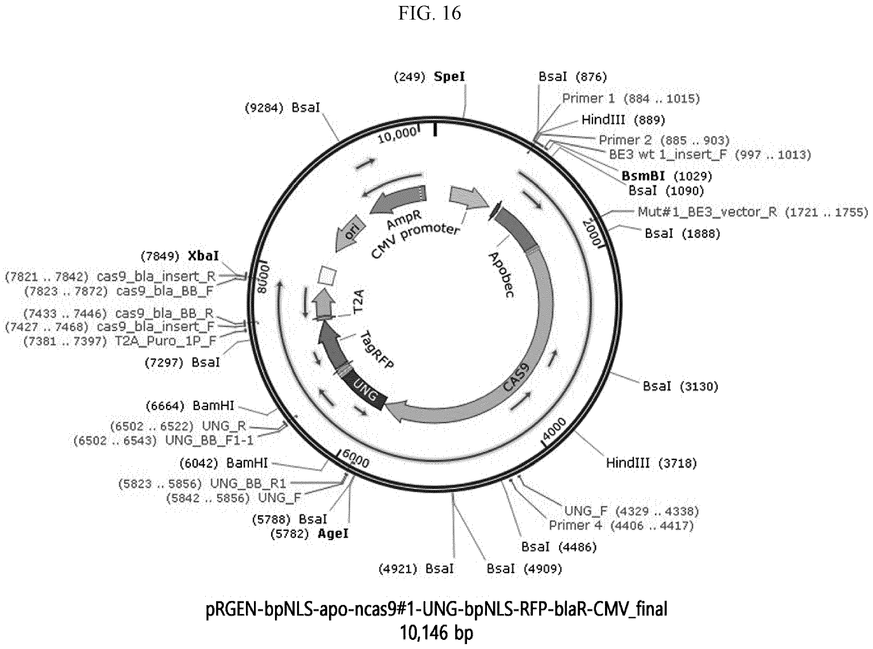

FIG. 16 shows the design of a plasmid encoding a protein for single base substitution using nCas9. The encoded protein for single base substitution is illustrated in 1) of FIG. 3(a).

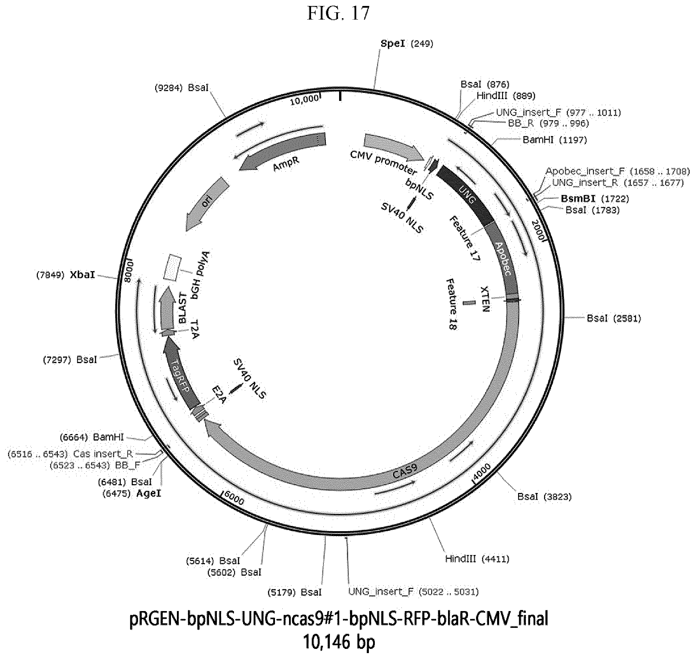

FIG. 17 shows the design of a plasmid of a CRISPR protein for single base substitution using Nureki nCas9. The encoded protein for single base substitution is illustrated in 2) of FIG. 3(c).

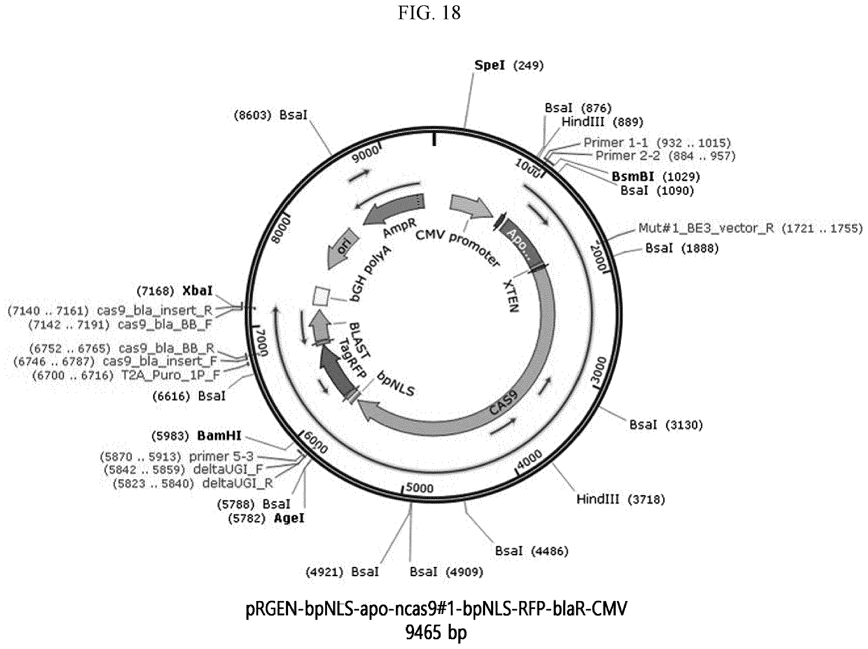

FIG. 18 shows the design of a plasmid encoding a protein for single base substitution using nCas9. The encoded protein for single base substitution is illustrated in 3) of FIG. 3(a).
FIG. 19 shows the design of a plasmid encoding a protein for single base substitution illustrated in FIG. 4(a).
FIG. 20 shows the design of a plasmid encoding the protein for single base substitution illustrated in FIG. 4(b).
FIG. 21 is a diagram illustrating the structures of fused base substitution domains including a single chain variable fragment (scFv).

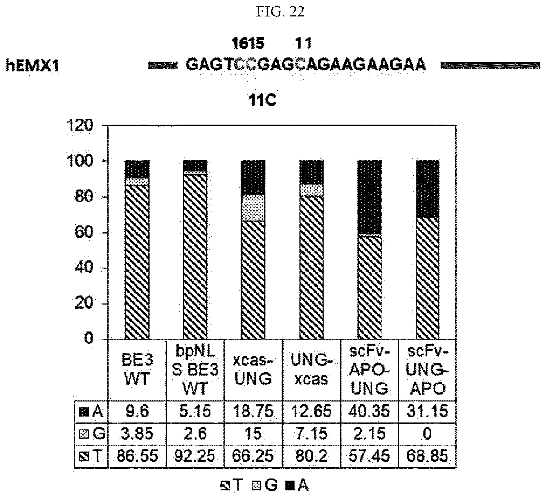

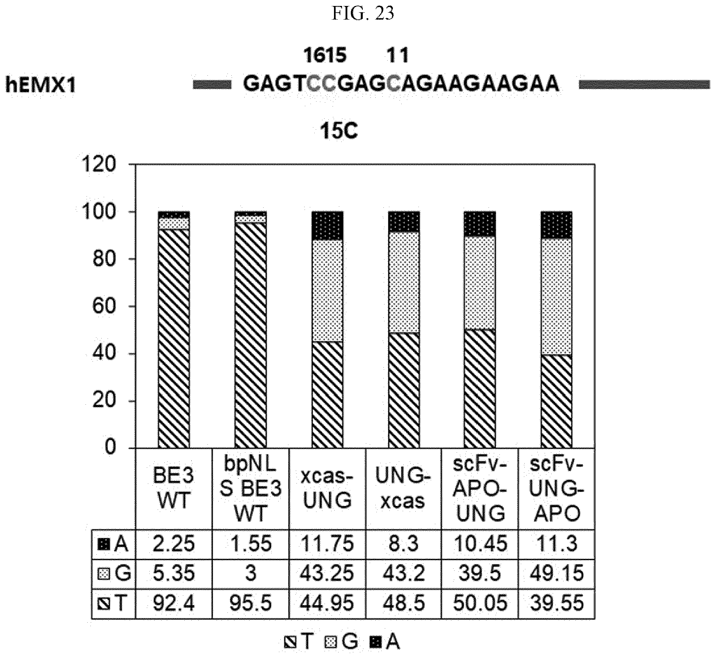

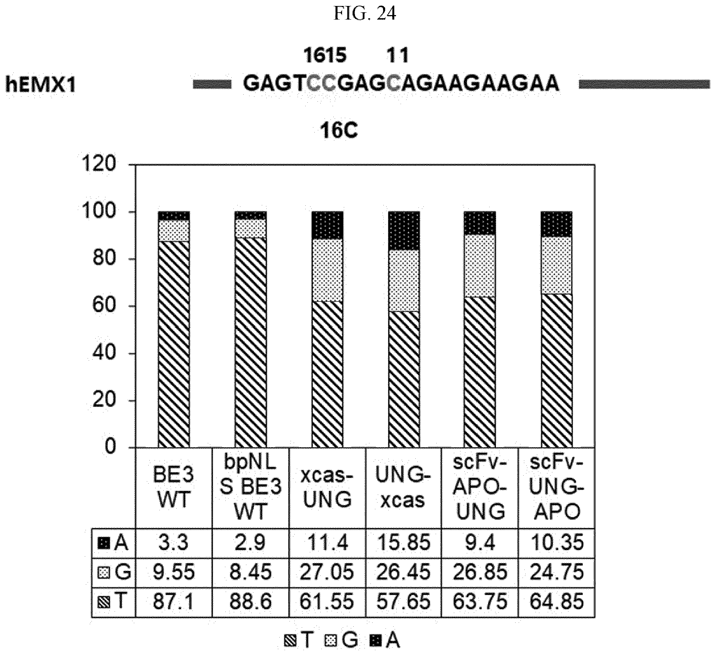

FIGS. 22 to 24 are graphs showing single base substitution efficiencies using complexes for single base substitution in HEK cells, in which FIG. 22 shows a C-to-G, C-to-A or C-to-G substitution rate at position 11C in the hEMX1 target nucleic acid sequence (SEQ ID NO: 1) targeted by GX20 sgRNA, FIG. 23 shows a C-to-G, C-to-A or C-to-G substitution rate at position 15C in the hEMX1 target nucleic acid sequence (SEQ ID NO: 1) targeted by GX20 sgRNA, and FIG. 24 shows a C-to-G, C-to-A or C-to-G substitution rate at position 16C in the hEMX1 target nucleic acid sequence (SEQ ID NO: 1) targeted by GX20 sgRNA.
FIG. 25 shows three (SEQ ID NOs: 2, 3 and 19) of sgRNAs (SEQ ID NOs: 2 to 20) shown in Extended Data Figure 2 in the article titled "Base Editing of A, T to G, C in Genomic DNA without DNA Cleavage" published in the science journal 'Nature'.
FIG. 26 is a set of graphs showing A to N base substitution rates in HEK293T cells using sgRNA1 (SEQ ID NO: 2) selected in FIG. 25.
FIG. 27 is a set of graphs showing A to N base substitution rates in HEK293T cells using sgRNA2 (SEQ ID NO: 3) selected in FIG. 25.
FIG. 28 is a set of graphs showing A to N base substitution rates in HEK293T cells using sgRNA3 (SEQ ID NO: 19) selected in FIG. 25.
FIG. 29 is a graph showing C to N base substitution rates in PC9 cells using sgRNA1 (SEQ ID NO: 21) and sgRNA2 (SEQ ID NO: 22) each of which can complimentarily bind to one region of an EGFR gene.
FIG. 30 is a set of graphs showing C-to- A, C-to-T or C-to-G base substitution rates in PC9 cells using sgRNA1 (SEQ ID NO: 21) and sgRNA2 (SEQ ID NO: 22) which can complimentarily bind to one region of an EGFR gene.
FIG. 31 is the result of analyzing cells which survived by culturing in a medium supplemented with osimertinib after random base substitution of cytosines.

### [Modes of the Invention]

Unless defined otherwise, all technical and scientific terms used in the specification have the same meanings as commonly understood by one of ordinary skill in the art to which the present invention belongs. Although methods and materials similar or equivalent to those described in the specification can be used in the practice or experiments of the present invention, suitable methods and materials are described below. All publications, patent applications, patents and other references mentioned in the present specification are incorporated by reference in their entirety. In addition, the materials, methods and examples are merely illustrative and not intended to be limiting.

The present application provides a protein for single base substitution (single base substitution protein), which includes (a) a CRISPR enzyme or a variant thereof, (b) a deaminase, and (c) a DNA glycosylase or a variant thereof.

The present application provides a composition for single base substitution including the protein for single base substitution and (d) guide RNA.

Here, the protein for single base substitution may simultaneously act with guide RNA to induce substitution of cytosine (C) or adenine (A) included in one or more nucleotides **in a target nucleic acid sequence** with any **nitrogenous base.**

A combination of (a) the CRISPR enzyme and (d) the guide RNA of the protein for single base substitution provided according to the present application may specifically direct the protein for single base substitution to a target region including a target nucleic acid sequence.

Here, the combination of (b) the deaminase and (c) the DNA glycosylase of the protein for single base substitution may induce substitution of base(s) of one or more nucleotides in a target region with another base.

### Nitrogenous base

The "nitrogenous base" used herein refers to a purine or pyrimidine base, which is one constituent of a nucleotide, or a nucleobase.

The nitrogenous base used herein may be simply called a base, and the base may refer to adenine (A), thymine (T), uracil (U), hypozanthine (H), guanine (G) or cytosine (C).

The abbreviation of the bases in the present application, such as A, T, C, G, U, or H, refers to the nitrogenous base when it is used in the context related to base substitution. , Besides, they refer to a nucleic acid or nucleotide which is generally used in the art, when it is used in the context related to a general nucleic acid, nucleotide sequence, or SEQ ID NO set in the specification.

In one example, the "substituting adenine (A) with guanine (G)" may mean that a nitrogenous base in nucleotides of the same position or the same type on a nucleic acid sequence is substituted from A to G.

In one example, the "substituting adenine (A) with thymine (T)" may mean that a nitrogenous base in nucleotides of the same position or the same type on a nucleic acid sequence is substituted from A to T.

In one example, the "substituting adenine (A) with cytosine (C)" may mean that a nitrogenous base in nucleotides of the same position or the same type on a nucleic acid sequence is substituted from A to C.

In one example, the "substituting cytosine (C) with guanine (G)" may mean that a nitrogenous base in nucleotides of the same position or the same type on a nucleic acid sequence is substituted from C to G.

In one example, the "substituting cytosine (C) with thymine (T)" may mean that a nitrogenous base in nucleotides of the same position or the same type on a nucleic acid sequence is substituted from C to T.

In one example, the "substituting C with A" may mean that a nitrogenous base in nucleotides of the same position or the same type on a nucleic acid sequence is substituted from C to A.

In one example, the "3'-ATGCAAA-5'" does not refer to a nitrogenous base, but represents a nucleic acid sequence or a nucleotide sequence commonly used in the art.

### Base substitution or base modification

The "base substitution" used herein means substitution of a base of a nucleotide in a target gene with another base. More specifically, a base of a nucleotide in a target region is substituted with another base.

In one example, base substitution may mean that adenine (A), guanine (G), cytosine (C), thymine (T), hypozanthine or uracil (U) is changed to another base.

In one exemplary embodiment, the base substitution may mean that adenine is substituted with cytosine, thymine, uracil, hypozanthine, or guanine.

In one exemplary embodiment, the base substitution may mean that cytosine is substituted with adenine, thymine, uracil, hypozanthine, or guanine.

In one exemplary embodiment, the base substitution may mean that guanine is substituted with cytosine, thymine, uracil, hypozanthine or adenine.

In one exemplary embodiment, the base substitution may mean that thymine is substituted with adenine, cytosine, uracil, hypozanthine, or guanine.

In one exemplary embodiment, the base substitution may mean that uracil is substituted with cytosine, thymine, adenine, hypozanthine, or guanine.

In one exemplary embodiment, the base substitution may mean that hypozanthine is substituted with adenine, thymine, uracil, or guanine.

However, the present invention is not limited thereto.

The "base substitution" used herein may be a concept including "base modification". Here, modification may mean changing to another base by modification of a base structure, and base substitution may mean changing of a base type.

In one example, the base modification is changing of the chemical structure of adenine (A), guanine (G), cytosine (C), thymine (T), hypozanthine or uracil (U).

In one exemplary embodiment, the base modification may be that adenine changes to hypoxanthine by deamination of adenine.

In one exemplary embodiment, the base modification may be that hypoxanthine changes to guanine.

In one exemplary embodiment, the base modification may be that cytosine changes to uracil by deamination of cytosine.

In one exemplary embodiment, the base modification may be that uracil changes to thymine.

However, the present invention is not limited thereto.

### Target nucleic acid sequence - nucleic acid sequence complementarily binding to guide RNA

A target nucleic acid sequence means a nucleotide sequence which may or can complementarily bind to guide RNA which is a constituent of a composition for single base substitution.

In one example, when intracellular double-stranded DNA is subjected to single base substitution, the intracellular double-stranded DNA consists of a first DNA strand and a second DNA strand. Here, any one of the first DNA strand of the double-stranded DNA and the second DNA strand complementary to the first DNA strand may include a target nucleic acid sequence. The first or second DNA strand including the target nucleic acid sequence may bind to the guide RNA. Here, the nucleic acid sequence in the first DNA strand or the second DNA strand, binding to the guide RNA, corresponds to the target nucleic acid sequence.

In one example, when intracellular double-stranded RNA is subjected to single base substitution, the intracellular double-stranded RNA consists of a first RNA strand and a second RNA strand. Any one of the first RNA strand of the double-stranded RNA and the second RNA strand complementary to the first RNA strand may include a target nucleic acid sequence. The first or second RNA strand including the target nucleic acid sequence may bind to the guide RNA. Here, the nucleic acid sequence of the first RNA strand or the second RNA strand, binding to the guide RNA, corresponds to the target nucleic acid sequence.

In one example, when intracellular double-stranded DNA or RNA is subjected to single base substitution, the single strand DNA or RNA may include a target nucleic acid sequence. That is, the single strand DNA or RNA may bind to guide RNA, and here, the nucleic acid sequence binding to the guide RNA corresponds to the target nucleic acid sequence.

In one example, the target nucleic acid sequence may be a nucleotide sequence of 10, 11, 12, 13, 14, 15, 16, 17, 18, 19, 20, 21, 22, 23, 24, 25, 26, 27, 28, 29 or 30 bp or more.

### Target region - region including base-substituted nucleotide

A target region is a region including a nucleotide in which base substitution is induced by a protein for single base substitution.

A target region is a region including a target nucleic acid sequence to which guide RNA binds. Here, the target nucleic acid sequence may include a nucleotide in which base substitution is induced by a protein for single base substitution.

A target region includes a nucleic acid sequence in a second DNA strand complementarily binding to a target nucleic acid sequence in a first DNA strand complementarily binding to guide RNA. Here, the nucleic acid sequence in the second DNA strand may include a nucleotide in which base substitution is induced by a protein for single base substitution.

In one example, a strand including the target nucleic acid sequence in double-stranded DNA or RNA may be referred to as a first strand, and a strand not including the target nucleic acid sequence may be referred to as a second strand. Here, a target region may include the target nucleic acid sequence complementarily binding to guide RNA in the first strand and the nucleic acid sequence in the second strand complementarily binding to the target nucleic acid sequence.

In one example, a strand including the target nucleic acid sequence in double-stranded DNA or RNA may be referred to as a second strand, and a strand not including the target nucleic acid sequence may be referred to as a first strand. Here, the target region may include the target nucleic acid sequence complementarily binding to guide RNA in the second strand and the nucleic acid sequence in the first strand complementarily binding to the target nucleic acid sequence.

A protein for single base substitution may induce base substitution of one or more nucleotides in the target region.

In one example, when guide RNA complementarily binds to a target nucleic acid sequence included in a first DNA strand of a double-stranded DNA, a protein for single base substitution may substitute (i) one or more nucleotide bases in the target nucleic acid sequence, or (ii) one or more nucleotide bases in a nucleic acid sequence complementarily binding to the target nucleic acid sequence in a second strand of the double-stranded DNA.

In one example, when guide RNA complementarily binds to a target nucleic acid sequence included in a first RNA strand of a double-stranded RNA, a protein for single base substitution may substitute (i) one or more nucleotide bases in the target nucleic acid sequence or (ii) one or more nucleotide bases in a nucleic acid sequence complementarily binding to the target nucleic acid sequence in a second strand of the double-stranded RNA.

In one exemplary embodiment, cytosines of one or more nucleotides in the target nucleic acid region may be substituted with guanine, thymine, uracil, hypoxanthine or adenine.

In one exemplary embodiment, adenines of one or more nucleotides in the target nucleic acid sequence may be substituted with guanine, thymine, uracil, hypoxanthine or cytosine.

The target gene used herein refers to a gene including a target region and a target nucleic acid sequence. In addition, the target gene in the present specification refers to a gene in which the cytosine(s) of one or more nucleotides in the target region is/are substituted with any base(s) by a protein for single base substitution.

### Technical feature - substitution with any base

A protein for single base substitution provided in the present application includes (i) a deaminase and (ii) a DNA glycosylase as essential constituents.

A combination of a first component of the protein for single base substitution, which is a deaminase, and a second component of the protein for single base substitution, which is a DNA glycosylase, may induce substitution of a base of a nucleotide in a nucleic acid sequence with any base.

Here, base substitution by the deaminase and the DNA glycosylase may be caused by two steps as follows: sequentially or simultaneously performing (i) base deamination and/or (ii) cleavage or repair by a DNA glycosylase.

### First process: deamination of base

Deamination means a biochemical reaction involving the cleavage of an amino group. In one example, in the case of DNA, deamination may refer to change of an amino group of a base, which is one constituent of a nucleotide, to a hydroxy or ketone group.

In one exemplary embodiment, a deaminase may be cytidine deaminase. The cytidine deaminase may provide uracil by deamination of cytosine. The cytidine deaminase may provide uracil by modification of cytosine.

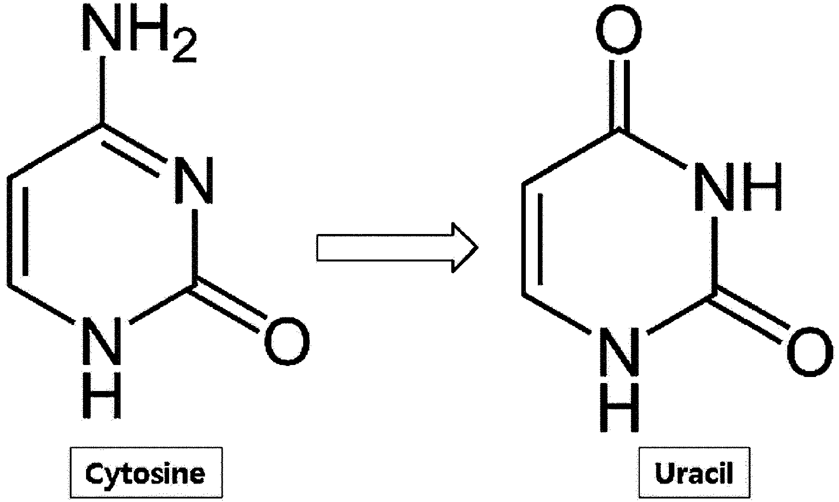

In one exemplary embodiment, the deaminase for a protein for single base substitution may be adenosine deaminase. The adenosine deaminase may provide hypoxanthine by deamination of adenine. The adenosine deaminase may provide hypozanthine(hypoxanthine) by modification of adenine.

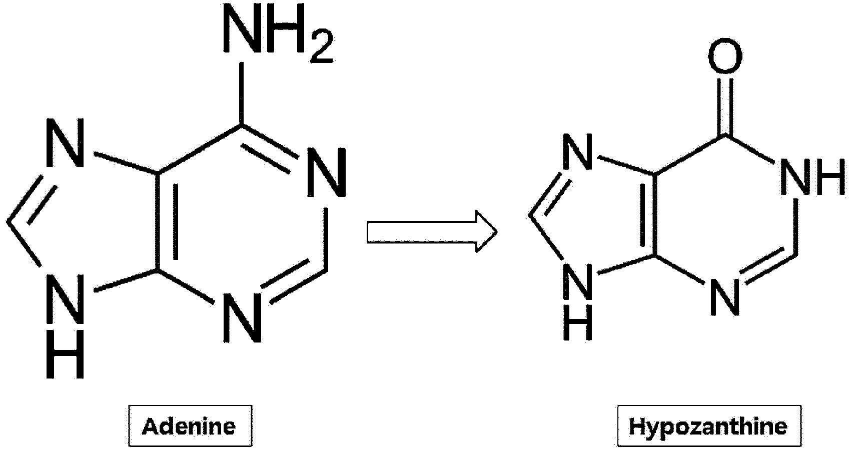

In one exemplary embodiment, the deaminase may be guanine deaminase. The guanine deaminase may provide xanthine by deamination of guanine. The guanine deaminase may provide xanthine by modification of guanine.

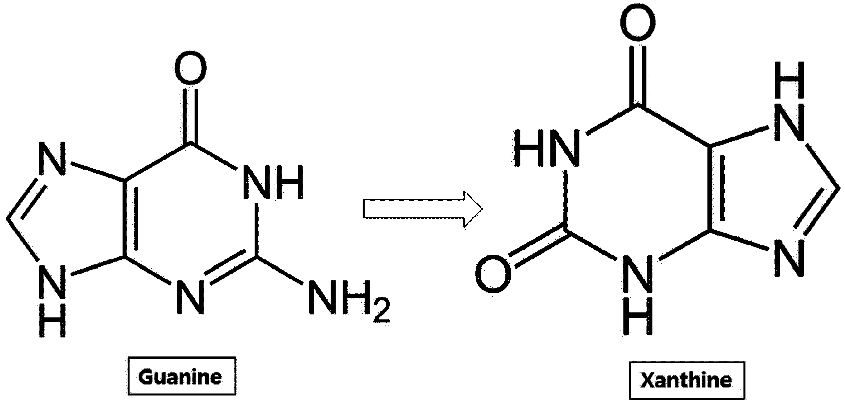

### Second process: DNA glycosylation

A DNA glycosylase is an enzyme involved in base excision repair (BER), and BER is a mechanism of removing and replacing a damaged base of DNA. The DNA glycosylase catalyzes the first step of the mechanism by hydrolyzing the N-glycoside linkage between a base and a deoxyribose of DNA. The DNA glycosylase removes a damaged nitrogenous base while leaving the sugar-phosphate backbone intact. As a result, an AP site, specifically an apurinic site or an apyrimidinic site, is made. Afterward, substitution with any base may be performed by an AP endonuclease, an end processing enzyme, a DNA polymerase, a flap endonuclease, and/or a DNA ligase.

In one exemplary embodiment, the DNA glycosylase may be uracil DNA glycosylase. The uracil DNA glycosylase hydrolyzes the N-glycoside linkage between uracil and deoxyribose in DNA. The uracil DNA glycosylase hydrolyzes the N-glycoside linkage between uracil and deoxyribose in a nucleotide including uracil. Here, the uracil-containing nucleotide may be provided by deamination using cytidine deaminase acting on a nucleotide including cytosine.

In one exemplary embodiment, the DNA glycosylase may be alkyladenine DNA glycosylase. The alkyladenine DNA glycosylase hydrolyzes the N-glycoside linkage between hypozanthine(hypoxanthine) and deoxyribose in DNA. The alkyladenine DNA glycosylase hydrolyzes the N-glycoside linkage between hypozanthine and deoxyribose in a nucleotide including hypozanthine. Here, the nucleotide including hypozanthine may be provided by deamination using adenosine deaminase acting on a nucleotide including adenine.

### Results of the first and second processes

One or more adenines or cytosines in a target region may be substituted with any base(s) using a protein for single base substitution provided in the present application.

In one example, a deaminase of the protein for single base substitution may be adenosine deaminase, and a DNA glycosylase of the protein for single base substitution may be alkyladenine-DNA glycosylase or a variant thereof. Here, the fusion protein for single base substitution (single base substitution fusion protein) may induce substitution of adenine(s) in one or more in nucleotides in a target nucleic acid sequence with any base(s) (guanine, thymine or cytosine).

In one exemplary embodiment, substitution of adenine(s) in one or more nucleotides in a target region with cytosine(s) may be induced by a protein for single base substitution including (a) CRISPR enzyme or variant thereof; (b) adenosine deaminase; and (c) alkyladenine DNA glycosylase.

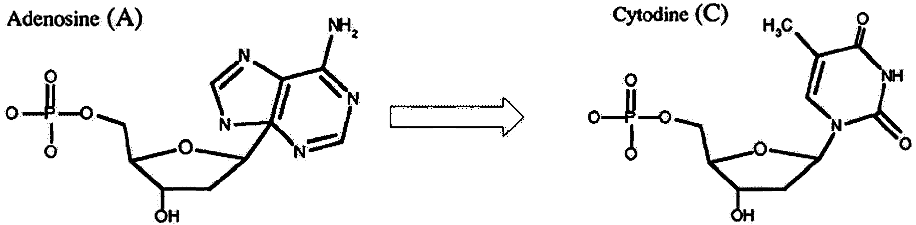

In one exemplary embodiment, substitution of adenine(s) in one or more nucleotides in a target region with thymine(s) may be induced by a protein for single base substitution including (a) CRISPR enzyme or variant thereof; (b) adenosine deaminase; and (c) alkyladenine DNA glycosylase.

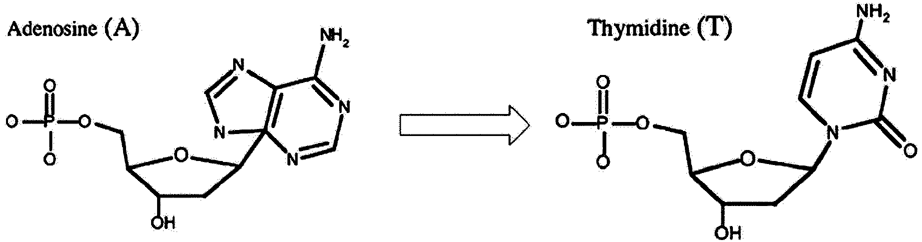

In one exemplary embodiment, substitution of adenine(s) in one or more nucleotide(s) in a target region with guanine(s) may be induced by a protein for single base substitution including (a) CRISPR enzyme or variant thereof; (b) adenosine deaminase; and (c) alkyladenine DNA glycosylase.

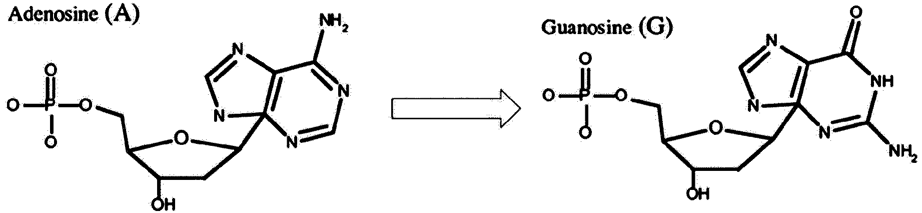

In one example, the deaminase of the protein for single base substitution may be cytidine deaminase, and the DNA glycosylase thereof may be uracil DNA glycosylase or variant thereof. Here, the fusion protein for single base substitution may induce substitution of cytosine(s) of one or more nucleotide(s) in target nucleic acid sequence with any base(s).

In one exemplary embodiment, substitution of cytosine(s) in one or more nucleotides in target region with adenine(s) may be induced by protein for single base substitution including (a) CRISPR enzyme or variant thereof; (b) cytidine deaminase; and (c) uracil DNA glycosylase.

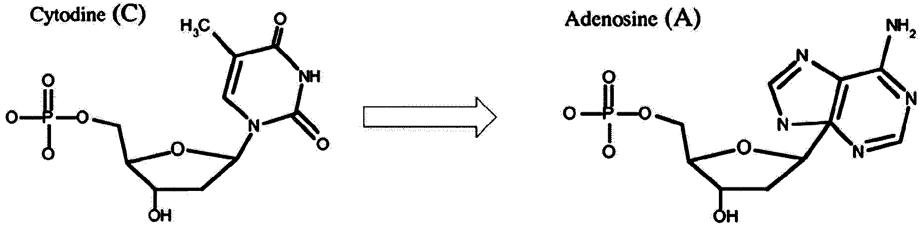

In one exemplary embodiment, substitution of cytosine(s) in one or more nucleotides in target region with thymine(s) may be induced by protein for single base substitution including (a) CRISPR enzyme or a variant thereof; (b) cytidine deaminase; and (c) uracil DNA glycosylase.

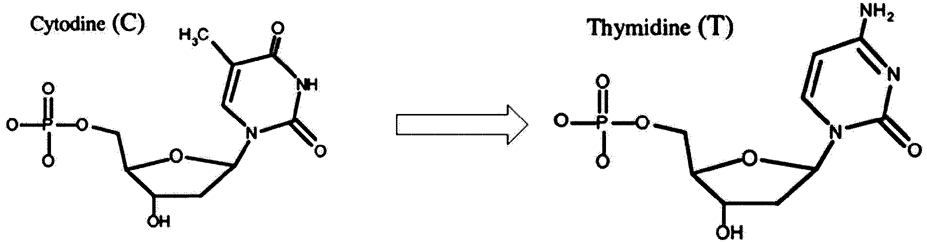

In one exemplary embodiment, substitution of cytosine(s) in one or more nucleotides in target region with guanine(s) may be induced by a protein for single base substitution including (a) CRISPR enzyme or variant thereof; (b) cytidine deaminase; and (c) uracil DNA glycosylase.

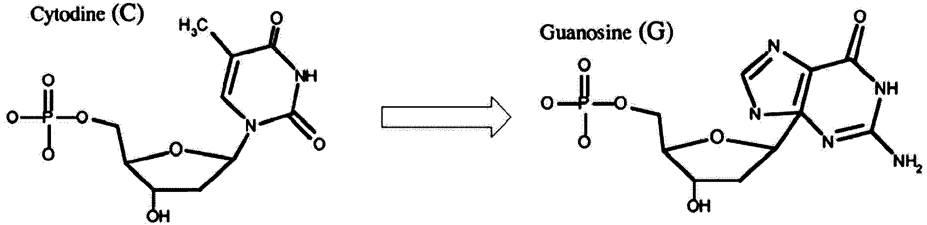

Hereinafter, the present invention will be described in detail.

### One aspect of the present invention disclosed in the specification is a protein for single base substitution.

A protein for single base substitution is a protein, polypeptide or peptide which is able to induce or generate single base substitution.

### Limitations of conventional base editor

A conventional base editor was used in the form of fusion, connection or linkage of a deaminase, a CRISPR enzyme and a DNA glycosylase inhibitor. As a representative example, using a base editor in which cytidine deaminase from a rat, such as rAPOBEC, nCas9 and uracil DNA glycosylase are linked, a cytosine base was substituted with thymine. In addition, adenine (A) was substituted with guanine (G) using adenosine deaminase, instead of cytidine deaminase.

It is significant that the conventional base editor can be used to treat a disease caused by a point mutation, for example, a genetic disorder by correcting a point mutation site in a gene. However, the conventional base editor has a limitation in that cytosine (C) is changed to only a specific base, thymine (T), or adenosine (A) is changed to only a specific base, guanine (G), by removing an amino group (-NH₂) or substituting an amino group with a keto group using a DNA glycosylase inhibitor.

### Utility of protein for single base substitution

The use of the conventional base editor has a limitation in that there is a low possibility of having a different type of amino acid expressed from a substituted base. Most diseases or disorders are not be caused by point mutations, but are likely to be generated by a structural or functional abnormality at the peptide, polypeptide or protein level, rather than the nucleotide level. After all, since the conventional base editor may only change adenine and cytosine into specific bases, the possibility of changing structure of peptide, polypeptide or protein is significantly reduced.

The limitations of the prior art can be overcome using the protein for single base substitution provided in the present specification. The protein for single base substitution provided in the present application has a novel combination consisting of (a) an editor protein, (b) a deaminase, and (c) a DNA glycosylase. That is, the protein for single base substitution provided in the present application has an advantage of substituting adenine (A), guanine (G), thymine (T) or cytosine (C) with any base (A, T, C, G, U or H).

In addition, the protein for single base substitution having the novel constituents and the novel combination thereof has an advantage of simultaneously substituting one or more bases present in a target nucleic acid sequence.

As a result, the protein for single base substitution provided in the present application may provide "mutations" in which various bases are randomly substituted. Peptides, polypeptides or proteins with various structures may be expressed from the mutated genes.

Due to the above technical effect, the protein for single base substitution provided in the present application may be used for epitope screening, drug resistance gene or protein screening, drug sensitization screening, and/or virus resistance gene or protein screening.

The protein for single base substitution provided in the present application may induce substitution of base(s) in the target region of the target gene with any base(s) by co-use with guide RNA.

### [First component of protein for single base substitution - deaminase]

A deaminase is an enzyme that is involved in removal of an amino group, and encompasses enzymes changing an amino group of compound to a hydroxyl or ketone group. There is an enzyme that catalyzes an amino group binding to each of cytosine, adenine, guanine, adenosine, cytidine, AMP and ADP, etc. and such an enzyme is generally contained in animal tissue.

The deaminase used herein may be referred to as a base substitution domain. Here, the base substitution domain refers to a peptide, polypeptide, domain, or protein which is involved in substitution of base(s) of one or more nucleotides in a target gene with any other base(s).

### The deaminase of the present application may be cytidine deaminase.

Here, the cytidine deaminase refers to any enzyme having the activity of removing an amino (-NH₂) group of cytosine, cytidine or deoxycytidine. The cytidine deaminase in the specification is used as a concept that includes cytosine deaminase. The cytidine deaminase in the specification may be used interchangeably with the cytosine deaminase.

The cytidine deaminase may change cytosine to uracil.

The cytidine deaminase may change cytidine to uridine.

The cytidine deaminase may change deoxycytidine to deoxyuridine.

The cytidine deaminase refers to any enzyme having the activity of converting cytosine (e.g., cytosine present in double-stranded DNA or RNA), which is a base present in a nucleotide, into uracil (C-to-U conversion or C-to-U editing), and converts cytosine located in a strand with a PAM sequence of the sequence of a target site (target nucleic acid sequence) into uracil.

In one example, the cytidine deaminase may be derived from prokaryotes such as *Escherichia coli;* or mammals such as primates such as humans and monkeys, and rodents such as rats and mice, but the present invention is not limited thereto. For example, the cytidine deaminase may be APOBEC ("apolipoprotein B mRNA editing enzyme, catalytic polypeptidelike") or one or more selected from enzymes belonging to the activation-induced cytidine deaminase (AID) family.

The cytidine deaminase may be APOBEC1, APOBEC2, APOBEC3B, APOBEC3C, APOBEC3D, APOBEC3F, APOBEC3G, APOBEC3H, APOBEC4, AID or CDA, but the present invention is not limited thereto.

For example, the cytidine deaminase may be human APOBEC 1, for example, a protein or polypeptide expressed by a gene or mRNA represented by NCBI Accession No. NM_005889, NM_001304566 orNM_001644. Alternatively, the cytidine deaminase may be human APOBEC 1, for example, a protein or polypeptide represented by NCBI Accession No. NP_001291495, NP_001635 or NP_005880.

For example, the cytidine deaminase may be mouse APOBEC 1, for example, a protein or polypeptide expressed by a gene or mRNA represented by NCBI Accession No. NM_001127863 or NM_112436. Alternatively, the cytidine deaminase may be mouse APOBEC1, for example, a protein or polypeptide represented by NCBI Accession No. NP_001127863 or NP_112436.

For example, the cytidine deaminase may be human AID, for example, a protein or polypeptide expressed by a gene or mRNA represented by NCBI Accession No. NM_020661 or NM_001330343. Alternatively, the cytidine deaminase may be human AID, for example, a protein or polypeptide expressed by a gene or mRNA represented by NCBI Accession No. NP_001317272 or NP_065712.

Hereinafter, examples of the cytidine deaminase are listed:
APOBEC1: a gene encoding human APOBEC1 (e.g., NCBI Accession No. NP_001291495, NP_001635, NP_005880), for example, an APOBEC1 gene represented by NCBI Accession No. NM_005889 or NM_001304566, NM_001644, or a gene encoding mouse APOBEC1 (e.g., NCBI Accession No. NP_001127863, NP_112436), for example, an APOBEC1 gene represented by NCBI Accession No. NM_001127863 or NM_112436.

APOBEC2: a gene encoding human APOBEC2 (e.g., NCBI Accession No. NP_006780), for example, an APOBEC2 gene represented by NCBI Accession No. NM_006789, or a gene encoding mouse APOBEC2 (e.g., NCBI Accession No. NP_033824), for example, an APOBEC2 gene represented by NCBI Accession No. NM_009694.

APOBEC3B: a gene encoding human APOBEC3B (e.g., NCBI Accession No. NP_001257340 or NP_004891), for example, an APOBEC3B gene represented by NCBI Accession No. NM_004900 or NM_001270411, or a gene encoding mouse APOBEC3B (e.g., NCBI Accession No. NP_001153887, NP_001333970 or NP_084531), for example, an APOBEC3B gene represented by NCBI Accession No. NM_001160415, NM_030255 or NM_001347041.

APOBE3C: a gene encoding human APOBEC3C (e.g., NCBI Accession No. NP_055323), for example, an APOBEC3C gene represented by NCBI Accession No. NM_014508.

APOBEC3D: a gene encoding human APOBEC3D (e.g., NCBI Accession No. NP_689639 or NP_0013570710), for example, an APOBEC3D gene represented by NCBI Accession No. NM_152426 or NM_001363781.

APOBEC3F: a gene encoding human APOBEC3F (e.g., NCBI Accession No. NP_001006667 or NP_660341), for example, an APOBEC3F gene represented by NCBI Accession No. NM_001006666 or NM_145298.

APOBEC3G: a gene encoding human APOBEC3G (e.g., NCBI Accession No. NP_068594, NP_001336365, NP_001336366 or NP_001336367), for example, an APOBEC3G gene represented by NCBI Accession No. NM_021822.

APOBEC3H: a gene encoding human APOBEC3H (e.g., NCBI Accession No. NP_001159474, NP_001159475, NP_001159476 or NP_861438), for example, an APOBEC3H gene represented by NCBI Accession No. NM_001166002, NM_001166003, NM_001166004 or NM_181773.

APOBEC4: a gene encoding human APOBEC4 (e.g., NCBI Accession No. NP_982279), for example, an APOBEC4 gene represented by NCBI Accession No. NM_203454, or a gene encoding mouse APOBEC4, for example, an APOBEC4 gene represented by NCBI Accession No. NM_001081197.

The cytidine deaminase may be expressed from an activation-induced cytidine deaminase (AID) gene. For example, the AID gene may be selected from the group consisting of the following genes, but the present invention is not limited thereto: a gene encoding a human AID gene (e.g., NP_001317272, NP_065712), for example, an AID gene represented by NCBI Accession No. NM_020661 or NM_001330343, or a gene encoding a mouse AID gene (e.g., NP_03377512), for example, an AID gene represented by NCBI Accession No. NM_009645.

The cytidine deaminase may be encoded from a CDA gene. For example, the CDA gene may be selected from the group consisting of the following genes, but the present invention is not limited thereto: a gene encoding human CDA (e.g., NCBI Accession No. NP_001776), for example, a CDA gene represented by NCBI Accession No. NM_001785, or a gene encoding mouse CDA (e.g., NCBI Accession No. NP_082452), for example, a CDA gene represented by NCBI Accession No. NM_028176.

The cytidine deaminase may be a cytidine deaminase variant.

The cytidine deaminase variant may be an enzyme which has higher cytidine deaminase activity than wild-type cytidine deaminase. The cytidine deaminase activity is understood to include the deamination of cytosine or one of analogs thereof.

For example, the cytidine deaminase variants may be enzymes in which one or more amino acid sequences in the cytidine deaminase are modified.

Wherein, the modification of the amino acid sequence may be any one selected from substitution, deletion and insertion.

### The deaminase of the present application may be adenosine deaminase.

The adenosine deaminase is any enzyme with the activity of removing an amino (-NH₂) group of adenine, adenosine or deoxyadenosine or substituting the amino group with a keto (=O) group. The adenosine deaminase in the specification is used as a concept that includes adenine deaminase. The adenosine deaminase in the specification is used as a concept that includes adenine deaminase.

The adenosine deaminase may change adenine to hypozanthine(hypoxanthine).

The adenosine deaminase may change adenosine to inosine.

The adenosine deaminase may change deoxyadenosine to deoxyinosine.

The adenosine deaminase may be derived from prokaryotes such as *Escherichia coli;* or mammals such as primates such as humans and monkeys, and rodents such as rats and mice, but the present invention is not limited thereto. For example, the adenosine deaminase may be tRNA-specific adenosine deaminase (TadA) or one or more selected from the enzymes belonging to the adenosine deaminase (ADA) family.

The adenosine deaminase may be TadA, Tad2p, ADA, ADA1, ADA2, ADAR2, ADAT2 or ADAT3, but the present invention is not limited thereto.

For example, the adenosine deaminase may be *Escherichia coli* TadA, for example, a protein or polypeptide expressed by a gene or mRNA represented by NCBI Accession No. NC_000913.3, etc. Alternatively, the adenosine deaminase may be *Escherichia coli* TadA, for example, a protein or polypeptide represented by NCBI Accession No. NP_417054.2, etc.

For example, the adenosine deaminase may be human ADA, for example, a protein or polypeptide expressed by a gene or mRNA represented by NCBI Accession No. NM_000022, NM_001322050 or NM_001322051, etc. Alternatively, the adenosine deaminase may be human ADA, for example, a protein or polypeptide represented by NCBI Accession No. NP_000013, NP_001308979 or NP_001308980, etc.

For example, the adenosine deaminase may be mouse ADA, for example, a protein or polypeptide expressed by a gene or mRNA represented by NCBI Accession No. NM_001272052 or NM_007398, etc. Alternatively, the adenosine deaminase may be mouse ADA, for example, a protein or polypeptide represented by NCBI Accession No. NP_001258981 or NP_031424, etc.

For example, the adenosine deaminase may be human ADAR2, for example, a protein or polypeptide expressed by a gene or mRNA represented by NCBI Accession No. NM_001033049, NM_001112, NM_001160230, NM_015833 or NM_015834, etc. Alternatively, the adenosine deaminase may be human ADAR2, for example, a protein or polypeptide represented by NCBI Accession No. NP_001103, NP_001153702, NP_001333616, NP_001333617 or NP_056648, etc.

For example, the adenosine deaminase may be mouse ADAR2, for example, a protein or polypeptide expressed by a gene or mRNA represented by NCBI Accession No. NM_001024837, NM_001024838, NM_001024839, NM_001024840 or NM_130895, etc. Alternatively, the adenosine deaminase may be mouse ADAR2, for example, a protein or polypeptide represented by NCBI Accession No. NP_001020008, NP_570965 or NP_001020009, etc.

For example, the adenosine deaminase may be human ADAT2, for example, a protein or polypeptide expressed by a gene or mRNA represented by NCBI Accession No. NM_182503.3 or NM_001286259.1, etc. Alternatively, the adenosine deaminase may be human ADAT2, for example, a protein or polypeptide represented by NCBI Accession No. NP_001273188.1 or NP_872309.2, etc.

The adenosine deaminase may be any one of adA variants, ADAR2 variants and ADAT2 variants, but the present invention is not limited thereto.

For example, the ADAR2 variant may be one or more selected from the group consisting of the following genes, but the present invention is not limited thereto. The gene may be a gene encoding human ADAR2, for example, a CDA gene represented by NCBI Accession No. NM_001282225, NM_001282226, NM_001282227, NM_001282228, NM_001282229, NM_017424 or NM_177405, etc.

The adenosine deaminase may be an adenosine deaminase variant.

The adenosine deaminase variant may be an enzyme with higher adenosine deaminase activity than wild-type adenosine deaminase.

For example, the adenosine deaminase variant may be an enzyme in which one or more amino acid sequences in the adenosine deaminase is changed.

The adenosine deaminase may be an adenosine deaminase variant.

The adenosine deaminase variant may be an enzyme with higher adenosine deaminase activity than wild-type adenosine deaminase. Wherein, the adenosine deaminase activity may include the removal of an amino (-NH₂) group of adenine, adenosine, deoxyadenosine or an analog thereof or substitution of the amino (-NH₂) group with a keto (=O) group, but the present invention is not limited thereto.

The adenosine deaminase variant may be an enzyme in which one or more amino acid sequences selected from amino acid sequences constituting wild-type adenosine deaminase are modified.

Wherein, the modification of the amino acid sequence may be any one selected from substitution, deletion and insertion of one or more amino acids.

The adenosine deaminase variant may be a TadA variant, a Tad2p variant, an ADA variant, an ADA1 variant, an ADA2 variant, an ADAR2 variant, an ADAT2 variant, or an ADAT3 variant, but the present invention is not limited thereto.

For example, the adenosine deaminase may be a TadA variant. For example, the TadA variant may be ABE0.1, ABE1.1, ABE1.2, ABE2.1, ABE2.9, ABE2.10, ABE3.1, ABE4.3, ABE5.1, ABE5.3, ABE6.3, ABE6.4, ABE7.4, ABE7.8, ABE7.9 or ABE7.10, and specific details about the TadA variants are described in detail in the article, titled "Base editing of A,T to C, G in genomic DNA without DNA cleavage"(Nicole M. Gaudelli et al., (2017) Nature, 551, 464-471), so the corresponding document can be referenced.

The adenosine deaminase may be fused adenosine deaminase.

The deaminase provided in the present application may be provided in a fused form in which, for example, one or more functional domains are linked to cytidine deaminase or adenosine deaminase.

Here, the deaminase and the functional domain may be linked or fused such that each function is expressed.

The functional domain may be a domain with methylase activity, demethylase activity, transcription activation activity, transcription repression activity, transcription release factor activity, histone modification activity, RNA cleavage activity or nucleic acid binding activity, or a tag or reporter gene for isolating and purifying a protein (including a peptide), but the present invention is not limited thereto.

The functional domain may be a tag or reporter gene for isolating and purifying a protein (including a peptide).

Here, the tag may include any one of a histidine (His) tag, a V5 tag, a FLAG tag, an influenza hemagglutinin (HA) tag, a Myc tag, a VSV-G tag and a thioredoxin (Trx) tag. Here, the reporter gene may include any one of autofluorescent proteins, for example, glutathione-S-transferase (GST), horseradish peroxidase (HRP), chloramphenicol acetyltransferase (CAT) beta-galactosidase, beta-glucuronidase, luciferase, green fluorescent protein (GFP), HcRed, DsRed, cyan fluorescent protein (CFP), yellow fluorescent protein (YFP) and blue fluorescent protein (BFP). However, the present invention is not limited thereto.

The functional domain may be a nuclear localization sequence or signal (NLS) or a nuclear export sequence or signal (NES).

Here, one or more of the NLS may be included at an amino end of the CRISPR enzyme or the vicinity thereof; a carboxy end of the CRISPR enzyme or the vicinity thereof; or a combination thereof. The NLS may be an NLS sequence derived from the following, but the present invention is not limited thereto: one or more of the NLS of the SV40 virus-large T-antigen having amino acid sequence PKKKRKV (SEQ ID NO: 23); the NLS from nucleoplasmin (e.g., nucleoplasmin bipartite NLS having the sequence KRPAATKKAGQAKKKK (SEQ ID NO: 24)); the c-myc NLS having the amino acid sequence PAAKRVKLD (SEQ ID NO: 25) or RQRRNELKRSP (SEQ ID NO: 26); the hRNPA1 M9 NLS having the sequence NQSSNFGPMKGGNFGGRSSGPYGGGGQYFAKPRNQGGY (SEQ ID NO: 27); the sequence RMRIZFKNKGKDTAELRRRRVEVSVELRKAKKDEQILKRRNV (SEQ ID NO: 28) of the IBB domain from importin-alpha; the sequences VSRKRPRP (SEQ ID NO: 29) and PPKKARED (SEQ ID NO: 30) of the myoma T protein; the sequence POPKKKPL (SEQ ID NO: 31) of human p53; the sequence SALIKKKKKMAP (SEQ ID NO: 32) of mouse c-abl IV; the sequences DRLRR (SEQ ID NO: 33) and PKQKKRK (SEQ ID NO: 34) of influenza virus NS1; the sequence RKLKKKIKKL (SEQ ID NO: 35) of the infectious virus delta antigen; the sequence REKKKFLKRR (SEQ ID NO: 36) of the mouse Mx1 protein; the sequence KRKGDEVDGVDEVAKKKSKK (SEQ ID NO: 37) of human poly(ADP-ribose) polymerase; and the sequence RKCLQAGMNLEARKTKK (SEQ ID NO: 38) of a receptor of a human steroid hormone, glucocorticoid.

The functional domain may be a binding domain capable of forming a complex with another domain, a peptide, a polypeptide or a protein.

The binding domain may be one of FRB and FKBP dimerization domains; inteins; one of ERT and VPR domains; one of a GCN4 peptide and a single chain variable fragment (scFv); or a domain forming a heterodimer.

The binding domain may be scFv. Wherein, the scFv may be paired with the GCN4 peptide, and may specifically bind or be linked to the GCN4.

In one example, a first fusion protein in which the scFv functional domain is linked to the adenosine deaminase may bind to a peptide, polypeptide, protein or second fusion protein, which includes a GCN4 peptide.

### [Second component of protein for single base substitution - DNA glycosylase]

The DNA glycosylase is an enzyme involved in base excision repair (BER), and BER is a mechanism of removing and replacing a damaged base of DNA. The DNA glycosylase catalyzes the first step of the mechanism by hydrolysis of the N-glycoside linkage between a base and deoxyribose in DNA. The DNA glycosylase removes a damaged nitrogenous base while leaving an intact sugar-phosphate backbone.

### The glycosylase of the present application may be uracil DNA glycosylase.

The uracil DNA glycosylase is an enzyme that acts to prevent mutations of DNA by removal of uracil (U) present in the DNA, and may be one or more selected from all enzymes acting to initiate a base-excision repair (BER) pathway by breaking the N-glycosidic bond of uracil.

The glycosylase may be uracil DNA glycosylase (UDG or UNG). The uracil DNA glycosylase (UNG) may be selected from the group consisting of the following genes, but the present invention is not limited thereto: genes encoding human UNG (e.g., NCBI Accession No. NP_003353 and NP_550433), for example, UNG genes represented by NCBI Accession No. NM_080911 and NM_003362, or genes encoding mouse UNG (e.g., NCBI Accession No. NP_001035781 and NP_035807), for example, UNG genes represented by NCBI Accession No. NM_001040691 and NM_011677 or genes encoding *Escherichia coli* UNG (e.g., NCBI Accession No. ADX49788.1, ACT28166.1, EFN36865.1, BAA10923.1, ACA76764.1, ACX38762.1, EFU59768.A, EFU53885.A, EFJ57281.1, EFU47398.1, EFK71412.1, EFJ92376.1, EFJ79936.1, EFO59084.1, EFK47562.1, KXH01728.1, ESE25979.1, ESD99489.1, ESD73882.1, and ESD69341.1).

The DNA glycosylase may be an uracil DNA glycosylase variant. The uracil DNA glycosylase variant may be an enzyme with higher DNA glycosylase activity than wild-type uracil DNA glycosylase.

For example, the uracil DNA glycosylase variant may be an enzyme in which one or more amino acid sequences of the wild-type uracil DNA glycosylase is(are) modified. Here, the modification of the amino acid sequence may be substitution, deletion, insertion of at least one or more amino acids, or a combination thereof.

The glycosylase may be fused uracil DNA glycosylase.

The glycosylase of the present application may be alkyladenine DNA glycosylase (AAG).

The alkyladenine DNA glycosylase is an enzyme that acts to prevent mutations of DNA by removal of an alkylated or deaminated base present in the DNA, and may be one or more selected from the all enzymes acting to initiate a base-excision repair (BER) pathway by catalyzing the hydrolysis of the N-glycosidic bond of an alkylated or deaminated base.

The DNA glycosylase may be alkyladenine DNA glycosylase (AAG) or a variant thereof.

For example, the alkyladenine DNA glycosylase (AAG) may be human AAG, for example, a protein or polypeptide expressed by a gene or mRNA represented by NCBI Accession No. NM_002434, NM_001015052 or NM_001015054, etc. Alternatively, the alkyladenine DNA glycosylase (AAG) may be human AAG, for example, a protein or polypeptide represented by NCBI Accession No. NP_001015052, NP_001015054 or NP_002425, etc.

For example, the alkyladenine DNA glycosylase (AAG) may be mouse AAG, for example, a protein or polypeptide expressed by a gene or mRNA represented by NCBI Accession No. NM_010822, etc. Alternatively, the alkyladenine DNA glycosylase (AAG) may be human AAG, for example, a protein or polypeptide represented by NCBI Accession No. NP_034952, etc.

The DNA glycosylase may be an alkyladenine DNA glycosylase variant. The alkyladenine DNA glycosylase variant may be an enzyme with higher DNA glycosylase activity than the wild-type alkyladenine DNA glycosylase.

For example, the alkyladenine DNA glycosylase variant may be an enzyme in which one or more amino acid sequences of the wild-type alkyl adenine DNA glycosylase are modified. Wherein, the modification of the amino acid sequence may be substitution, deletion, insertion of at least one amino acid or a combination thereof.

The glycosylase may be fused alkyladenine DNA glycosylase.

The present application may provide fused uracil DNA glycosylase or fused alkyladenine DNA glycosylase in which one or more functional domains are linked to uracil DNA glycosylase or alkyladenine DNA glycosylase. Wherein, the uracil DNA glycosylase or the alkyladenine DNA glycosylase may be linked or fused to each functional domain such that each function is expressed.

The functional domain may be a domain with methylase activity, demethylase activity, transcription activation activity, transcription repression activity, transcription release factor activity, histone modification activity, RNA cleavage activity or nucleic acid binding activity, or a tag or reporter gene for isolating or purifying a protein (including a peptide), but the present invention is not limited thereto.

Here, the functional domain may be a tag or reporter gene for isolating and purifying a protein (including a peptide).

Here, the tag may include any one of a histidine (His) tag, a V5 tag, a FLAG tag, an influenza hemagglutinin (HA) tag, a Myc tag, a VSV-G tag and a thioredoxin (Trx) tag. Here, the reporter gene may include any one of autofluorescent proteins, for example, glutathione-S-transferase (GST), horseradish peroxidase (HRP), chloramphenicol acetyltransferase (CAT) beta-galactosidase, beta-glucuronidase, luciferase, green fluorescent protein (GFP), HcRed, DsRed, cyan fluorescent protein (CFP), yellow fluorescent protein (YFP) and blue fluorescent protein (BFP). However, the present invention is not limited thereto.

The functional domain may be a nuclear localization sequence or signal (NLS) or a nuclear export sequence or signal (NES).

Here, one or more of the NLS may be included at an amino end of the CRISPR enzyme or the vicinity thereof; a carboxy end or the vicinity thereof; or a combination thereof. The NLS may be an NLS sequence derived from the following, but the present invention is not limited thereto: any one or more of the NLS of the SV40 virus-large T-antigen having amino acid sequence PKKKRKV (SEQ ID NO: 23); the NLS from nucleoplasmin (e.g., nucleoplasmin bipartite NLS having the sequence KRPAATKKAGQAKKKK (SEQ ID NO: 24)); the c-myc NLS having the amino acid sequence PAAKRVKLD (SEQ ID NO: 25) or RQRRNELKRSP (SEQ ID NO: 26); the hRNPA1 M9 NLS having the sequence NQSSNFGPMKGGNFGGRSSGPYGGGGQYFAKPRNQGGY (SEQ ID NO: 27); the sequence RMRIZFKNKGKDTAELRRRRVEVSVELRKAKKDEQILKRRNV (SEQ ID NO: 28) of the IBB domain from importin-alpha; the sequences VSRKRPRP (SEQ ID NO: 29) and PPKKARED (SEQ ID NO: 30) of the myoma T protein; the sequence POPKKKPL (SEQ ID NO: 31) of human p53; the sequence SALIKKKKKMAP (SEQ ID NO: 32) of mouse c-abl IV; the sequences DRLRR (SEQ ID NO: 33) and PKQKKRK (SEQ ID NO: 34) of influenza virus NS1; the sequence RKLKKKIKKL (SEQ ID NO: 35) of the infectious virus delta antigen; the sequence REKKKFLKRR (SEQ ID NO: 36) of the mouse Mx1 protein; the sequence KRKGDEVDGVDEVAKKKSKK (SEQ ID NO: 37) of human poly(ADP-ribose) polymerase; and the sequence RKCLQAGMNLEARKTKK (SEQ ID NO: 38) of a receptor of a human steroid hormone, glucocorticoid.

The functional domain may be a binding domain capable of forming a complex with another domain, peptide, polypeptide or protein.

The binding domain may be one of FRB and FKBP dimerization domains; inteins; one of ERT and VPR domains; one of a GCN4 peptide and a single chain variable fragment (scFv); or a domain forming a heterodimer.

The binding domain may be scFv. Wherein, the scFv may be paired with the GCN4 peptide, and may specifically bind or be linked to the GCN4.

In one example, a first fusion protein in which the scFv functional domain is linked to the uracil DNA glycosylase or the alkyladenine DNA glycosylase may bind to a peptide, polypeptide, protein or second fusion protein, which includes a GCN4 peptide.

### [Third component of protein for single base substitution- CRISPR enzyme]

The protein for single base substitution provided in the present application includes a CRISPR enzyme or a CRISPR system including the same. The CRISPR enzyme in the specification may be referred to as a CRISPR protein.

The CRISPR system is a system that can introduce artificial mutations by targeting a target nucleic acid sequence near a proto-spacer-adjacent motif (PAM) sequence on genomic DNA. Specifically, the guide RNA and Cas protein bind (or interact with) to each other to form a guide RNA-Cas protein complex, and a mutation, indel, may be induced on the genomic DNA by cleavage of a target DNA sequence.

For more detailed descriptions on the guide RNA, Cas protein, and guide RNA-Cas protein complex, Korean Patent Publication No. 10-2017-0126636 can be referenced.

The Cas protein is used in the specification as a concept that includes all of variants capable of acting as an activated endonuclease or Nickase in cooperation with guide RNA, in addition to a wild-type protein. The activated endonuclease or nickase may cleave a target nucleic acid sequence, and may be used to manipulate or modify the nucleic acid sequence. In addition, the inactivated variants may be used to regulate transcription or isolate targeted DNA.

The CRISPR protein in the present application may be Cas9 or Cpf1 derived various microorganisms such as *Streptococcus pyogenes, Streptococcus thermophilus,* Streptococcus sp., *Staphylococcus aureus, Campylobacter jejuni, Nocardiopsis dassonvillei, Streptomyces pristinaespiralis, Streptomyces viridochromogenes, Streptomyces viridochromogenes, Streptosporangium roseum, Streptosporangium roseum, AlicyclobacHlus acidocaldarius, Bacillus pseudomycoides, Bacillus selenitireducens, Exiguobacterium sibiricum, Lactobacillus delbrueckii, Lactobacillus salivarius, Microscilla marina, Burkholderiales bacterium, Polaromonas naphthalenivorans,* Polaromonas sp., *Crocosphaera watsonii,* Cyanothece sp., *Microcystis aeruginosa,* Synechococcus sp., *Acetohalobium arabaticum, Ammonifex degensii, Caldicelulosiruptor bescii, Candidatus Desulforudis, Clostridium botulinum, Clostridium difficile, Finegoldia magna, Natranaerobius thermophilus, Pelotomaculum thermopropionicum, Acidithiobacillus caldus, Acidithiobacillusferrooxidans, Allochromatium vinosum,* Marinobacter sp., *Nitrosococcus halophilus, Nitrosococcus watsoni, Pseudoalteromonas haloplanktis, Ktedonobacter racemifer, Methanohalobium evestigatum, Anabaena variabilis, Nodularia spumigena,* Nostoc sp., *Arthrospira maxima, Arthrospira platensis,* Arthrospira sp., Lyngbya sp., *Microcoleus chthonoplastes,* Oscillatoria sp., *Petrotoga mobilis, Thermosipho africanus* or *Acaryochloris marina.*

The CRISPR enzyme may be a fully active CRISPR enzyme.

In one embodiment, the fully active CRISPR enzyme variants may be Cas9 protein variants derived from SpCas9 *Streptococcus pyogenes.* Hereinafter, examples of the variants are listed:
The variants may be enzymes in which one or more amino acids of E108G, E217A, A262T, R324L, S409I, E480K, E543D, M694I, E1219V, E480K, E543D, E1219V, A262T, S409I, E480K, E543D, E1219V, A262T, S409I, E480K, E543D, M694I, E1219V, E108G, E217A, A262T, S409I, E480K, E543D, M694I, E1219V, A262T, R324L, S409I, E480K, E543D, M694I, E1219V, L111R, D1135V, G1218R, E1219F, A1322R, R1335V and T1337R are substituted. Wherein, the CRISPR enzyme variants may recognize different PAM sequences, expand a target nucleic acid sequence in the genome by shortening the length of the PAM sequence that is able to be recognized by the CRISPR enzyme, and improve nucleic acid approaching ability.

As a specific example, in the case of SpCas9, when SpCas9 is mutated such as L111R, D1135V, G1218R, E1219F, A1322R, R1335V and T1337R, the SpCas9 variants may operate by recognizing only "NG" of the PAM sequence (the originally recognized PAM sequence is "NGG") (N is one of A, T, C and G).

Wherein, the SpCas9 variants (L111R, D1135V, G1218R, E1219F, A1322R, R1335V and T1337R) can be used interchangeably with "Nureki Cas9" ("CRISPR-Cas9 nuclease with expanded targeting space" Masu et al., (2018) Science 361, 1259-1262).

The CRISPR enzyme may be a nickase.

For example, when the type II CRISPR enzyme is wild-type SpCas9, the nickase may be a SpCas9 variant in which the nuclease activity of a HNH domain is inactivated by mutation of histidine 840 in the amino acid sequence of the wild-type SpCas9 to alanine. Here, since the generated nickase, that is, a SpCas9 variant, has nuclease activity generated by an RuvC domain, a non-complementary strand of a target gene or nucleic acid, that is, a strand that does not complementarily bind to gRNA, may be cleaved.

In another example, when the type II CRISPR enzyme is wild-type CjCas9, the nickase may be a CjCas9 variant in which the nuclease activity of a HNH domain is inactivated by mutation of histidine 559 in the amino acid sequence of the wild-type CjCas9 to alanine. Here, since the generated nickase, that is, a CjCas9 variant has nuclease activity by an RuvC domain, a non-complementary strand of a target gene or nucleic acid, that is, a strand that does not complementarily bind to gRNA, may be cleaved.

In addition, the nickase may have nuclease activity by a HNH domain of the CRISPR enzyme. That is, the nickase may not include nuclease activity by an RuvC domain of the CRISPR enzyme, and therefore, the RuvC domain may be manipulated or modified.

In one example, when the CRISPR enzyme is a type II CRISPR enzyme, the nickase may be a type II CRISPR enzyme including the modified RuvC domain.

For example, when the type II CRISPR enzyme is wild-type SpCas9, the nickase may be a SpCas9 variant in which the nuclease activity of the RuvC domain is inactivated by mutation of aspartic acid 10 in the amino acid sequence of the wild-type SpCas9 to alanine. Here, since the generated nickase, that is, a SpCas9 variant has nuclease activity by a HNH domain, a complementary strand of a target gene or nucleic acid, that is, a strand that complementarily binds to gRNA, may be cleaved.

In still another example, when the type II CRISPR enzyme is wild-type CjCas9, the nickase may be a CjCas9 variant in which the nuclease activity of a RuvC domain is inactivated by mutation of aspartic acid 8 in the amino acid sequence of the wild-type CjCas9 to alanine. Here, since the generated nickase, that is, a CjCas9 variant has nuclease activity by a HNH domain, a complementary strand of a target gene or nucleic acid, that is, a strand that complementarily binds to gRNA, may be cleaved.

In one embodiment, the nickase may be a Nureki Cas9 variant in which the nuclease activity of a RuvC domain is inactivated by mutation of aspartic acid 10 in the amino acid sequence of Nureki Cas9 to alanine, which is Nureki Cas9 nickase (Nureki nCas9). Here, since the generated Nureki nCas9 has nuclease activity by a HNH domain, a complementary strand of a target gene or nucleic acid, that is, a strand that complementarily binds to gRNA, may be cleaved.

In another embodiment, the nickase may be a Nureki Cas9 variant in which the nuclease activity of a HNH domain is inactivated by mutation of histidine 840 in the amino acid sequence of Nureki Cas9 to alanine, which is Nureki Cas9 nickase (Nureki nCas9). Here, since the generated Nureki nCas9 has nuclease activity by the RuvC domain, a non-complementary strand of a target gene or nucleic acid, that is, a strand that does not complementarily bind to gRNA, may be cleaved.

The CRISPR enzyme may be an inactive CRISPR enzyme.

The "inactive" refers to a state in which the functions of a wild-type CRISPR enzyme is lost, that is, both of a first function of cleaving the first strand of a double-stranded DNA and a second function of cleaving the second strand of a double-stranded DNA are lost. The CRISPR enzyme in this state is called an inactive CRISPR enzyme.

The inactive CRISPR enzyme may have nuclease inactivation due to mutation of a domain having nuclease activity of the wild-type CRISPR enzyme.

The inactive CRISPR enzyme may have nuclease inactivity caused by mutations in the RuvC domain and the HNH domain. That is, the inactive CRISPR enzyme may not include nuclease activity by the RuvC domain and the HNH domain of the CRISPR enzyme, and to this end, the RuvC domain and the HNH domain may be manipulated or modified.

In one example, when the CRISPR enzyme is a type II CRISPR enzyme, the inactive CRISPR enzyme may be a type II CRISPR enzyme including modified RuvC and HNH domains.

For example, when the Type II CRISPR enzyme is wild-type SpCas9, the inactive CRISPR enzyme may be a SpCas9 variant in which the nuclease activities of the RuvC domain and the HNH domain are inactivated by mutation of both of aspartic acid 10 and histidine 840 in the amino acid sequence of the wild-type SpCas9 to alanines. Here, since the generated inactive CRISPR enzyme, that is, the SpCas9 variant, has inactive nuclease activity of the RuvC domain and the HNH domain, it cannot cleave both the double strand of the target gene or nucleic acid.

In another example, when the type II CRISPR enzyme is wild-type CjCas9, the inactive CRISPR enzyme may be a CjCas9 variant in which the nuclease activities of the RuvC domain and the HNH domain are inactivated by mutation of both of aspartic acid 8 and histidine 559 in the amino acid sequence of the wild-type CjCas9 to alanines. Here, since generated inactive CRISPR enzyme, that is, the CjCas9 variant, has inactive nuclease activity of the RuvC domain and the HNH domain, it cannot cleave both the double strand of the target gene or nucleic acid.

In addition, the present application may provide a CRISPR enzyme linked to a functional domain. Here, the CRISPR enzyme variant may have an additional function, in addition to the original function of the wild-type CRISPR enzyme.

The functional domain may be a domain with methylase activity, demethylase activity, transcription activation activity, transcription repression activity, transcription release factor activity, histone modification activity, RNA cleavage activity or nucleic acid binding activity, or a tag or reporter gene for isolating and purifying a protein (including a peptide), but the present invention is not limited thereto.

The functional domain may be a tag or reporter gene for isolating and purifying a protein (including a peptide).

Here, the tag may include any one of a histidine (His) tag, a V5 tag, a FLAG tag, an influenza hemagglutinin (HA) tag, a Myc tag, a VSV-G tag and a thioredoxin (Trx) tag. Here, the reporter gene may include any one of autofluorescent proteins, for example, glutathione-S-transferase (GST), horseradish peroxidase (HRP), chloramphenicol acetyltransferase (CAT) beta-galactosidase, beta-glucuronidase, luciferase, green fluorescent protein (GFP), HcRed, DsRed, cyan fluorescent protein (CFP), yellow fluorescent protein (YFP) and blue fluorescent protein (BFP). However, the present invention is not limited thereto.

The functional domain may be a nuclear localization sequence or signal (NLS) or a nuclear export sequence or signal (NES).

Here, one or more of the NLS may be included at an amino end of the CRISPR enzyme or the vicinity thereof; a carboxy end of the CRISPR enzyme or the vicinity thereof; or a combination thereof. The NLS may be an NLS sequence derived from the following, but the present invention is not limited thereto: one or more of the NLS of the SV40 virus-large T-antigen having amino acid sequence PKKKRKV (SEQ ID NO: 23); the NLS from nucleoplasmin (e.g., nucleoplasmin bipartite NLS having the sequence KRPAATKKAGQAKKKK (SEQ ID NO: 24)); the c-myc NLS having the amino acid sequence PAAKRVKLD (SEQ ID NO: 25) or RQRRNELKRSP (SEQ ID NO: 26); the hRNPA1 M9 NLS having the sequence NQSSNFGPMKGGNFGGRSSGPYGGGGQYFAKPRNQGGY (SEQ ID NO: 27); the sequence RMRIZFKNKGKDTAELRRRRVEVSVELRKAKKDEQILKRRNV (SEQ ID NO: 28) of the IBB domain from importin-alpha; the sequences VSRKRPRP (SEQ ID NO: 29) and PPKKARED (SEQ ID NO: 30) of the myoma T protein; the sequence POPKKKPL (SEQ ID NO: 31) of human p53; the sequence SALIKKKKKMAP (SEQ ID NO: 32) of mouse c-abl IV; the sequences DRLRR (SEQ ID NO: 33) and PKQKKRK (SEQ ID NO: 34) of the influenza virus NS1; the sequence RKLKKKIKKL (SEQ ID NO: 35) of the infectious virus delta antigen; the sequence REKKKFLKRR (SEQ ID NO: 36) of the mouse Mx1 protein; the sequence KRKGDEVDGVDEVAKKKSKK (SEQ ID NO: 37) of human poly(ADP-ribose) polymerase; and the sequence RKCLQAGMNLEARKTKK (SEQ ID NO: 38) of a receptor of a human steroid hormone, glucocorticoid.

The functional domain may be a binding domain capable of forming a complex with another domain, a peptide, a polypeptide or a protein.

The binding domain may be one of FRB and FKBP dimerization domains; inteins; one of ERT and VPR domains; one of a GCN4 peptide and a single chain variable fragment (scFv); or a domain forming a heterodimer.

The binding domain may be a GCN4 peptide. Here, the GCN4 peptide may be paired with scFv, and may specifically bind or be linked to scFv.

In one example, a first fusion protein in which a GCN4 peptide functional domain is linked to the CRISPR enzyme may bind to a peptide, polypeptide, protein or second fusion protein including scFv,

### [First aspect of protein for single base substitution - fusion protein for single base substitution or nucleic acid encoding the same]

One aspect of the protein for single base substitution disclosed in the specification is a fusion protein for single base substitution.

In one example, the fusion protein for single base substitution or a nucleic acid encoding the same may include:
(a) a CRISPR enzyme or a variant thereof;
(b) a deaminase; and
(c) a DNA glycosylase or a variant thereof.

Here, the fusion protein for adenine substitution may induce substitution of cytosine(s) or adenine(s) included in one or more nucleotides in a target nucleic acid sequence with any base.

In one exemplary embodiment, the fusion protein for single base substitution may includes a linking moiety which is interposed between one selected from (a), (b), and (c), and the other one selected from (a), (b), and (c).

In one exemplary embodiment, the fusion protein for single base substitution may have any one component of:
(i) N terminus-[CRISPR enzyme]-[deaminase]-[DNA glycosylase]-C terminus;
(ii) N terminus-[CRISPR enzyme]-[DNA glycosylase]-[deaminase]-C terminus;
(iii) N terminus-[deaminase]-[CRISPR enzyme]-[DNA glycosylase]-C terminus;
(iv) N terminus-[deaminase]-[DNA glycosylase]-[CRISPR enzyme]-C terminus;
(v) N terminus-[DNA glycosylase]-[CRISPR enzyme]-[deaminase]-C terminus; and
(vi) N terminus-[DNA glycosylase]-[deaminase]-[CRISPR enzyme]-C terminus.

In one exemplary embodiment, the CRISPR enzyme or a variant thereof may include any one or more selected from the group consisting of a *Streptococcus pyogenes-derived* Cas9 protein, a *Campylobacter jejuni-derived* Cas9 protein, a *Streptococcus thermophilus-*derived Cas9 protein, a *Streptococcus aureus-derived* Cas9 protein, a *Neisseria meningitidis-*derived Cas9 protein, and a Cpf1 protein.

In one exemplary embodiment, the CRISPR enzyme variant may be characterized in that any one or more of the RuvC domain and the HNH domain is/are inactivated.

In one exemplary embodiment, the CRISPR enzyme variant may be a nickase.

In one embodiment a fusion protein for adenine substitution may be provided.

The fusion protein for adenine substitution or nucleic acid encoding the same may include:
(a) a CRISPR enzyme or a variant thereof;
(b) adenosine deaminase; and
(c) alkyladenine DNA glycosylase or a variant thereof.

Wherein, the fusion protein for adenine substitution may induce substitution of adenine(s) included in one or more nucleotides in a target nucleic acid sequence with any base(s).

The protein for adenine base substitution may be composed in the order of N terminus-[CRISPR enzyme]-[adenosine deaminase]-[alkyladenine DNA glycosylase]-C terminus.

The protein for adenine base substitution may be composed in the order of N terminus-[alkyladenine DNA glycosylase]-[CRISPR enzyme]-[adenosine deaminase]-C terminus.

The protein for adenine base substitution may be composed in the order of N terminus-[alkyladenine DNA glycosylase]-[adenosine deaminase]-[CRISPR enzyme]-C terminus.

The protein for adenine base substitution may be composed in the order of N terminus-[adenosine deaminase]-[CRISPR enzyme]-[alkyladenine DNA glycosylase]-C terminus.

The protein for adenine base substitution may be composed in the order of N terminus-[CRISPR enzyme]-[alkyladenine DNA glycosylase]-[adenosine deaminase]-C terminus.

The protein for adenine base substitution may be composed in the order of N terminus-[adenosine deaminase]-[alkyladenine DNA glycosylase]-[CRISPR enzyme]-C terminus.

The protein for adenine base substitution may further include a linking domain.

In one example, the linking domain may be a domain which operably links the CRISPR enzyme and the adenosine deaminase, the adenosine deaminase and the alkyladenine DNA glycosylase, and/or the CRISPR enzyme and the alkyladenine DNA glycosylase, and may be a domain that links the CRISPR enzyme, the adenosine deaminase and the alkyladenine DNA glycosylase to activate each function.

In one example, the linking domain may be an amino acid, peptide or polypeptide which does not affect the functional activities and/or structures of the CRISPR enzyme, the adenosine deaminase and the alkyladenine DNA glycosylase.

In one example, the domain for adenine base substitution may be composed in the order of N terminus-[CRISPR enzyme]-[linking domain]-[adenosine deaminase]-[alkyladenine DNA glycosylase]-C terminus; N terminus-[CRISPR enzyme]-[adenosine deaminase]-[linking domain]-[alkyladenine DNA glycosylase]-C terminus; or N terminus-[CRISPR enzyme]-[linking domain]-[adenosine deaminase]-[linking domain]-[alkyladenine DNA glycosylase]-C terminus.

In one example, the protein for adenine base substitution may be composed in the order of N terminus-[alkyladenine DNA glycosylase]-[linking domain]-[CRISPR enzyme]-[adenosine deaminase]-C terminus; N terminus-[alkyladenine DNA glycosylase]-[CRISPR enzyme]-[linking domain]-[adenosine deaminase]-C terminus; or N terminus-[alkyladenine DNA glycosylase]-[linking domain]-[CRISPR enzyme]-[linking domain]-[adenosine deaminase]-C terminus.

In one example, the protein for adenine base substitution may be composed in the order of N terminus-[alkyladenine DNA glycosylase]-[linking domain]-[adenosine deaminase]-[CRISPR enzyme]-C terminus; N terminus-[alkyladenine DNA glycosylase]-[adenosine deaminase]-[linking domain]-[CRISPR enzyme]-C terminus; or N terminus-[alkyladenine DNA glycosylase]-[linking domain]-[adenosine deaminase]-[linking domain]-[CRISPR enzyme]-C terminus.

In one example, the protein for adenine base substitution may be composed in the order of N terminus-[adenosine deaminase]-[linking domain]-[CRISPR enzyme]-[alkyladenine DNA glycosylase]-C terminus; N terminus-[adenosine deaminase]-[CRISPR enzyme]-[linking domain]-[alkyladenine DNA glycosylase]-C terminus; or N terminus-[adenosine deaminase]-[linking domain]-[CRISPR enzyme]-[linking domain]-[alkyladenine DNA glycosylase]-C terminus.

In one example, the protein for adenine base substitution may be composed in the order of N terminus-[CRISPR enzyme]-[linking domain]-[alkyladenine DNA glycosylase]-[adenosine deaminase]-C terminus; N terminus-[CRISPR enzyme]-[alkyladenine DNA glycosylase]-[linking domain]-[adenosine deaminase]-C terminus; or N terminus-[CRISPR enzyme]-[linking domain]-[alkyladenine DNA glycosylase]-[linking domain]-[adenosine deaminase]-C terminus.

In one example, the protein for adenine base substitution may be composed in the order of N terminus-[adenosine deaminase]-[linking domain]-[alkyladenine DNA glycosylase]-[CRISPR enzyme]-C terminus; N terminus-[adenosine deaminase]-[alkyladenine DNA glycosylase]-[linking domain]-[CRISPR enzyme]-C terminus; or N terminus-[adenosine deaminase]-[linking domain]-[alkyladenine DNA glycosylase]-[linking domain]-[CRISPR enzyme]-C terminus.

In one embodiment, a fusion protein for cytosine substitution may be provided.

The fusion protein for cytosine substitution or nucleic acid encoding the same may include:
(a) a CRISPR enzyme or a variant thereof;
(b) cytidine deaminase; and
(c) uracil DNA glycosylase or a variant thereof.

Wherein, the fusion protein for single base substitution may induced substitution of cytosine(s) included in one or more nucleotides in a target nucleic acid sequence with any base(s).

The protein for cytosine base substitution may be composed in the order of N terminus-[CRISPR enzyme]-[cytidine deaminase]-[uracil DNA glycosylase]-C terminus.

The protein for cytosine base substitution may be composed in the order of N terminus-[uracil DNA glycosylase]-[CRISPR enzyme]-[cytidine deaminase]-C terminus.

The protein for cytosine base substitution may be composed in the order of N terminus-[uracil DNA glycosylase]-[cytidine deaminase]-[CRISPR enzyme]-C terminus.

The protein for cytosine base substitution may be composed in the order of N terminus-[cytidine deaminase]-[CRISPR enzyme]-[uracil DNA glycosylase]-C terminus.

The protein for cytosine base substitution may be composed in the order of N terminus-[CRISPR enzyme]-[uracil DNA glycosylase]-[cytidine deaminase]-C terminus.

The protein for cytosine base substitution may be composed in the order of N terminus-[cytidine deaminase]-[uracil DNA glycosylase]-[CRISPR enzyme]-C terminus.

The protein for cytosine base substitution may further include a linking domain.

In one example, the linking domain may be a domain which operably links the CRISPR enzyme and the cytidine deaminase; the cytidine deaminase and the uracil DNA glycosylase; and/or the CRISPR enzyme and the uracil DNA glycosylase, and may be a domain that links the CRISPR enzyme, the cytidine deaminase and the uracil DNA glycosylase to activate each function.

In one example, the linking domain may be an amino acid, peptide or polypeptide which does not affect the functional activities and/or structures of the CRISPR enzyme, the cytidine deaminase and the uracil DNA glycosylase.

In one example, the cytosine base substitution domain may be composed in the order of N terminus-[CRISPR enzyme]-[linking domain]-[cytidine deaminase]-[uracil DNA glycosylase]-C terminus; N terminus-[CRISPR enzyme]-[cytidine deaminase]-[linking domain]-[uracil DNA glycosylase]-C terminus; or N terminus-[CRISPR enzyme]-[linking domain]-[cytidine deaminase]-[linking domain]-[uracil DNA glycosylase]-C terminus.

In one example, the protein for cytosine base substitution may be composed of N terminus-[uracil DNA glycosylase]-[linking domain]-[CRISPR enzyme]-[cytidine deaminase]-C terminus; N terminus-[uracil DNA glycosylase]-[CRISPR enzyme]-[linking domain]-[cytidine deaminase]-C terminus; or N terminus-[uracil DNA glycosylase]-[linking domain]-[CRISPR enzyme]-[linking domain]-[cytidine deaminase]-C terminus.

The protein for cytosine base substitution may be composed in the order of N terminus-[uracil DNA glycosylase]-[linking domain]-[cytidine deaminase]-[CRISPR enzyme]-C terminus; N terminus-[uracil DNA glycosylase]-[cytidine deaminase]-[linking domain]-[CRISPR enzyme]-C terminus; or N terminus-[uracil DNA glycosylase]-[linking domain]-[cytidine deaminase]-[linking domain]-[CRISPR enzyme]-C terminus.

The protein for cytosine base substitution may be composed in the order of N terminus-[cytidine deaminase]-[linking domain]-[CRISPR enzyme]-[uracil DNA glycosylase]-C terminus; N terminus-[cytidine deaminase]-[CRISPR enzyme]-[linking domain]-[uracil DNA glycosylase]-C terminus; or N terminus-[cytidine deaminase]-[linking domain]-[CRISPR enzyme]-[linking domain]-[uracil DNA glycosylase]-C terminus.

The protein for cytosine base substitution may be composed in the order of N terminus-[CRISPR enzyme]-[linking domain]-[uracil DNA glycosylase]-[cytidine deaminase]-C terminus; N terminus-[CRISPR enzyme]-[uracil DNA glycosylase]-[linking domain]-[cytidine deaminase]-C terminus; or N terminus-[CRISPR enzyme]-[linking domain]-[uracil DNA glycosylase]-[linking domain]-[cytidine deaminase]-C terminus.

The cytosine base modification protein may be composed in the order of N terminus-[cytidine deaminase]-[linking domain]-[uracil DNA glycosylase]-[CRISPR enzyme]-C terminus; N terminus-[cytidine deaminase]-[uracil DNA glycosylase]-[linking domain]-[CRISPR enzyme]-C terminus; or N terminus-[cytidine deaminase]-[linking domain]-[uracil DNA glycosylase]-[linking domain]-[CRISPR enzyme]-C terminus.

### [Second aspect of protein for single base substitution - complex for single base substitution]

One aspect of the protein for single base substitution disclosed in the specification is a complex for single base substitution (single base substitution complex).

In one example, the complex for single base substitution may include:
(a) a CRISPR enzyme or a variant thereof;
(b) a deaminase;
(c) a DNA glycosylase; and
(d) two or more binding domains.

Wherein, the fusion protein for single base substitution may induce substitution of cytosine(s) or adenine(s) included in one or more nucleotides in a target nucleic acid sequence with any base(s).

In one example, in the complex for single base substitution, the CRISPR enzyme may be linked with two or more binding domains.

Here, any one of the two or more binding domains linked to the CRISPR enzyme may be paired with the binding domain linked to (b) the deaminase, and the other one thereof may be paired with the binding domain linked to (c) the DNA glycosylase. Here, due to the binding between the pairs, the components (a) CRISPR enzyme, (b) deaminase and (c) DNA glycosylase form a complex to provide the complex for single base substitution.

In one exemplary embodiment, the CRISPR enzyme linked to two or more of the binding domains may have a configuration of [binding domain (functional domain)]ₙ-CRISPR enzyme (n may be an integer of 2 or more).

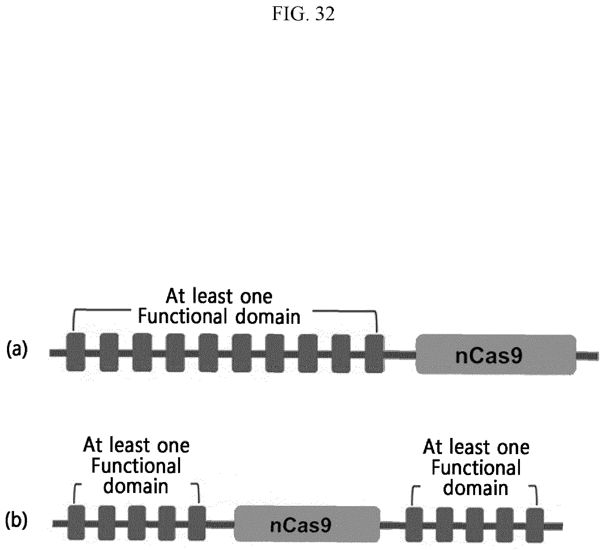

For example, the CRISPR enzyme may be shown in FIG. 32(a).

Here, the GCN4 may be an example of a binding domain linked to the CRISPR enzyme, and a different type of binding domain may be linked thereto. However, the present invention is not limited thereto.

Here, the CRISPR enzyme may be linked to 1, 2, 3, 4, 5, 6, 7, 8, 9, 10 or more binding domains.

In another example, the CRISPR enzyme may be shown in FIG. 32(b).

Here, the GCN4 may be one example of a binding domain linked to the CRISPR enzyme, and a different type of binding domain may be linked thereto. However, the present invention is not limited thereto.

Here, the CRISPR enzyme may be linked to 1, 2, 3, 4, 5, 6, 7, 8, 9, 10 or more binding domains at the C and N termini.

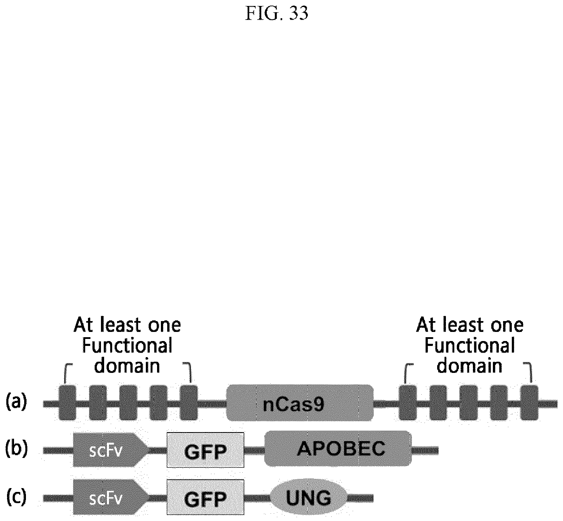

In one exemplary embodiment, the complex for single base substitution provided in the present application may be provided by
specific binding of the binding domains in the constituents (a), (b) and (c) of FIG. 33.

Here, a binding domain GCN4 of (a), a binding domain scFv of (b), and a binding domain scFv of (c) are merely examples and the present invention is not limited thereto. The APOBEC may be replaced with adenosine deaminase, and the UNG may be replaced with alkyladenine DNA glycosylase.

Wherein, a plurality of (b) and/or a plurality of (c) may bind to one (a).

Wherein, the "plurality" means an integer of 2, 3, 4, 5, 6, 7, 8, 9, 10 or more.

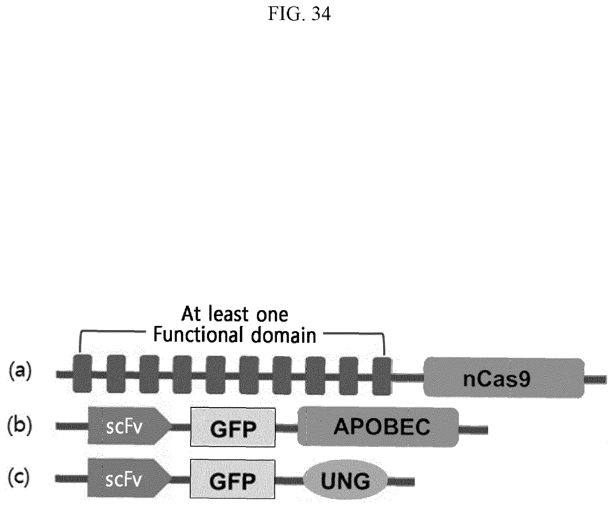

In one exemplary embodiment, the complex for single base substitution provided in the present application may be provided by
specific binding of binding domains in the constituents (a), (b) and (c) of FIG. 34.

Here, a binding domain GCN4 of (a), a binding domain scFv of (b), and a binding domain scFv of (c) are merely examples and the present invention is not limited thereto. The APOBEC may be replaced with adenosine deaminase, and the UNG may be replaced with alkyladenine DNA glycosylase.

Here, a plurality of (b) and/or a plurality of (c) may bind to one (a).

Here, the "plurality" means an integer of 2, 3, 4, 5, 6, 7, 8, 9, 10 or more.

In one example, in the complex for single base substitution, the deaminase may be linked with two or more binding domains. Here, each of the two or more binding domains linked to the deaminase is paired with a binding domain linked to (a) the CRISPR enzyme and a binding domain linked to (c) the DNA glycosylase. Here, due to the bind between the pairs, the components (a) CRISPR enzyme, (b) deaminase, and (c) DNA glycosylase form a complex, and a complex for single base substitution can be provided.

In one example, in the complex for single base substitution, the DNA glycosylase may be linked with two or more binding domains. Here, each of the two or more binding domains linked to the DNA glycosylase is paired with a binding domain linked to (a) the CRISPR enzyme and a binding domain linked to (b) the deaminase. Here, due to the binding between the pairs, the components (a) CRISPR enzyme, (b) deaminase, and (c) DNA glycosylase form a complex to provide the complex for single base substitution.

In one example, in the complex for single base substitution, the CRISPR enzyme may be linked with two or more binding domains, and may be present in a fusion protein in which the deaminase and the DNA glycosylase are linked. Here, the fusion protein includes one or more binding domains. In one exemplary embodiment, one binding domain linked to the CRISPR enzyme is paired with a binding domain of the fusion protein. Here, due to the binding between the pairs, the components (a) CRISPR enzyme, (b) deaminase, and (c) DNA glycosylase form a complex to provide the complex for single base substitution.

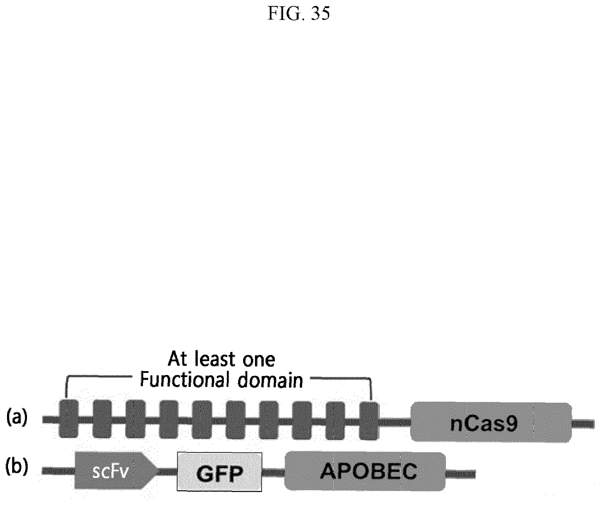

In one exemplary embodiment, the complex for single base substitution provided in the present application may be formed by specific binding of a binding domain of (a) and a binding domain of (b) in FIG. 35.

Here, a binding domain GCN4 of (a) and a binding domain scFv of (b) are merely examples and the present invention is not limited thereto. The APOBEC may be replaced with adenosine deaminase or a different type of cytidine deaminase, and the UNG may be replaced with alkyladenine DNA glycosylase.

Here, a plurality of the (b) may bind to one (a).

Here, the "plurality" means an integer of 2, 3, 4, 5, 6, 7, 8, 9, 10 or more.

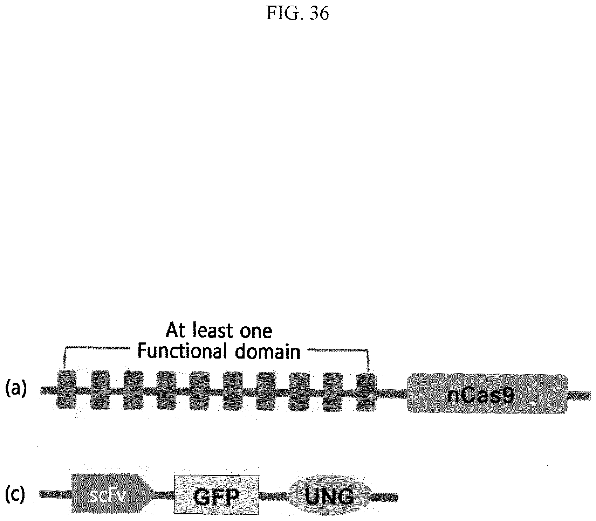

In one exemplary embodiment, the complex for single base substitution provided in the present application may be formed by specific binding of a binding domain of (a) and a binding domain of (c) in FIG. 36.

Here, a binding domain GCN4 of (a) and a binding domain scFv of (b) are merely examples and the present invention is not limited thereto. The APOBEC may be replaced with adenosine deaminase or a different type of cytidine deaminase, and the UNG may be replaced with alkyladenine DNA glycosylase.

Here, a plurality of the (b) may bind to one (a).

Here, the "plurality" means an integer of 2, 3, 4, 5, 6, 7, 8, 9, 10 or more.

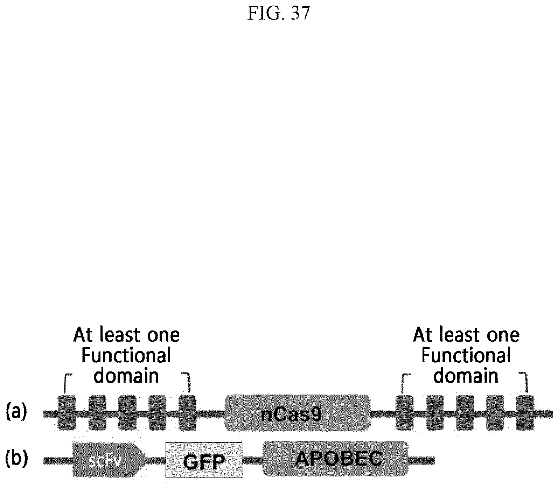

In one exemplary embodiment, the complex for single base substitution provided in the present application may be formed by specific binding of a binding domain of (a) and a binding domain of (b) in FIG. 37.

Here, a binding domain GCN4 of (a) and a binding domain scFv of (b) are merely examples and the present invention is not limited thereto. The APOBEC may be replaced with adenosine deaminase or a different type of cytidine deaminase, and the UNG may be replaced with alkyladenine DNA glycosylase.

Here, a plurality of the (b) may bind to one (a).

Here, the "plurality" means an integer of 2, 3, 4, 5, 6, 7, 8, 9, 10 or more.

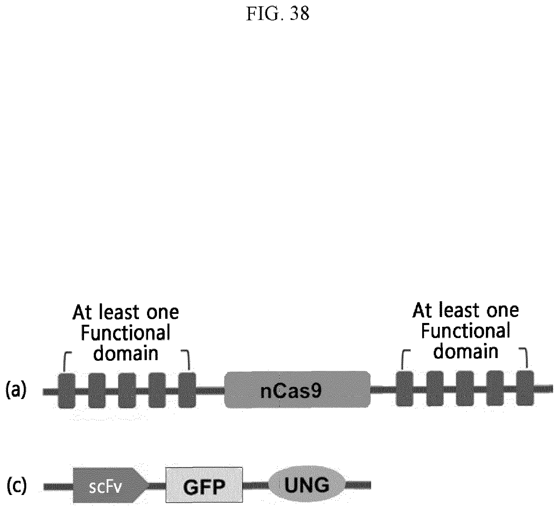

In one exemplary embodiment, the complex for single base substitution provided in the present application may be formed by specific binding of a binding domain of (a) and a binding domain of (c) in FIG. 38.

Here, a binding domain GCN4 of (a) and a binding domain scFv of (b) are merely examples and the present invention is not limited thereto. The APOBEC may be replaced with adenosine deaminase or a different type of cytidine deaminase, and the UNG may be replaced with alkyladenine DNA glycosylase.

Here, a plurality of the (b) may bind to one (a).

Here, the "plurality" means an integer of 2, 3, 4, 5, 6, 7, 8, 9, 10 or more.

In one example, the complex for single base substitution may be present in the form of a fusion protein in which the deaminase is linked with two or more binding domains, and linked with the CRISPR enzyme and the DNA glycosylase. Here, the fusion protein includes one or more binding domains. In one exemplary embodiment, one binding domain linked to the deaminase is paired with a binding domain of the fusion protein. Here, due to the binding between the pairs, the components (a) CRISPR enzyme, (b) deaminase, and (c) DNA glycosylase form a complex to provide the complex for single base substitution.

In one example, the complex for single base substitution may be present in the form of a fusion protein in which the DNA glycosylase is linked with two or more binding domains, and the deaminase and the CRISPR enzyme are linked. Here, the fusion protein includes one or more binding domains. In one exemplary embodiment, one binding domain linked to the DNA glycosylase is paired with a binding domain of the fusion protein. Here, due to the binding between the pairs, the components (a) CRISPR enzyme, (b) deaminase, and (c) DNA glycosylase form a complex to provide the complex for single base substitution.

In one example, the complex for single base substitution may include (i) a first fusion protein including two components selected from the CRISPR enzyme, the deaminase, and the DNA glycosylase, and a first binding domain, and (ii) a second fusion protein including the remaining component which has not been selected, and a second binding domain. Wherein, the first binding domain and the second binding domain are interactive pair, and here, the complex is formed by the pair. Wherein, the second fusion protein may further include a plurality of binding domains in addition to the second binding domain.

In one exemplary embodiment, the complex for single base substitution may include (i) a first fusion protein including the deaminase, the DNA glycosylase and the first binding domain, and (ii) a second fusion protein including the CRISPR enzyme and the second binding domain. Here, the second fusion protein may further include a plurality of binding domains in addition to the second binding domain. Here, the first binding domain may be a single chain variable fragment (scFv), and the second fusion protein may be a GCN4 peptide. Here, the scFv may provide the complex for single base substitution by interaction with the GCN4 peptide.

In one exemplary embodiment, the complex for single base substitution may include (i) a first fusion protein including the deaminase, the CRISPR enzyme and the first binding domain, and (ii) a second fusion protein including the DNA glycosylase and the second binding domain. Here, the second fusion protein may further include a plurality of binding domains in addition to the second binding domain. Here, the first binding domain may be a single chain variable fragment (scFv), and the second fusion protein may be a GCN4 peptide. Here, the scFv may provide the complex for single base substitution through interaction with the GCN4 peptide.

In one exemplary embodiment, the complex for single base substitution may include (i) a first fusion protein including the CRISPR enzyme, the DNA glycosylase and a first binding domain, and (ii) a second fusion protein including the deaminase and a second binding domain. Here, the second fusion protein may further include a plurality of binding domains in addition to the second binding domain. Here, the first binding domain may be a single chain variable fragment (scFv), and the second fusion protein may be a GCN4 peptide. Here, the scFv may provide a complex for single base substitution through interaction with the GCN4 peptide.

In one example, any one of the CRISPR enzyme, the deaminase, and the DNA glycosylase is linked to the first binding domain and the second binding domain, and here, the first binding domain is a pair interacting with another binding domain. Here, the second binding domain is a pair interacting with the other binding domain, and the complex for single base substitution may be provided by the pairs.

In one embodiment, the CRISPR enzyme may be linked to the first binding domain and the second binding domain. Here, the first binding domain is a pair interacting with a binding domain of the deaminase, and the second binding domain is a pair interacting with a binding domain of the DNA glycosylase. Here, the complex for single base substitution may be provided by the pairs.

In one embodiment, the deaminase may be linked to the first binding domain and the second binding domain. Here, the first binding domain is a pair interacting with a binding domain of the CRISPR enzyme, and the second binding domain is a pair interacting with a binding domain of the DNA glycosylase. Here, the complex for single base substitution may be provided by the pairs.

In one embodiment, the DNA glycosylase may be linked to a first binding domain and a second binding domain. Here, the first binding domain is a pair interacting with a binding domain of the deaminase, and the second binding domain is a pair interacting with a binding domain of the CRISPR enzyme. Here, the complex for single base substitution may be provided by the pairs.

Here, the binding domain may be one of FRB and FKBP dimerization domains; inteins; one of ERT and VPR domains; one of a GCN4 peptide and a single chain variable fragment (scFv); or a domain forming a heterodimer.

Here, the pair may be any one of the following sets:
FRB and FKBP dimerization domains;
first and second inteins;
ERT and VPR domains;
a GCN4 peptide and a single chain variable fragment (scFv); and
first and second domains forming a heterodimer.

The present application may provide a cytosine substitution complex.

For example, the deaminase may be cytidine deaminase, and the DNA glycosylase may be uracil DNA glycosylase or a variant thereof. Here, the fusion protein for single base substitution may be a complex for single base substitution which induces substitution of cytosine(s) included in one or more nucleotides in a target nucleic acid sequence with any base(s).

In one example, the cytidine deaminase is APOBEC, activation-induced cytidine deaminase (AID) or a variant thereof.

In one example, any one of the CRISPR enzyme, the cytidine deaminase, and the uracil DNA glycosylase may be linked to a first binding domain and a second binding domain. Here, the first binding domain is an interactive pair interacting with another binding domain, and here, the second binding domain is an interactive pair interacting with the other binding domain. Here, the complex for single base substitution may be provided by the pairs.

In one embodiment, the CRISPR enzyme may be linked to the first binding domain and the second binding domain. Here, the first binding domain is an interactive pair interacting with a binding domain of the deaminase, and the second binding domain is an interactive pair interacting with a binding domain of the DNA glycosylase. Here, the complex for single base substitution may be provided by the pairs.

In one example, the complex for single base substitution may include (i) a first fusion protein including a first binding domain, and two components selected from the CRISPR enzyme, the cytidine deaminase, and the uracil DNA glycosylase, and (ii) a second fusion protein including the remaining component which has not been selected, and a second binding domain. Here, the first binding domain and the second binding domain are interactive pair interacting with each other, and here, the complex may be formed by the pairs. Here, the second fusion protein may further include a plurality of binding domains in addition to the second binding domain.

Here, the pair may be any one of the following sets:
FRB and FKBP dimerization domains;
first and second inteins;
ERT and VPR domains;
a GCN4 peptide and a single chain variable fragment (scFv); and
first and second domains forming a heterodimer.

The present application may provide an adenine substitution complex.

In one example, the deaminase may be adenosine deaminase, and the DNA glycosylase may be alkyladenine DNA glycosylase or a variant thereof. Here, the fusion protein for single base substitution may be a complex for single base substitution which induces substitution of adenine(s) included in one or more nucleotides in a target nucleic acid sequence with any base(s).

In one example, the adenine cytidine deaminase may be TadA, Tad2p, ADA, ADA1, ADA2, ADAR2, ADAT2, ADAT3 or a variant thereof.

In one example, any one of the CRISPR enzyme, the adenosine deaminase, and the alkyladenine DNA glycosylase may be linked to a first binding domain and a second binding domain. Here, the first binding domain is an interactive pair interacting with a binding domain of another component, and the second binding domain is an interactive pair interacting with a binding domain of the other component. The complex for single base substitution may be provided by the pairs.

In one embodiment, the CRISPR enzyme may be linked to a first binding domain and a second binding domain. Here, the first binding domain is an interactive pair interacting with a binding domain of the deaminase, and the second binding domain is an interactive pair interacting with a binding domain of the DNA glycosylase. Here, the complex for single base substitution may be provided by the pairs.

In one example, the complex for single base substitution may include (i) a first fusion protein including a first binding domain and two components selected from the CRISPR enzyme, the adenosine deaminase and the alkyladenine DNA glycosylase, and (ii) a second fusion protein including a second binding domain and the remaining component which has not been selected. Here, the first binding domain and the second binding domain are interactive pair interacting with each other, and the complex is formed by the pairs. Here, the second fusion protein may further include a plurality of binding domains in addition to the second binding domain.

Here, the pair may be any one of the following sets:
FRB and FKBP dimerization domains;
first and second inteins;
ERT and VPR domains;
a GCN4 peptide and a single chain variable fragment (scFv); and
first and second domains forming a heterodimer.

### One aspect of the present invention disclosed in the specification is a composition for base substitution and a method using the same.

The composition for single base substitution may be used to artificially modify base(s) of one or more nucleotides in a gene.

The term "artificially modified or artificially engineered" refers to a state that has been artificially modified, not the state occurring in nature. For example, the artificially modified state may be a modification that artificially causes a mutation in a wild-type gene. Hereinafter, a non-natural, artificially-modified polymorphism-dependent gene may be used interchangeably with the term artificial polymorphism-dependent gene.

The composition for base modification may further include guide RNA or a nucleic acid encoding the same.

In one example, the present invention provides a composition for single base substitution comprising:
(a) a guide RNA or a nucleic acid encoding the same, and (b) a fusion protein for single base substitution or a nucleic acid encoding the same, or a complex for single base substitution. wherein the guide RNA may complementarily bind to a target nucleic acid sequence, wherein the target nucleic acid sequence binding to the guide RNA has a length of 15 to 25 bp, wherein the fusion protein for single base substitution or the complex for single base substitution induces substitution of one or more cytosines or adenines present in a target region including the target nucleic acid sequence with any base(s).

### [First component of composition for base substitution - guide RNA]

A composition for base substitution may include a guide RNA or a nucleic acid encoding the same.

The guide RNA (gRNA) refers to RNA capable of specifically directing a gRNA-CRISPR enzyme complex, that is, a CRISPR complex, to a target gene or nucleic acid. In addition, the gRNA refers to target gene or nucleic acid-specific RNA, and may bind to a CRISPR enzyme to guide the CRISPR enzyme to a target gene or nucleic acid.

The guide RNA may complementarily bind to a partial sequence of any one strand of the double strands of a target gene or nucleic acid. The partial sequence may refer to a target nucleic acid sequence.

The guide RNA may serve to induce a guide RNA-CRISPR enzyme complex to a location with a specific nucleotide sequence of the target gene or nucleic acid.

The guide RNA refers to RNA capable of specifically directing a gRNA-CRISPR enzyme complex, that is, a CRISPR complex, to a target gene, a target region or a target nucleic acid sequence. In addition, the gRNA refers to target gene or nucleic acid-specific RNA, and may bind to the CRISPR enzyme to guide the CRISPR enzyme to a target gene, a target region or a target nucleic acid sequence.

The guide RNA may be referred to as single-stranded guide RNA (a single RNA molecule; single gRNA; sgRNA); or double-stranded guide RNA (including more than one, generally, two, separate RNA molecules).

The guide RNA includes a site complementarily binding to the target sequence (hereinafter, referred to as a guide site) and a site involved in forming a complex with a Cas protein (hereinafter, referred to as a complex-forming site).

In one example, the guide RNA may interact with a SpCas9 protein, and may be any one selected from SEQ ID NOs. 48 to 81.

In another example, the guide RNA may interact with a CjCas9 protein, and may include any one selected from SEQ ID NOs. 82 to 92.

**[Table 1]**

| **NO.** | **Name** | **sequence (5'→3')** |
|---|---|---|
| **SEQ ID NO. 48** | Sp20-viHBV-β-#10G | GUAACACGAGCAGGGGUCCU |
| **SEQ ID NO. 49** | Sp20-viHBV-B-#11G | CCCCGCCUGUAACACGAGCA |
| **SEQ ID NO. 50** | Sp20-viHBV-B-#12G | ACCCCGCCUGUAACACGAGC |
| **SEQ ID NO. 51** | Sp20-viHBV-B-#13G | AGGACCCCUGCUCGUGUUAC |
| **SEQ ID NO. 52** | Sp20-viHBV-B-#14G | ACCCCUGCUCGUGUUACAGG |
| **SEQ ID NO. 53** | Sp20-viHBV-B-#17G | CACCACGAGUCUAGACUCUG |
| **SEQ ID NO. 54** | Sp20-viHBV-B-#20G | GGACUUCUCUCAAUUUUCUA |
| **SEQ ID NO. 55** | Sp20-viHBV-B-#52G | CCUACGAACCACUGAACAAA |
| **SEQ ID NO. 56** | Sp20-viHBV-B-#53G | CCAUUUGUUCAGUGGUUCGU |
| **SEQ ID NO. 57** | Sp20-viHBV-B-#54G | CAUUUGUUCAGUGGUUCGUA |
| **SEQ ID NO. 58** | Sp20-viHBV-B-#89G | GGGUUGCGUCAGCAAACACU |
| **SEQ ID NO. 59** | Sp20-viHBV-B-#90G | UUUGCUGACGCAACCCCCAC |
| **SEQ ID NO. 60** | Sp20-viHBV-B-#101G | UCCGCAGUAUGGAUCGGCAG |
| **SEQ ID NO. 61** | Sp20-viHBV-B-#102G | AGGAGUUCCGCAGUAUGGAU |
| **SEQ ID NO. 62** | Sp20-viHBV-B-#103G | UCCUCUGCCGAUCCAUACUG |
| **SEQ ID NO. 63** | Sp20-viHBV-B-#113G | CGUCCCGCGCAGGAUCCAGU |
| **SEQ ID NO. 64** | Sp20-viHBV-B-#117G | CCGCGGGAUUCAGCGCCGAC |
| **SEQ ID NO. 65** | Sp20-viHBV-β-#118G | UCCGCGGGAUUCAGCGCCGA |
| **SEQ ID NO. 66** | Sp20-viHBV-β-#119G | CCCGUCGGCGCUGAAUCCCG |
| **SEQ ID NO. 67** | Sp20-viHBV-B-#138G | GUAAAGAGAGGUGCGCCCCG |
| **SEQ ID NO. 68** | Sp20-viHBV-B-#140G | GGGGCGCACCUCUCUUUACG |
| **SEQ ID NO. 69** | Sp20-viHBV-B-#142G | GAAGCGAAGUGCACACGGUC |
| **SEQ ID NO. 70** | Sp20-viHBV-B-#143G | GGUCUCCAUGCGACGUGCAG |
| **SEQ ID NO. 71** | Sp20-viHBV-B-#154G | AAUGUCAACGACCGACCUUG |
| **SEQ ID NO. 72** | Sp20-viHBV-B-#159G | AGGAGGCUGUAGGCAUAAAU |
| **SEQ ID NO. 73** | Sp20-viHBV-B-#186G | CGGAAGUGUUGAUAAGAUAG |
| **SEQ ID NO. 74** | Sp20-viHBV-B-#187G | CCGGAAGUGUUGAUAAGAUA |
| **SEQ ID NO. 75** | Sp20-viHBV-B-#193G | GCGAGGGAGUUCUUCUUCUA |
| **SEQ ID NO. 76** | Sp20-viHBV-B-#194G | GACCUUCGUCUGCGAGGCGA |
| **SEQ ID NO. 77** | Sp20-viHBV-B-#196G | GAUUGAGACCUUCGUCUGCG |
| **SEQ ID NO. 78** | Sp20-viHBV-B-#197G | CUCCCUCGCCUCGCAGACGA |
| **SEQ ID NO. 79** | Sp20-viHBV-B-#198G | GAUUGAGAUCUUCUGCGACG |
| **SEQ ID NO. 80** | Sp20-viHBV-B-#199G | GUCGCAGAAGAUCUCAAUCU |
| **SEQ ID NO. 81** | Sp20-viHBV-B-#200G | UCGCAGAAGAUCUCAAUCUC |
| **SEQ ID NO. 82** | Cj22-viHBV-B-#06G | UGUCAACAAGAAAAACCCCGCC |
| **SEQ ID NO. 83** | Cj22-viHBV-B-#20G | AAGCCCUACGAACCACUGAACA |
| **SEQ ID NO. 84** | Cj22-viHBV-B-#23G | UUACCAAUUUUCUUUUGUCUUU |
| **SEQ ID NO. 85** | Cj22-viHBV-B-#40G | ACGUCCCGCGCAGGAUCCAGUU |
| **SEQ ID NO. 86** | Cj22-viHBV-B-#44G | GUGCACACGGUCCGGCAGAUGA |
| **SEQ ID NO. 87** | Cj22-viHBV-B-#45G | GUGCCUUCUCAUCUGCCGGACC |
| **SEQ ID NO. 88** | Cj22-viHBV-B-#46G | CGACGUGCAGAGGUGAAGCGAA |
| **SEQ ID NO. 89** | Cj22-viHBV-B-#47G | UGCGACGUGCAGAGGUGAAGCG |
| **SEQ ID NO. 90** | Cj22-viHBV-B-#48G | GACCGUGUGCACUUCGCUUCAC |
| **SEQ ID NO. 91** | Cj22-viHBV-B-#57G | AUGUCCAUGCCCCAAAGCCACC |
| **SEQ ID NO. 92** | Cj22-viHBV-B-#67G | GACCACCAAAUGCCCCUAUCUU |

Here, the complex-forming site may be determined according to the type of Cas9 protein-derived microorganism. For example, in the case of the guide RNA interacting with the SpCas9 protein, the complex-forming site may include 5'-GUUUUAGUCCCUGAAAAGGGACUAAAAUAAAGAGUUUGCGGGACUCUGCGGG GUUACAAUCCCCUAAAACCGCUUUU-3' (SEQ. ID NO: 45), and in the case of the guide RNA interacting with the CjCas9 protein, the complex-forming site may include 5'-GUUUUAGAGCUAGAAAUAGCAAGUUAAAAUAAGGCUAGUCCGUUAUCAACUU GAAAAAGUGGCACCGAGUCGGUGC-3' (SEQ ID NO: 46).

As the proto-spacer-adjacent motif (PAM) sequence, when the spCas9 protein is used, NGG (N is A, T, C or G) is considered, and when the CjCas9 protein is used, NNNNRYAC (SEQ ID NO: 47) is considered (N is each independently A, T, C or G, R is A or G, and Y is C or T).

The composition may include one or a plurality of guide RNAs.

### [Second component of composition for base substitution- protein for single base substitution]

The composition for base substitution may include a protein for single base substitution or a nucleic acid encoding the same.

The protein for single base substitution is the same as described above.

### [Third component of composition for base substitution- vector]

The composition for base modification may be in the form of a vector.

The "vector" may deliver a gene sequence to a cell. Typically, the "vector structure," "expression vector," and "gene transfer vector" may direct the expression of a desired gene, and refers to any nucleic acid structure capable of delivering a gene sequence to a target cell. Accordingly, the term "vector" includes vectors, as well as cloning and expression vehicles.

Here, the vector may be a virus or non-viral vector (e.g., a plasmid).

Here, the vector may include one or more regulatory/control element.

Here, the regulatory/control element may include a promoter, an enhancer, an intron, a polyadenylation signal, a Kozak consensus sequence, an internal ribosome entry site (IRES), a splice acceptor and/or a 2A sequence.

The promoter may be a promoter recognized by RNA polymerase II.

The promoter may be a promoter recognized by RNA polymerase III.

The promoter may be an inducible promoter.

The promoter may be a target-specific promoter.

The promoter may be a viral or non-viral promoter.

As the promoter, a suitable promoter according to a control region (that is, a nucleic acid sequence encoding guide RNA or a CRISPR enzyme) may be used.

For example, a promoter useful for the guide RNA may be a HI, EF-1a, tRNA or U6 promoter. For example, a promoter for the CRISPR enzyme may be a CMV, EF-1a, EFS, MSCV, PGK or CAG promoter.

The vector may be a viral vector or a recombinant viral vector.

The virus may be a DNA virus or RNA virus.

Here, the DNA virus may be a double-stranded DNA (dsDNA) virus or a single-stranded DNA (ssDNA) virus.

Here, the RNA virus may be a single-stranded RNA (ssRNA) virus.

The virus may be retrovirus, lentivirus, adenovirus, adeno-associated virus (AAV), vaccinia virus, poxvirus or herpes simplex virus, but the present invention is not limited thereto.

Generally, the virus may infect a host (e.g., cells) to introduce a nucleic acid encoding genetic information of the virus into a host, or insert a nucleic acid encoding genetic information into the genome of a host. The guide RNA and/or the CRISPR enzyme may be introduced into a target using a virus with the above characteristics. The guide RNA and/or CRISPR enzyme introduced using such a virus may be temporarily expressed in a subject (e.g., cells). Alternatively, the guide RNA and/or CRISPR enzyme introduced using such a virus may be continuously expressed in a subject(e.g., cells) for a long period of time (e.g., 1 week, 2 weeks, 3 weeks, 1 month, 2 months, 3 months, 6 months, 9 months, 1 year, 2 years or permanently).

A virus packaging capacity may vary from at least 2 kb to 50 kb, depending on a virus type. According to such packaging capacity, a viral vector independently including the guide RNA or the CRISPR enzyme or a viral vector including both of the guide RNA and the CRISPR enzyme may be designed. Alternatively, a viral vector including the guide RNA, the CRISPR enzyme and additional components may be designed.

For example, a retroviral vector has a packaging capacity for a foreign sequence of up to 6 to 10 kb, and consists of cis-acting long terminal repeats (LTRs). The retroviral vector inserts a therapeutic gene in to cells, and provides permanent expression of an inserted gene.

In another example, an adeno-associated viral vector has a very high introduction efficiency into various cells (muscular, brain, liver, lung, retinal, ear, heart and blood vessel cells) regardless of cell division and has no pathogenicity, and since most of the viral genome may be replaced by a therapeutic gene and does not induce an immune response, repeated administration is possible. In addition, AAV is inserted into the chromosome of a target cell, thereby stably expressing the therapeutic protein for a long time. For example, AAV is useful for generating a nucleic acid and a peptide *in vitro* and transducing the nucleic acid or the peptide to a target nucleic acid of cells *in vivo* and *ex vivo.* However, AAV is small in size and has a packaging capacity of less than 4.5 kb.

Wherein, the composition for base modification may include a vector including a nucleic acid encoding guide RNA; and an adenine base substitution protein.

Wherein, the composition for base modification may include a guide RNA; and a vector including a nucleic acid encoding a protein for adenine base substitution.

Wherein, the composition for base modification may include a vector including a nucleic acid encoding guide RNA; and a vector including a nucleic acid encoding an protein for adenine base substitution.

Wherein, the composition for base modification may include a vector including a nucleic acid encoding guide RNA and a nucleic acid encoding an adenine base substitution protein.

In another example, the composition for base modification may include
(a) a CRISPR enzyme including a first binding domain or a nucleic acid encoding the same; and
(b) an adenosine deaminase including a second binding domain or a nucleic acid encoding the same.

Wherein, the CRISPR enzyme may be a wild-type CRISPR enzyme or a CRISPR enzyme variant.

Wherein, the CRISPR enzyme variant may be a nickase.

The adenosine deaminase may be TadA, Tad2p, ADA, ADA1, ADA2, ADAR2, ADAT2, ADAT3 or a variant thereof.

The first binding domain may form a non-covalent bond with a second binding domain.

Wherein, the first binding domain may be one of FRB and FKBP dimerization domains; inteins; one of ERT and VPR domains; one of a GCN4 peptide and a single chain variable fragment (scFv); or a domain forming a heterodimer.

Wherein, the second binding domain may be one of FRB and FKBP dimerization domains; inteins; one of ERT and VPR domains; one of a GCN4 peptide and a single chain variable fragment (scFv); or a domain forming a heterodimer.

The composition for base modification may further include one or more guide RNAs or nucleic acids encoding the same.

Wherein, the composition for base modification may be in the form of ribonucleoprotein (RNP), that is a complex comprising
a guide RNA; -
a CRISPR enzyme having a first binding domain; and -
an adenosine deaminase having a second binding domain.

Wherein, the composition for base modification may include
a vector including a nucleic acid encoding guide RNA;
a vector including a nucleic acid encoding a CRISPR enzyme having a first binding domain; and
a vector including a nucleic acid encoding adenosine deaminase having a second binding domain.

Wherein, the composition for base modification may include
A vector including a nucleic acid encoding guide RNA; and
a complex of a CRISPR enzyme including first binding domain- and adenosine deaminase including second binding domain.

Wherein, the composition for base modification may include a vector including a nucleic acid encoding guide RNA; a nucleic acid encoding a CRISPR enzyme having a first binding domain and a nucleic acid encoding adenosine deaminase having a second binding domain.

Wherein, the composition for base modification may include a vector including a nucleic acid encoding guide RNA and a nucleic acid encoding CRISPR enzyme having a first binding domain; and a vector including a nucleic acid encoding adenosine deaminase having a second binding domain.

Wherein, the composition for base modification may include a vector including a nucleic acid encoding a CRISPR enzyme having a first binding domain; and a vector including a nucleic acid encoding guide RNA and a nucleic acid encoding adenosine deaminase having a second binding domain.

Wherein, the composition for base modification may include a vector including a nucleic acid encoding guide RNA; a CRISPR enzyme having a first binding domain; and a vector including a nucleic acid encoding adenosine deaminase having a second binding domain.

Wherein, the composition for base modification may include a vector including a nucleic acid encoding guide RNA; a vector including a nucleic acid encoding a CRISPR enzyme having a first binding domain; and adenosine deaminase having a second binding domain.

Wherein, the composition for base modification may include a vector including a nucleic acid encoding guide RNA and a nucleic acid encoding a CRISPR enzyme having a first binding domain; and adenosine deaminase having a second binding domain.

Wherein, the composition for base modification may include a CRISPR enzyme having a first binding domain; and a vector including a nucleic acid encoding guide RNA and a nucleic acid encoding adenosine deaminase having a second binding domain.

### [Fourth component of composition for base substitution- guide RNA- protein for single base substitution complex]

The composition for base modification may be a nucleic acid-protein complex. Wherein, the nucleic acid-protein complex may be a complex of guide RNA-protein for adenine base substitution. Wherein, the nucleic acid-protein complex may be a complex of guide RNA-protein for cytosine base substitution.

Wherein, the complex of guide RNA-protein for adenine base substitution may be formed by a non-covalent bond between the guide RNA and the protein for adenine base substitution.

Wherein, the complex of guide RNA-protein for cytosine base substitution may be formed by a non-covalent bond between the guide RNA and the protein for cytosine base substitution.

The composition for base modification may be a non-vector type.

Here, the non-vector may be naked DNA, a DNA complex or mRNA.

The composition for base modification may be in the form of a vector.

The descriptions of vectors have been provided above.

In one example, the composition for base modification may include a protein for adenine base substitution having a CRISPR enzyme and adenosine deaminase, or a nucleic acid encoding the same.

Wherein, the CRISPR enzyme may be a wild-type CRISPR enzyme or a CRISPR enzyme variant.

Wherein, the CRISPR enzyme variant may be a nickase.

The adenosine deaminase may be TadA, Tad2p, ADA, ADA1, ADA2, ADAR2, ADAT2, ADAT3 or a variant thereof.

The protein for adenine base substitution may be composed in the order of N terminus-[CRISPR enzyme]-[adenosine deaminase]-C terminus.

The protein for adenine base substitution may be composed in the order of N terminus-[adenosine deaminase]-[CRISPR enzyme]-C terminus.

Wherein, the protein for adenine base substitution may further include a linking domain.

The composition for base modification may further include one or more guide RNAs or nucleic acids encoding the same.

Wherein, the composition for base modification may be in the form of a guide RNA-protein for adenine base substitution complex, that is, a ribonucleoprotein (RNP).

**One aspect of the present invention disclosed in the specification is the use of a protein for single base substitution or a composition for single base substitution including the same.**

The following uses of the protein for single base substitution provided in the present application may be provided.

The composition for base modification may be used to artificially modify a base(s) of one or more nucleotides in a target gene.
(i) The composition for base modification may be used to obtain the information on a part mutated so as not to identify a material expressed from the modified nucleic acid sequence, that is, an epitope having an antibody resistance, by artificially modifying base(s) of one or more nucleotides of a target region of a specific gene.
(ii) The composition for base modification may be used to obtain the information on whether the sensitivity of a material expressed from a modified nucleotide to a specific drug is reduced or lost, by artificially modifying base(s) of one or more nucleotides of a target region of a specific gene. That is, the composition for base modification may be used to find or confirm a region of a target gene or a protein encoded by the target gene (a target protein), affecting a specific drug.
(iii) The composition for base modification may be used to obtain the information on whether the sensitivity of a material expressed from a modified nucleotide to a specific drug is increased, by artificially modifying base(s) of one or more nucleotides of a target region of a specific gene. That is, the composition for base modification may be used to find or confirm a region of a target gene or a protein encoded by the target gene (a target protein), affecting an increase in sensitivity to a specific drug.
(iv) The composition for base modification may be used to obtain the information on whether a material expressed from a modified nucleic acid sequence has a resistance to a virus, by artificially modifying base(s) of one or more nucleotides of a target region of a specific gene. That is, the composition for base modification may be used for screening a virus resistance gene or a virus resistance protein.

### [First use - epitope screening]

In one embodiment, a protein for single base substitution or a composition for base substitution including the same may be used for epitope screening.

The "epitope" refers to a specific part of an antigen that allows an immune system such as an antibody, a B cell or a T cell to identify the antigen, and is also called an antigenic determinant. Epitopes of a protein are largely classified into conformational epitopes and linear epitopes according to a shape or a mode of acting with an antigen-binding site which is a specific part of an antibody which identifies an epitope. A conformational epitope consists of a discontinuous amino acid sequence of an antigen, that is, a protein. A conformational epitope reacts with the three-dimensional structure of the antigen-binding site of an antibody. Most epitopes are conformational epitopes. Conversely, a linear epitope reacts with the one-dimensional structure of the antigen-binding site of an antibody, and the amino acids constituting the linear epitope of an antigen are arranged sequentially.

The "epitope screening" means finding or detecting a specific part of an antigen, which allows an immune system such as an antibody, a B cell or a T cell to identify the antigen, or a method, composition or kit for finding or detecting a specific part of an antigen, which is mutated so that an immune system such as an antibody, a B cell or a T cell does not identify the antigen. Wherein, the specific part of an antigen, which is mutated for an immune system such as an antibody, a B cell or a T cell to not identify the antigen, may be an epitope with antibody resistance.

The single base substitution protein or a composition for base substitution including the same may artificially generate a single nucleotide polymorphism (SNP) to reveal the location of the SNP involved in changes in the body, such as generation, inhibition, increase or reduction of the expression of a specific factor, generation or loss of a specific function, the presence or absence of a disease, or the difference in reactivity to an external drug or compound, such as sequences available as epitopes and positions of single-nucleotide polymorphisms involved in drug resistance.

The descriptions of the single base substitution protein and the composition for base substitution have been provided above.

For the epitope screening, the single base substitution protein may be used to induce artificial SNPs in genome.

Here, the artificial SNPs may cause point mutations.

Point mutations refer to mutations caused by modification of one nucleotide. The point mutations are classified into a missense mutation, a nonsense mutation and a silent mutation.

The missense mutation refers a case in which a mutated codon encodes another amino acid due to one or more modified nucleotides. The nonsense mutation refers to a case in which a codon mutated by one or more modified nucleotides is a stop codon. The silent mutation refers to a case in which a codon mutated by one or more modified nucleotides encodes the same amino acid as encoded by a codon that is not mutated.

In one example, by substitution of one base A with another base C, T or G, a codon may be changed to a codon encoding a different amino acid. In other words, a missense mutation may occur. For example, when A is substituted with C, leucine may be changed to glycine.

In another example, by substitution of one base A with another base C, T or G, a codon may be changed to another codon encoding the same amino acid. In other words, a silent mutation may occur. For example, when A is substituted with C, a codon encoding the same proline may be generated.

In still another example, when A is substituted with C, T or G, thereby generating TAG, TGC or TAA, one of stop codons such as UAA, UAG and UGA may be generated. In other words, a nonsense mutation may occur.

The single base substitution protein may induce or generate artificial substitution at base(s) of one or more nucleotides in a gene, thereby causing a point mutation.

The composition for base substitution may induce or generate artificial substitution at base(s) of one or more nucleotides in a gene, thereby causing a point mutation.

The induction of artificial substitution of a single base has been described above.

A protein encoded by a point mutation that has been caused by the single base substitution protein or the composition for base substitution including the same may be a protein variant in which at least one or more amino acids are changed.

For example, when a point mutation is generated in a gene encoding EGFR by the single base substitution protein or the composition for base substitution including the same, a protein encoded by the generated point mutation may be an EGFR variant in which at least one amino acid is different from those of wild-type EGFR.

One or more modified amino acids may be changed to amino acids with similar properties.

A hydrophobic amino acid may be changed to a different hydrophobic amino acid. The hydrophobic amino acid may be one of glycine, alanine, valine, isoleucine, leucine, methionine, phenylalanine, tyrosine and tryptophan.

A basic amino acid may be changed to another basic amino acid. The basic amino acid is one of arginine and histidine.

The acidic amino acid may be changed to another acidic amino acid. The acidic amino acid is one of glutamic acid and aspartic acid.

A polar amino acid may be changed to another polar amino acid. The polar amino acid is one of serine, threonine, asparagine and glutamine.

One or more modified amino acids may be changed to amino acids with different properties.

In one example, a hydrophobic amino acid may be changed to a polar amino acid.

In another example, a hydrophobic amino acid may be changed to an acidic amino acid.

In one example, a hydrophobic amino acid may be changed to a basic amino acid.

In another example, a polar amino acid may be changed to a hydrophobic amino acid.

In one example, an acidic amino acid may be changed to a basic amino acid.

In another example, a basic amino acid may be changed to an acidic amino acid.

The protein variant in which at least one amino acid is changed may have a modified three-dimensional structure. When one or more amino acids in an amino acid sequence are changed to amino acid(s) with different properties, due to a changed binding strength between amino acid sequences, the three-dimensional structure may be changed. When the three-dimensional structure is changed, a conformational epitope may be modified. The modification may be induced using the single base substitution protein provided in the present application or the composition including the same.

For example, when a point mutation of a gene encoding ATM is caused by the protein for single base substitution or the composition for base modification including the same, the three-dimensional structure of an ATM variant encoded by the generated point mutation may be partially changed, and thus a conformational epitope may be modified. The modification may be induced using the single base substitution protein provided in the present application or the composition including the same.

A gene having an artificial SNP may adjust an amount of a synthesized protein.

In one example, the gene having an artificial SNP may be increased or decreased in transcription amount of mRNA. Therefore, a protein synthesis amount may be increased or decreased.

In another example, when the regulatory region in the gene includes one or more artificial polymorphisms, the amount of protein synthesized from the gene containing the single nucleotide polymorphism may be increased or decreased.

The artificial SNP present in a gene may regulate the activity of a protein.

In one example, the one or more artificial SNPs may promote and/or reduce protein activity.

For example, when the artificial SNPs are included in a gene encoding a nuclear membrane receptor, all factors or mechanisms (phosphorylation, acetylation, etc.) involved in a signaling process by recognition of a ligand and binding to a ligand may be activated or reduced.

For example, when the artificial SNPs are included in a gene encoding a specific enzyme, the function of an enzyme such as an acetylase, that is, a degree of acetylation of a target gene may be promoted or reduced.

The artificial SNPs present in a gene may change the protein function.

In one example, the original function of the protein may be added and/or inhibited by one or more artificial SNPs.

For example, when artificial SNPs are included in a gene encoding a nuclear membrane receptor, a capability of recognizing and/or binding to a ligand may be inhibited.

Alternatively, for example, when artificial SNPs are included in a gene encoding a nuclear membrane receptor, some of the signaling functions to a downstream factor by binding to a ligand may be inhibited.

In one embodiment, an epitope screening method may include:
a) preparing cells capable of expressing one or more guide RNAs of one or more guide RNA libraries complementarily binding to a target nucleic acid sequence present in a target gene, the cell having a target nucleic acid sequence;
b) introducing a single base substitution protein or a nucleic acid encoding the same into the cells;
c) treating the cells of b) with a drug or therapeutic agent;
d) isolating viable cells; and
e) analyzing a nucleic acid sequence of the target gene in the isolated cells.

In one embodiment, the epitope screening method may include:
a) preparing cells capable of expressing one or more guide RNAs of one or more guide RNA libraries complementarily binding to a target nucleic acid sequence present in a target gene, the cells having a target nucleic acid sequence;
b) introducing a protein for single base substitution or a nucleic acid encoding the same into the cells;
c) treating the cells of b) with a drug or therapeutic agent;
d) isolating viable cells; and
e) obtaining information on a desired SNP from the isolated cells.

Here, the desired SNP may be associated with the structure or function of a protein expressed from the target gene.

In one embodiment, the epitope screening method may include:
a) introducing a protein for single base substitution or nucleic acid encoding the same, and one or more guide RNAs of one or more guide RNA libraries or nucleic acids encoding the same into cells having a target nucleic acid sequence;
b) treating the cells of a) with a drug or therapeutic agent;
c) isolating viable cells; and
d) obtaining information on a desired SNP from the isolated cells.

Here, the desired SNP may be associated with the structure or function of a protein expressed from the target gene.

In another embodiment, the epitope screening method may include:
a) introducing a composition for base substitution into cells having a target nucleic acid sequence;
b) treating the cells of a) with a drug or therapeutic agent;
c) isolating viable cells; and
d) analyzing a nucleic acid sequence of the target gene in the isolated cells.

In another embodiment, the epitope screening method may include:
a) introducing a composition for base substitution into the cell having a target nucleic acid sequence;
b) treating the cells of a) with a drug or therapeutic agent;
c) isolating viable cells; and
d) obtaining information on a desired SNP from the isolated cells.

Here, the desired SNP may be associated with the structure or function of a protein expressed from the target gene.

The guide RNA library may be a group of one or more guide RNAs complementarily binding to a partial nucleic acid sequence of a target sequence. Although nucleic acids encoding the same guide RNA library are introduced into each cell, the cell may have different guide RNA. As a result of introduction of nucleic acids encoding the same guide RNA library into each cell, the cell may have the same guide RNA.

The descriptions of the guide RNA have been described above.

The protein for single base substitution may be a protein for adenine substitution or a protein for cytosine substitution.

The descriptions of the protein for single base substitution, the protein for adenine substitution and the protein for cytosine substitution have been described above.

The introduction may be performed by one or more methods selected from electroporation, liposomes, plasmids, viral vectors, nanoparticles and a protein translocation domain (PTD)-fused protein.

The antibody treated as above may be an antibody identifying a protein encoding a target gene (hereinafter, referred to as a target protein), and may be an antibody capable of reacting with an epitope of the target protein.

The viable cells may be cells that do not react with the antibody treated as above.

The isolated cells may be cells having at least one nucleotide modification in a target gene.

Here, the modification of one or more nucleotides may be one or more artificial SNPs generated in a target gene.

Here, the one or more artificial SNPs may induce point mutations.

In the present application, the modification of at least one nucleotide present in a target gene, that is, one or more artificial SNPs, may be confirmed. Accordingly, target information may be obtained.

Here, a nucleic acid sequence including the confirmed modification of at least one nucleotide, that is, one or more artificial SNPs, may be a nucleic acid sequence encoding an epitope.

### [Second use - screening of drug resistance gene or drug resistance protein]

In another embodiment, the protein for single base substitution or the composition for base substitution including the same may be used for screening of a drug resistance gene or a drug resistance protein.

Drug resistance screening may provide information on one region of a target gene affecting the reduction or loss of sensitivity to a specific drug or a protein encoding the target gene (hereinafter, referred to as a target protein). The region may be found or identified using the single base substitution protein provided in the present application or the composition including the same.

The present application provides a method of screening a drug resistance gene or a drug resistance protein. Hereinafter, in one example of the screening method, specific steps will be described.

### Preparation of sgRNA library

Guide RNA capable of complementarily binding to one region of a target gene is prepared. In one embodiment, guide RNA capable of complementarily binding to one region of an exon in a target gene is prepared. Here, 1, 2, 3, 4, 5, 6, 7, 8, 9, 10, 20, 30, 40, 50, 60, 70, 80, 90, 100, 200, 500, 1,000, 2,000 or 3,000 or more guide RNAs may be prepared. Here, a plurality of the prepared guide RNAs may complementarily bind to one region of an exon in a target gene.

In one example, the guide RNA includes site(s) capable of complementarily binding to nucleotide sequence(s) corresponding to 1, 2, 3, 4, 5, 6, 7, 8, 9, 10, 11, 12, 13, 14, 15, 16, 17, 18, 19, 20, 21, 22, 23, 24, 25, 26, 27, 28, 29 or 30 or more regions of an exon region in a target gene.

### Preparation of transformed cells capable of expressing guide RNA

Cells that can prepare guide RNA capable of complementarily binding to one region of an exon in a target gene are prepared. The cells may be transfected by a vector encoding the prepared sgRNA library. Here, the cells may express one or more guide RNAs which are encoded in the sgRNA library.

### Introduction of single base substitution protein into transformed cells

A single base substitution protein or a nucleic acid encoding the same is introduced into transformed cells capable of expressing one or more guide RNAs encoded in an sgRNA library. The single base substitution protein may induce substitution of any one or more bases in a target region with any base(s).

The single base substitution protein may induce the generation of at least one SNP in a target gene.

The single base substitution protein may induce the generation of at least one SNP in a target region.

In one example, when the introduced single base substitution protein is a cytidine substitution protein, at least one cytosine in a target region may be substituted with any base.

In one example, when the introduced single base substitution protein is an adenine substitution protein, at least one adenine in a target region may be substituted with any base.

### Preparation of transformed cells

Instead of the steps of preparing transformed cells capable of expressing guide RNA and introducing a protein for single base substitution into the transformed cells, the method of the present application may be performed by the following steps.

Cells having a target gene are prepared.

The single base substitution protein and the guide RNA are introduced into the cells. Here, the single base substitution protein and the guide RNA may be introduced in the form of an RNP complex (ribonucleoprotein complex), or the form of nucleic acids encoding them, respectively.

### Treatment of transformed cells with drug or therapeutic agent

The transformed cells are treated with a material that can be used as a drug or therapeutic agent such an antibiotic, an anticancer agent or an antibody. Here, the treated drug or therapeutic agent may specifically bind to or react with a peptide, polypeptide or protein expressed from the target gene. Alternatively, the treated drug or therapeutic agent may reduce or lose the activity or function of a peptide, polypeptide or protein expressed from the target gene. Alternatively, the treated drug or therapeutic agent may improve or increase the activity or function of a peptide, polypeptide or protein expressed from the target gene.

The transformed cells may be killed by the drug or therapeutic agent.

The transformed cells may survive despite the treatment of the drug or therapeutic agent.

### Cell selection

Despite the treatment of the drug or therapeutic agent, viable cells may be isolated, selected or obtained.

In the viable cells, at least one base in a target region of a target gene may be substituted with any base using at least one guide RNA and a protein for single base substitution. The cells in which a base in the target gene is substituted with any base using the protein for single base substitution may have resistance to the treated drug or therapeutic agent.

Here, a peptide, polypeptide or protein expressed from the target gene of the surviving cell may have resistance to the drug or therapeutic agent.

### Obtaining of information

The nucleic acid sequence of the genome or target gene of the viable cells may be analyzed to obtain information on a site having resistance to the treated drug or therapeutic agent.

The nucleic acid sequence of the genome or target gene of the viable cells may be analyzed to obtain information on whether the structure or function of a peptide, polypeptide or protein expressed from the target gene is changed. The changed structure or function may play a critical role for determining whether there is drug resistance.

In one embodiment, the method of screening a drug resistance gene or a drug resistance protein may include:
a) preparing cells having a target gene;
b) introducing one or more guide RNAs of one or more guide RNA libraries capable of complementarily binding to a target nucleic acid sequence or nucleic acids encoding the same, and a single base substitution protein or a nucleic acid encoding the same into the cells;
c) treating the cells of b) with a drug or therapeutic agent;
d) isolating viable cells; and
e) analyzing the nucleic acid sequence of the target gene in the isolated cells.

In one embodiment, the method of screening a drug resistance gene or a drug resistance protein may include:
a) preparing cells capable of expressing one or more guide RNAs of one or more guide RNA libraries which can complementarily bind to a target nucleic acid sequence present in a target gene;
b) introducing a single base substitution protein or a nucleic acid encoding the same into the cells;
c) treating the cells of b) with a drug or therapeutic agent;
d) isolating viable cells; and
e) analyzing the nucleic acid sequence of the target gene in the isolated cells.

In one embodiment, the method of screening a drug resistance gene or a drug resistance protein may include:
a) preparing cells capable of expressing one or more guide RNAs of one or more guide RNA libraries which can complementarily bind to a target nucleic acid sequence present in a target gene;
b) introducing a single base substitution protein or a nucleic acid encoding the same into the cells;
c) treating the cells of b) with a drug or therapeutic agent;
d) isolating viable cells; and
e) obtaining information on a desired SNP from the isolated cells.

Here, the desired SNP may be associated with the structure of function of the protein expressed from the target gene.

In one embodiment, the method of screening a drug resistance gene or a drug resistance protein may include:
a) introducing a single base substitution protein or a nucleic acid encoding the same, and any one or more of guide RNAs of a guide RNA library or a nucleic acid encoding the same into cells;
b) treating the cells of a) with a drug or therapeutic agent;
c) isolating viable cells; and
d) obtaining information on a desired SNP from the isolated cells.

Here, the desired SNP may be associated with the structure of function of the protein expressed from the target gene.

In another embodiment, the method of screening a drug resistance gene or a drug resistance protein may include:
a) introducing a composition for base substitution into cells having a target nucleic acid sequence;
b) treating the cells of a) with a drug or therapeutic agent;
c) isolating viable cells; and
d) analyzing the nucleic acid sequence of the target gene in the isolated cells.

In another embodiment, the method of screening a drug resistance gene or a drug resistance protein may include:
a) introducing a composition for base substitution into cells having a target nucleic acid sequence;
b) treating the cells of a) with a drug or therapeutic agent;
c) isolating viable cells; and
d) obtaining information on a desired SNP from the isolated cells.

Here, the desired SNP may be associated with the structure of function of the protein expressed from the target gene.

The guide RNA library may be a group of one or more guide RNAs which can complementarily bind to a partial nucleic acid sequence of a target sequence. Although nucleic acids encoding the same guide RNA library are introduced into cells, respectively, each cell may include different guide RNAs. As a result of introduction of nucleic acids encoding the same guide RNA library into each cell, each cell may have the same guide RNA.

The descriptions of the guide RNA have been provided above.

The single base substitution protein may be a protein for adenine substitution or a protein for cytidine substitution.

The descriptions of the single base substitution protein, the adenine substitution protein and the cytidine substitution protein have been provided above.

The introduction may be performed by one or more methods selected from electroporation, liposomes, plasmids, viral vectors, nanoparticles and a protein translocation domain (PTD)-fused protein.

The drug treated as above may be a material that suppresses or inhibits the activity or function of a protein encoded by a target gene (hereinafter, referred to as a target protein). Here, the material may be a biological material (e.g., RNA, DNA, a protein, a peptide or an antibody) or a non-biological material (e.g., a compound).

The drug treated as above may be a material that promotes or increases the activity or function of a protein encoded by a target gene (hereinafter, referred to as a target protein). Here, the material may be a biological material (e.g., RNA, DNA, a protein, a peptide or an antibody) or a non-biological material (e.g., a compound).

The viable cells may be cells which have the activity of a target protein, such as drug resistance without functional change by the drug treated as above.

The isolated cells may be cells having modification of at least one nucleotide in a target gene.

Here, the modification of one or more nucleotides may be one or more artificial SNPs generated in the target gene.

Here, the one or more artificial SNPs may induce a point mutation.

Here, the modification of at least one nucleotide, that is, one or more artificial SNPs, present in the target gene may be identified. Accordingly, desired information may be obtained.

Here, a nucleic acid sequence including the identified modification of at least one nucleotide, that is, one or more artificial SNPs, may be a nucleic acid sequence encoding one region of a protein affecting drug resistance.

The drug treated as above may be an anticancer agent. However, it is not limited to an anticancer agent, and includes materials or therapeutic agents for treating all known diseases or disorders.

In one example, the drug may use a mechanism of interrupting the growth of cancer cells by inhibiting an epidermal growth factor receptor (EGFR), inhibiting angiogenesis toward cancer cells by blocking a vascular endothelial growth factor (VEGF), or inhibiting anaplastic lymphoma kinase.

In one embodiment, the method of screening a drug resistance mutation may include inducing artificial SNPs on a target gene by introducing the composition for single base substitution into cells including the target gene, treating the cells with a specific drug, selecting viable cells having a desired SNP, and obtaining information on the desired SNP by analyzing the selected cells. Wherein, the desired SNP may be associated with the structure or function of a protein expressed from the target gene.

In one embodiment, the target gene may be an EGFR gene, a VEGF gene, or an anaplastic lymphoma kinase gene. However, the present invention is not limited thereto.

In one embodiment, the drug treated as above may be cisplatin, carboplatin, vinorelbine, paclitaxel, docetaxel, gemcitabine, pemetrexed, iressa, tarceva, giotrif, tagrisso, Xalkori, zykadia, alectinib, Alunbrig (brigatinib), Avastin (bevacizumab), Avastin (bevacizumab), keytruda (pembrolizumab), Opdivo (nivolumab), Tecentriq (atezolizumab), Imfinzi (durvalumab) or osimertinib. However, the present invention is not limited thereto.

In one embodiment, a method of screening an EGFR mutant gene having osimertinib resistance may be performed as follows.

In one embodiment, a method of screening a drug resistance mutant may include inducing an artificial SNP on an EGFR gene by introducing a composition for single base substitution into cells having the EGFR gene, treating the cells with a drug, selecting viable cells having a desired SNP, and obtaining information on the desired SNP by analyzing the selected cells. Wherein, the desired SNP may be associated with the structure or function of the EGFR.

Here, the treated drug may be osimertinib. However, the present invention is not limited thereto, and may be any material for inhibiting or losing the EGFR function.

In one embodiment, a method of screening a drug resistance mutant may include inducing an artificial SNP on an EGFR gene by introducing a composition for single base substitution including C797S sgRNA1 and/or C797S sgRNA2 into cells having the EGFR gene, treating the cells with drug, selecting viable cells having a desired SNP, and obtaining information on the desired SNP by analyzing the selected cells. Wherein, the desired SNP is associated with the structure or function of the EGFR.

Wherein, the treated drug may be osimertinib. However, the present invention is not limited thereto, and the treated drug may be any material for inhibiting or losing the EGFR function.

According to one embodiment, an EGFR region having osimertinib resistance was identified. It was confirmed that, in the EGFR region having osimertinib resistance, SNPs are induced by the introduced composition for single base substitution or single base substitution protein.

That is, information on various positions which can show resistance to the osimertinib may be obtained by substituting cytosine present in an EGFR gene in cells with any base using the single base substitution protein provided in the present application.

In one embodiment, the present application may provide a method of obtaining EGFR resistance SNP information, which may include:
a) introducing a single base substitution protein or a nucleic acid encoding the same, and any one or more guide RNAs of a guide RNA library or nucleic acids encoding the same into cells;
b) treating the cells of a) with a drug or therapeutic agent;
c) isolating viable cells; and
d) obtaining information on a desired SNP from the isolated cells.

Wherein, the desired SNP may be associated with the structure or function of a protein expressed from the target gene.

### [Third use - drug sensitization screening]

In one embodiment, a single base substitution protein or a composition for base modification including the same may be used in drug sensitization screening.

The "drug sensitization" refers to being hypersensitive to a specific drug, and a state in which the sensitivity to a specific drug is increased. Conversely, the "desensitization" refers to a state in which the sensitivity to a specific drug is lost, and a state in which there is resistance to a specific drug.

Drug sensitization screening refers to a method, composition or kit of finding or confirming one region of a target gene affecting an increase in sensitivity to a specific drug or a protein encoding the target gene (hereinafter, referred to as a target protein).

In one embodiment, the drug sensitization screening method may include:
a) preparing cells which can express any one or more guide RNAs of one or more guide RNA libraries capable of complementarily binding to a target nucleic acid present in a target gene;
b) introducing a single base substitution protein or a nucleic acid encoding the same into the cells;
c) treating the cells of b) with a drug or therapeutic agent;
d) isolating viable cells; and
e) analyzing a nucleic acid sequence of the target gene in the isolated cells.

In one embodiment, a drug sensitization screening method may include:
a) preparing cells which can express any one or more guide RNAs of one or more guide RNA libraries capable of complementarily binding to a target nucleic acid present in a target gene, wherein the cells comprise a target nucleic acid sequence;
b) introducing a single base substitution protein or a nucleic acid encoding the same into the cells;
c) treating the cells of b) with a drug or therapeutic agent;
d) isolating viable cells; and
e) obtaining information on a desired SNP from the isolated cells.

Wherein, the desired SNP may be associated with the structure or function of a protein expressed from the target gene.

In one embodiment, a drug sensitization screening method may include:
a) introducing a protein for single base substitution or a nucleic acid encoding the same, and any one or more guide RNAs of a guide RNA library or nucleic acids encoding the same into cells having a target nucleic acid sequence;
b) treating the cells of a) with a drug or therapeutic agent;
c) isolating viable cells; and
d) obtaining information on a desired SNP from the isolated cells.

Here, the desired SNP may be associated with the structure or function of a protein expressed from the target gene.

In another embodiment, a drug sensitization screening method may include:
a) introducing a composition for base substitution into cells having a target nucleic acid sequence;
b) treating the cells of a) with a drug or therapeutic agent;
c) isolating viable cells; and
d) analyzing a nucleic acid sequence of a target gene from the isolated cells.

In another embodiment, a drug sensitization screening method may include:
a) introducing a composition for base substitution into cells having a target nucleic acid sequence;
b) treating the cells of a) with a drug or therapeutic agent;
c) isolating viable cells; and
d) obtaining information on a desired SNP from the isolated cells.

Wherein, the desired SNP may be associated with the structure or function of a protein expressed from the target gene.

The guide RNA library may be a group of one or more guide RNAs complementarily binding to a partial nucleic acid of a target sequence. Although nucleic acids encoding the same guide RNA library are introduced into each cell, the cell may have different guide RNAs. As a result of introduction of nucleic acids encoding the same guide RNA library into each cell, the cell may have the same guide RNA.

The descriptions of the guide RNA have been described above.

The single base substitution protein may be an adenine substitution protein or cytosine substitution protein.

The descriptions of the single base substitution protein, the adenine substitution protein and the cytosine substitution protein have been described above.

The introduction may be performed by one or more methods selected from electroporation, liposomes, plasmids, viral vectors, nanoparticles and a protein translocation domain (PTD)-fused protein.

The drug treated as above may be a material that suppresses or inhibits the activity or function of a protein encoded by a target gene (hereinafter, referred to as a target protein). Here, the material may be a biological material (e.g., RNA, DNA, a protein, a peptide or an antibody) or a non-biological material (e.g., a compound).

The drug treated as above may be a material that promotes or increases the activity or function of a target protein. Here, the material may be a biological material (e.g., RNA, DNA, a protein, a peptide or an antibody) or a non-biological material (e.g., a compound).

The isolated cells may be cells which have considerably changed activity or function of a target protein, that is, an increased drug sensitivity, due to the drug treated in c).

Here, the cells having increased drug sensitivity may be viable cells after drug treatment.

The isolated cells may be cells having modification of at least one nucleotide in a target gene.

Wherein, the modification of one or more nucleotide may be one or more artificial SNPs generated in a target gene.

Wherein, the one or more artificial SNPs may induce a point mutation.

The modification of at least one nucleotide present in a target gene, that is, one or more artificial SNPs may be confirmed. Accordingly, desired information may be obtained.

Here, a nucleic acid sequence including the confirmed modification of at least one nucleotide, that is, one or more artificial SNPs, may be a nucleic acid sequence encoding one region of a protein affecting an increase in drug sensitivity.

### [Fourth use - screening of virus resistance gene or protein]

In another embodiment, a single base substitution protein or a composition for base modification including the same may be used for screening of a virus resistance gene or protein.

In one embodiment, a method of screening a virus resistance gene or protein may include:
a) preparing cells which can express any one or more guide RNAs of one or more guide RNA libraries capable of complementarily binding to a target nucleic acid present in a target gene;
b) introducing a protein for single base substitution or a nucleic acid encoding the same into the cells;
c) treating the cells of b) with a drug or therapeutic agent;
d) isolating viable cells; and
e) analyzing a nucleic acid sequence of the target gene in the isolated cells.

In one embodiment, a method of screening a virus resistance gene or protein may include:
a) preparing cells which can express any one or more guide RNAs of one or more guide RNA libraries capable of complementarily binding to a target nucleic acid present in a target gene, wherein the cells comprise the target nucleic acid sequence;
b) introducing a protein for single base substitution or a nucleic acid encoding the same into the cells;
c) treating the cells of b) with a drug or therapeutic agent;
d) isolating viable cells; and
e) obtaining information on a desired SNP from the isolated cells.

Wherein, the desired SNP may be associated with the structure or function of a protein expressed from the target gene.

In one embodiment, a method of screening a virus resistance gene or protein may include:
a) introducing a protein for single base substitution or a nucleic acid encoding the same, and any one or more guide RNAs of a guide RNA library or nucleic acids encoding the same into cells having a target nucleic acid sequence;
b) treating the cells of a) with a drug or therapeutic agent;
c) isolating viable cells; and
d) obtaining information on a desired SNP from the isolated cells.

Wherein, the desired SNP may be associated with the structure or function of a protein expressed from the target gene.

In another embodiment, a method of screening a virus resistance gene or protein may include:
a) introducing a composition for base substitution into cells having a target nucleic acid sequence;
b) treating the cells of a) with a drug or therapeutic agent;
c) isolating viable cells; and
d) analyzing a nucleic acid sequence of a target gene from the isolated cells.

In another embodiment, a method of screening a virus resistance gene or protein may include:
a) introducing a composition for base substitution into cells having a target nucleic acid sequence;
b) treating the cells of a) with a drug or therapeutic agent;
c) isolating viable cells; and
d) obtaining information on a desired SNP from the isolated cells.

Here, the desired SNP may be associated with the structure or function of a protein expressed from the target gene.

The guide RNA library may be a group of one or more guide RNAs complementarily binding to a partial nucleic acid sequence of a target sequence. Although nucleic acids encoding the same guide RNA library are introduced into each cell, the cell may have different guide RNAs. As a result of introduction of nucleic acids encoding the same guide RNA library into each cell, the cell may have the same guide RNA.

The descriptions of the guide RNA have been described above.

The protein for single base substitution may be a protein for adenine substitution or a protein for cytosine substitution protein.

The descriptions of the protein for single base substitution, the protein for adenine substitution and the protein for cytosine substitution have been described above.

The introduction may be performed by one or more methods selected from electroporation, liposomes, plasmids, viral vectors, nanoparticles and a protein translocation domain (PTD)-fused protein.

The virus treated as above may be introduced into the cells by interacting with a protein encoding a target gene (hereinafter, referred to as a target protein).

The viable cells may be cells which do not interact with the virus treated in c), that is, have virus resistance.

The isolated cells may be cells having the modification of at least one nucleotide in a target gene.

The isolated cells may be cells having the modification of at least one nucleotide in a target gene.

Wherein, the modification of one or more nucleotides may be one or more artificial SNPs generated in a target gene.

Wherein, the one or more artificial SNPs may induce a point mutation.

The modification of at least one nucleotide present in a target gene, that is, one or more artificial SNPs may be confirmed. Accordingly, desired information may be obtained.

Wherein, a nucleic acid sequence including the confirmed modification of at least one nucleotide, that is, one or more artificial SNPs, may be a nucleic acid sequence encoding one region of a protein critical for interaction with a virus.

### One aspect of the present invention disclosed in the specification is a method for single base substitution.

The composition for base substitution may induce or generate artificial modification in base(s) of one or more nucleotides in a gene.

The artificial modification or substitution may be induced or generated by a guide RNA-single base substitution protein complex.

Here, the guide RNA-single base substitution protein complex may be applied to one or more steps of i) targeting a target nucleic acid sequence, ii) cleaving a target nucleic acid sequence, iii) deamination of one or more nucleotides in a target nucleic acid sequence, iv) removal of the deaminated base, and v) repair or recovery of the base-removed target nucleic acid sequence. Here, the steps may be performed sequentially or simultaneously, and the order of the steps may be changed.

### i) Targeting of target nucleic acid sequence

The "target nucleic acid sequence" is a nucleotide sequence present in a target gene or nucleic acid, and specifically, a partial nucleotide sequence of a target region in the target gene or nucleic acid. Here, "target region" is a site which may be modified by the guide RNA-protein for base substitution complex in a target gene or nucleic acid.

Hereinafter, the "target sequence" may be used as a term meaning both types of nucleotide sequence information. For example, in the case of a target gene, a target nucleic acid sequence may refer to sequence information of a transcribed strand of DNA in a target gene, or a nucleotide sequence information of a non-transcribed strand.

For example, the target nucleic acid sequence may refer to 5'-ATCATTGGCAGACTAGTTCG-3' (SEQ ID NO: 17), which is a partial nucleotide sequence of a target region of target gene A (transcribed strand), and 5'-CGAACTAGTCTGCCAATGAT-3' (SEQ ID NO: 18), which is a nucleotide sequence complementary thereto (non-transcribed strand).

The target nucleic acid sequence may be a sequence of 5 to 50 nucleotides.

In one embodiment, the target nucleic acid sequence may be a 16 nt sequence, a 17 nt sequence, an 18 nt sequence, a 19 nt sequence, a 20 nt sequence, a 21 nt sequence, a 22 nt sequence, a 23 nt sequence, a 24 nt sequence or a 25 nt sequence.

The target nucleic acid sequence includes a guide RNA-binding sequence or a guide RNA-non-binding sequence.

The "guide RNA-binding sequence (guide nucleic acid-binding sequence)" is a nucleotide sequence having partial or full complementarity to a guide sequence included in a guide domain of the guide RNA, and is capable of complementary binding to the guide sequence included in the guide domain of the guide RNA. The target nucleic acid sequence and the guide RNA-binding sequence are nucleotide sequences which can be changed according to a target gene or nucleic acid, that is, a target subjected to gene manipulation or modification, and may be designed in various ways depending on a target gene or nucleic acid.

The "guide RNA non-binding sequence (guide nucleic acid-non-binding sequence)" is partially or fully complementary to a guide sequence included in a guide domain of the guide RNA, and may not have complementary bonding with the guide sequence included in the guide domain of the guide RNA. In addition, the guide RNA non-binding sequence is a nucleotide sequence having complementarity to a guide RNA-binding sequence, and may have complementary bonding with the guide RNA-binding sequence.

The guide RNA-binding sequence may be a partial nucleotide sequence of a target nucleic acid sequence, and may be one of two nucleotide sequences having two different sequences of a target nucleic acid sequence, that is, two nucleotide sequences which can complementarily bind to each other. Wherein, the guide RNA non-binding sequence may be a nucleotide sequence other than the guide RNA-binding sequence among the target nucleic acid sequence.

For example, when 5'-ATCATTGGCAGACTAGTTCG-3' (SEQ ID NO: 17), which is a partial nucleotide sequence of a target region of target gene A, and 5'-CGAACTAGTCTGCCAATGAT-3' (SEQ ID NO: 18), which is a nucleotide sequence complementary thereto are used as target nucleic acid sequences, the guide RNA-binding sequence may be one of the two target nucleic acid sequences, for example, 5'-ATCATTGGCAGACTAGTTCG-3' (SEQ ID NO: 17) or 5-CGAACTAGTCTGCCAATGAT-3' (SEQ ID NO: 18). Here, when the guide RNA-binding sequence is 5'-ATCATTGGCAGACTAGTTCG-3' (SEQ ID NO: 17), the guide RNA non-binding sequence may be 5'-CGAACTAGTCTGCCAATGAT-3' (SEQ ID NO: 18), or when the guide RNA-binding sequence is 5'-CGAACTAGTCTGCCAATGAT-3' (SEQ ID NO: 18), the guide RNA non-binding sequence may be 5'-ATCATTGGCAGACTAGTTCG-3' (SEQ ID NO: 17).

The guide RNA-binding sequence may be one nucleotide sequence selected from target nucleic acid sequences, that is, the same nucleotide sequence as a transcribed strand and the same nucleotide sequence as a non-transcribed strand. Here, the guide RNA non-binding sequence may be a nucleotide sequence excluding one nucleotide sequence selected from guide RNA-binding sequences of a target nucleic acid sequence, that is, the same nucleotide sequence as a transcribed strand and the same nucleotide sequence as a non-transcribed strand.

The guide RNA-binding sequence may have the same length as that of the target nucleic acid sequence.

The guide RNA non-binding sequence may have the same length as that of the target nucleic acid sequence or guide RNA-binding sequence.

The guide RNA-binding sequence may be a sequence of 5 to 50 nucleotides.

In one embodiment, the guide RNA-binding sequence may be a 16- nucleotide sequence, a 17 nt sequence, an 18 nt sequence, a 19 nt sequence, a 20 nt sequence, a 21 nt sequence, a 22 nt sequence, a 23 nt sequence, a 24 nt sequence or a 25 nt sequence.

The guide RNA non-binding sequence may be a sequence of 5 to 50 nucleotides.

In one embodiment, the guide RNA non-binding sequence may be a 16- nucleotide sequence, a 17 nt sequence, an 18 nt sequence, a 19 nt sequence, a 20 nt sequence, a 21 nt sequence, a 22 nt sequence, a 23 nt sequence, a 24 nt sequence or a 25 nt sequence.

The guide RNA-binding sequence may have partial or full complementary binding to a guide sequence included in a guide domain of guide RNA, and the length of the guide RNA-binding sequence may be the same as that of the guide sequence.

The guide RNA-binding sequence may be a nucleotide sequence complementary to the guide sequence included in the guide domain of the guide RNA, and for example, a nucleotide sequence which has at least 70%, 75%, 80%, 85%, 90% or 95% complementarity or full complementarity.

In one example, the guide RNA-binding sequence may have or include a sequence of 1 to 8 nucleotides, which is not complementary to the guide sequence included in the guide domain of the guide RNA.

The guide RNA non-binding sequence may have partial or complete sequence homology with the guide sequence included in the guide domain of the guide RNA, and the length of the guide RNA non-binding sequence may be the same as that of the guide sequence.

The guide RNA non-binding sequence may be a nucleotide sequence having homology to the guide sequence included in the guide domain of the guide RNA, and for example, a nucleotide sequence which has at least 70%, 75%, 80%, 85%, 90% or 95% sequence homology, or complete identity.

In one example, the guide RNA non-binding sequence may have or include a sequence of 1 to 8 nucleotides, which is not homologous to the guide sequence included in the guide domain of the guide RNA.

The guide RNA non-binding sequence may complementarily bind to the guide RNA-binding sequence, and have the same length as that of the guide RNA-binding sequence.

The guide RNA non-binding sequence may be a nucleotide sequence complementary to the guide RNA-binding sequence, and for example, a nucleotide sequence which has at least 90% or 95% complementarity or full complementarity.

In one example, the guide RNA non-binding sequence may have or include a sequence of 1 to 2 nucleotides, which is not complementary to the guide RNA-binding sequence.

In addition, the guide RNA-binding sequence may be a nucleotide sequence located near a nucleotide sequence which can be recognized by a CRISPR enzyme.

In one example, the guide RNA-binding sequence may be a sequence of 5 to 50 consecutive nucleotides, which is located adjacent to the 5' terminus and/or the 3' terminus of the nucleotide sequence which can be recognized by the CRISPR enzyme.

In addition, the guide RNA non-binding sequence may be a nucleotide sequence located near a nucleotide sequence which can be recognized by a CRISPR enzyme.

In one example, the guide RNA non-binding sequence may be a sequence of 5 to 50 consecutive nucleotides, which is located adjacent to the 5' terminus and/or the 3' terminus of the nucleotide sequence which can be recognized by the CRISPR enzyme.

The "targeting" refers to complementary binding to a guide RNA-binding sequence among target nucleic acid sequences present in a target gene or nucleic acid. Here, the complementary binding may be 100% complete complementary binding, or 70 or more and less than 100% incomplete complementary binding. Therefore, the "targeting gRNA" refers to gRNA complementarily binding a guide RNA-binding sequence among target nucleic acid sequences present in a target gene or nucleic acid.

The guide RNA-protein for single base substitution complex may target a target nucleic acid sequence.

### ii) cleaving a target nucleic acid sequence

The guide RNA-single base substitution protein complex may cleave a target nucleic acid sequence.

Here, when the target nucleic acid sequence is a double-stranded nucleic acid, the cleavage may be cleaving both of the double strands. Alternatively, the cleavage may be cleaving one of the double strands.

Here, when the target nucleic acid sequence is a single-stranded nucleic acid, the cleavage may be cleavage of a single strand.

Alternatively, a cleavage form of the cleavage of the target nucleic acid sequence may be changed according to the type of CRISPR enzyme constituting a guide RNA-single base substitution protein complex.

For example, when the CRISPR enzyme constituting the guide RNA-single base substitution protein complex is a wild-type CRISPR enzyme (e.g., SpCas9), the cleavage of the target nucleic acid sequence may be cleavage of both of the double strands of the target nucleic acid sequence.

In another example, when the CRISPR enzyme constituting the guide RNA-single base substitution protein complex is a nickase (e.g., Nureki nCas9), the cleavage of the target nucleic acid sequence may be cleavage of one of the double strands of the target nucleic acid sequence.

### iii) deamination of one or more nucleotides in a target nucleic acid sequence

The guide RNA-single base substitution protein complex may deaminate an amino (-NH₂) group of base(s) of one or more nucleotides in a target nucleic acid sequence.

Here, the deamination may occur at a cytosine or adenine base.

For example, when there are five nucleotides having adenine in a target nucleic acid sequence (here, the five nucleotides may or may not be consecutive), the guide RNA-single base substitution protein complex may deaminate all of the amino (-NH₂) groups of adenines in the five nucleotides with adenine.

In another example, when there are eight nucleotides having cytosine in a target nucleic acid sequence (here, the five nucleotides may or may not be consecutive), the guide RNA-single base substitution protein complex may deaminate the amino (-NH₂) group of cytosines in three of the 8 nucleotides with cytosine.

A deaminated base may vary according to the type of deaminase constituting the guide RNA-single base substitution protein complex.

For example, when the deaminase constituting the guide RNA-single base substitution protein complex is adenosine deaminase (e.g., a TadA or TadA variant), the deamination may occur at adenine. Here, as the amino (-NH₂) group of adenine is deaminated, a keto (=O) group may be formed. Hypoxanthine may be generated by deamination of the adenine.

In another example, when the deaminase constituting the guide RNA-single base substitution protein complex is cytidine deaminase (e.g., an APOBEC1 or APOBEC1 variant), the deamination may occur at cytosine. Here, when the amino (-NH₂) group of cytosine is deaminated, a keto (=O) group may be formed. Uracil may be generated by deamination of the cytosine.

### iv) Removal of the deaminated base

The guide RNA-single base substitution protein complex may remove the deaminated base generated in step iii). Here, the removal of the deaminated base may remove all or a part of the deaminated bases generated in step iii).

Here, the deaminated base may be deaminated cytosine or adenine.

Here, the deaminated base may be uracil or hypoxanthine.

The removal of the deaminated base may vary according to the type of DNA glycosylase constituting the guide RNA-single base substitution protein complex.

For example, when the DNA glycosylase constituting the guide RNA-single base substitution protein complex is alkyladenine DNA glycosylase (AAG) or an AAG variant, an N-glycoside linkage connecting deoxyribose or ribose and a base (deaminated adenine or hypoxanthine) constituting a nucleotide may be hydrolyzed. In addition, an AP site (apurinic/apyrimidinic site) may be formed. The AP site may be located in DNA (or RNA) without a purine or pyrimidine base either spontaneously or due to DNA (or RNA) damage.

In another example, when the DNA glycosylase constituting the guide RNA-single base substitution protein complex is uracil DNA glycosylase (UDG or UNG) or a UDG variant, an N-glycoside linkage connecting deoxyribose or ribose and a base (deaminated cytosine or uracil) constituting a nucleotide may be hydrolyzed. In addition, an AP site (apurinic/apyrimidinic site) may be formed.

### v) ) repair or recovery of the base-removed target nucleic acid sequence

The repair or recovery of a base-removed target nucleic acid sequence includes the repair or recovery of a target nucleic acid sequence following cleavage.

The base-removed target nucleic acid sequence may be a cleaved target nucleic acid sequence.

Wherein, the cleaved target nucleic acid sequence may be a target nucleic acid sequence in which both double strands are cleaved.

Wherein, the cleaved target nucleic acid sequence may be a target nucleic acid sequence in which one of the double strands is cleaved. Wherein, the cleaved strand may be a base-removed strand. Alternatively, the cleaved strand may be a strand from which a base is not removed.

The repair or recovery of a base-removed target nucleic acid sequence may be the repair or recovery with any base, that is, adenine, cytosine, guanine, thymine or uracil at an AP site of one or more base-removed nucleotides in the target nucleic acid sequence.

For example, the AP site of one or more deaminated adenine-removed nucleotides in the target nucleic acid sequence may be repaired to guanine. Alternatively, the AP site of one or more deaminated adenine-removed nucleotides in the target nucleic acid sequence may be repaired to cytosine. The AP site of one or more deaminated adenine-removed nucleotides in the target nucleic acid sequence may be repaired to thymine. The AP site of one or more one or more deaminated adenine-removed nucleotides in a target nucleic acid sequence may be repaired to uracil. The AP site of one or more deaminated adenine-removed nucleotides in a target nucleic acid sequence may be repaired to adenine.

In another example, the AP site of one or more deaminated cytosine-removed nucleotides in the target nucleic acid sequence may be repaired to adenine. Alternatively, the AP site of one or more deaminated cytosine-removed nucleotides in the target nucleic acid sequence may be repaired to guanine. Alternatively, the AP site of one or more deaminated cytosine-removed nucleotides in the target nucleic acid sequence may be repaired to thymine. Alternatively, the AP site of one or more deaminated cytosine-removed nucleotides in the target nucleic acid sequence may be repaired to uracil. Alternatively, the AP site of one or more deaminated cytosine-removed nucleotides in the target nucleic acid sequence may be repaired to cytosine.

The artificial modification may occur at an exon or intron of a gene, a splicing site, a regulatory region (an enhancer, or suppressor region), the 5' terminus or an adjacent region thereof, or the 3' terminus or an adjacent region thereof.

For example, the artificial modification may be substitution of one or more bases in an exon region. For example, one or more As and/or Cs may be substituted with a different base (A, C, T, G or U) in the exon region of a gene.

In another example, the artificial modification may be substitution of one or more bases in an intron region. For example, one or more As and/or Cs may be substituted with a different base (A, C, T, G or U) in the intron region of a gene.

For example, the artificial modification may substitution of one or more bases at a splicing site. For example, one or more As and/or Cs may be substituted with a different base (A, C, T, G or U) at the splicing site of a gene.

In another example, the artificial modification may be substitution of one or more bases in a regulatory region (an enhancer or a suppressor region). For example, one or more As and/or Cs may be substituted with a different base (A, C, T, G or U) in the regulatory region (an enhancer or a suppressor region).

The artificial modification may be modification of a codon sequence of a gene encoding a protein.

The "codon" refers to one of genetic codes encoding an amino acid from a gene. When DNA is transcribed into messenger RNA (mRNA), three nucleotides of such mRNA form each codon. A codon may encode one type of amino acid, or a stop codon that terminates amino acid synthesis.

The artificial modification may be modification of a codon sequence encoding a protein by one or more single base modifications, and the modified codon sequence may encode the same amino acid or a different amino acid.

For example, when one or more nucleic acid sequences are changed from C to T, a codon of CCC encoding proline may be changed to CUU or CUC encoding leucine, UCC or UCU encoding serine, or UUC or UUU encoding phenyl-alanine.

For example, when one or more bases are changed from A to C, ACC or ACA encoding threonine may be changed to CCC or CCA encoding proline.

For example, when one or more bases are changed from A to G, a codon of AAA encoding Lysine may be changed to GAA or GAG encoding glutamic acid, GGA or GGG encoding glycine, or AGA or AGG encoding arginine.

### [Examples]

Hereinafter, the present invention will be described in further detail with reference to examples.

Hereinafter, the present invention will be described in further detail with reference to examples. The examples are merely provided to more specifically describe the present invention, and it will be obvious to those of ordinary skill in the art that the scope of the present invention is not limited to the examples according to the gist of the present invention.

### Experimental methods

### [Example 1]

### Example 1-1: Plasmid construction

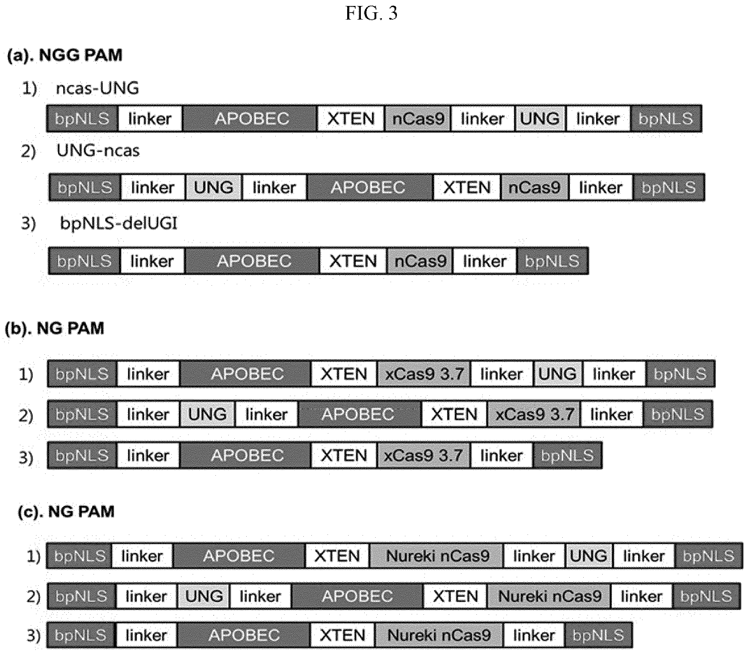

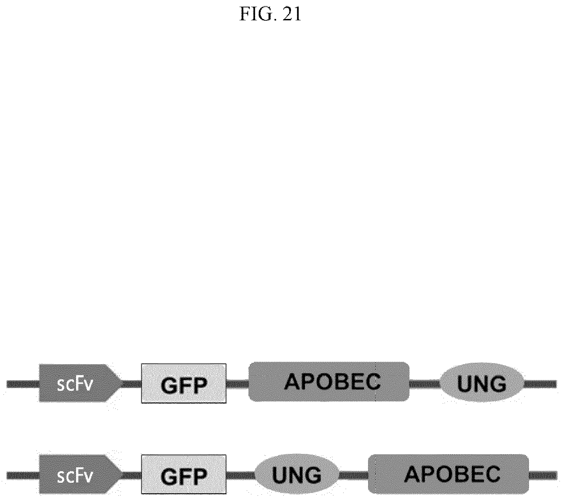

Plasmids were constructed using Gibson Assembly (NEBuilder HiFi DNA Assembly kit, NEB). After each of fragments of FIGS. 3(a), 7(a) and 21 was amplified by PCR, a DNA fragment amplified by PCR was added to the Gibson Assembly Master mix, and incubated at 50 °C for 60 minutes. All plasmids include a CMV promoter, a p15A replication origin, and a selection marker for an ampicillin resistance gene. Some plasmids include human codon-optimized WT-Cas9 (P3s-Cas9HC; Addgene plasmid #43945) or a variant thereof.

### Example 1-2: Cell culture and transfection

### (1) HEK293T cells: single base substitution CRISPR protein transfection

HEK293T cells were incubated in a Dulbecco's Modified Eagle's medium (DMEM, Welgene) supplemented with 10% FBS and 1% antibiotic in 5% CO₂ at 37 °C. Before transfection, the HEK293T cells were dispensed into a 6-well plate at a density of 2x10⁵ cells per well. Subsequently, 1 µg ofBE3 (WT, bpNLS, xCas-UNG, UNG-xCas, scFv-APO-UNG or scFv-UNG-APO) and 1 µg of sgRNA-expression plasmids (hEMX1 GX19 or GX20) were transfected in 200 µl of an Opti-MEM medium using 4 uL of Lipofectamine^{™} 2000 (Thermo Fisher Scientific, 11668019).

### (2) Hela cells: single base substitution CRISPR protein transfection

Hela cells were incubated in a Dulbecco's Modified Eagle's medium (DMEM, Welgene) supplemented with 10% FBS and 1% antibiotic in 5% CO₂ at 37 °C. Before transfection, the Hela cells were dispensed into a 6-well plate at a density of 2x10⁵ cells per well. Subsequently, 1 µg of base substitution plasmids (BE3 WT, bpNLS BE3, ung-ncas, ncas-ung or ncas-delta UNG) and 1 µg of sgRNA-expression plasmids were transfected in 200 µl of an Opti-MEM medium using 4 uL of Lipofectamine^{™} 2000 (Thermo Fisher Scientific, 11668019).

### (3) HEK293T cells: single base substitution CRISPR protein transfection

HEK293T cells were incubated in a Dulbecco's Modified Eagle's medium (DMEM, Welgene) supplemented with 10% FBS and a 1% antibiotic in 5% CO₂ at 37 °C. Before transfection, the HEK293T cells were dispensed into a 6-well plate at a density of 2x10⁵ cells per well. Subsequently,500 ng of base substitution plasmids (bpNLS-UNG-APOBEC-Nureki nCas9-bpNLS), 500 ng of sgRNA-expression plasmids (hEMX1 GX19 or GX20) were transfected in a 200 µl of an Opti-MEM medium using 2 uL of Lipofectamine^{™} 2000 (Thermo Fisher Scientific, 11668019).

### Example 1-3: Design and synthesis of hEMX1 GX19 sgRNA, hEMX1 GX20 sgRNA

### (1) Design and synthesis of sgRNA

Guide RNA considering "NGG PAM" or "NG" PAM of a hEMX gene was designed using CRISPR RGEN tools ((http://www.rgenome.net; Park et al, Bioinformatics 31:4014-4016, 2015). The designed guide RNA was considered not to have a 1-base or 2-base mismatch except for an on-target site.

After oligonucleotides (see Table 1) used to generate sgRNA expression plasmids were annealed and elongated, and they were cloned into a Bsa1 site of a pRG2 plasmid.

**[Table 2]**

| **sgRNA name** | **sequence** |
|---|---|
| GX19 | GAGTCCGAGCAGAAGAAGAA (SEQ ID NO. 39) |
| GX20 | TGCCCCTCCCTCCCTGGCCC (SEQ ID NO. 40) |
| Nureki sgRNA 1 | GAGGACAAAGTACAAACGGC (SEQ ID NO. 41) |
| Nureki sgRNA 2 | GGGCTCCCATCACATCAACC (SEQ ID NO. 42) |
| Nureki sgRNA 3 | GGCCCCAGTGGCTGCTCTGG (SEQ ID NO. 43) |
| Nureki sgRNA 4 | GCTTTACCCAGTTCTCTGGG (SEQ ID NO. 44) |

### (2) Deep sequencing

Using HiPi Plus DNA polymerase (Elpis-Bio), on-target and off-target sites were amplified by PCR to a size of 200 to 300 bp. A PCR product obtained by the above method was sequenced using a MiSeq (Illumina) device and analyzed using a Cas analyzer provided from CRISPR RGEN Tools (www.rgenome.net). Substitution within 5 bp from a CRISPR/Cas9 cleavage site was considered a mutation induced from a single base substitution CRISPR protein.

### Example 1-4: Experimental results

Using the single base substitution CRISPR protein according to this example, an effect of substituting cytosine (C) with adenine (A), thymine (T) or guanine (G) was confirmed.

### (1) bpNLS verification

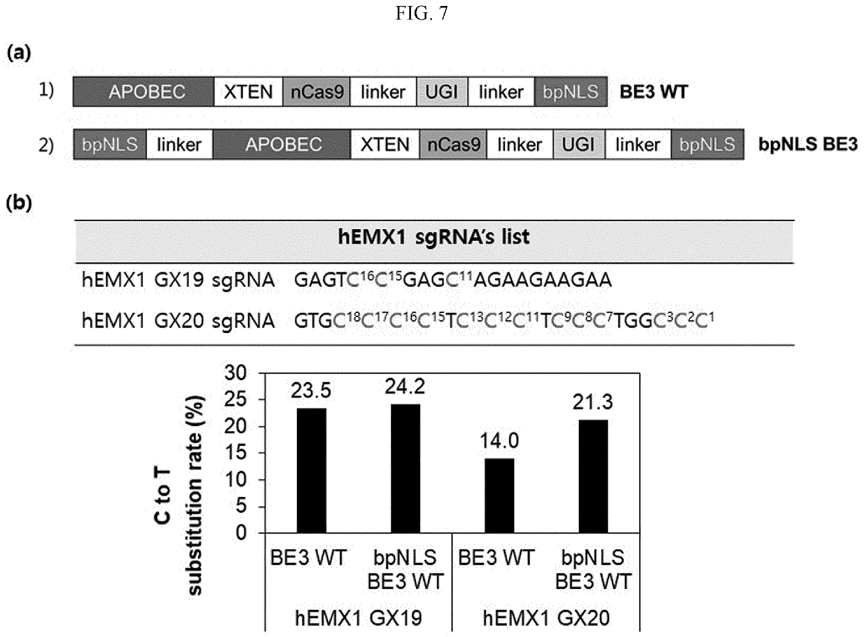

It was confirmed that bpNLS BE3 WT increased a C to T substitution rate compared to BE3 WT using BE3 WT and bpNLS BE3 WT in HEK cells (see FIG. 7B).

### (2) Confirmation of base substitution efficiency of single base substitution CRISPR protein

### 1) Confirmation of C to N (A, T, G) efficiency in Hela cells

C to N substitution rate in a hEMX1 GX19 sgRNA target was confirmed using the single base substitution CRISPR protein in Hela cells.

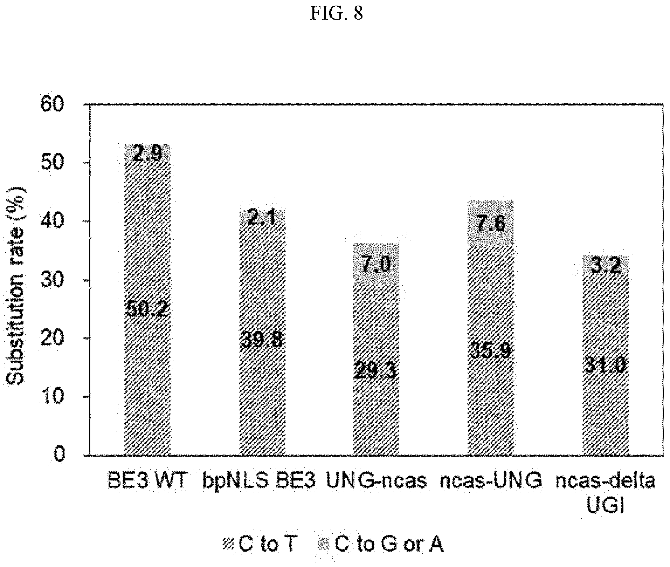

As an experimental result, it was confirmed that UGI-removed ncas-delta UGI has almost no difference in a C to G or C to A substitution rate from BE3 WT. However, it was confirmed that, compared to BE3 WT, substitution rate of C to G or C to A of UNG-fused UNG-ncas and ncas-UNG were increased (see FIG. 8). From this result, it was confirmed that, when UGI is substituted with UNG in BE3 WT, the probability of C to G or C to A substitution increases.

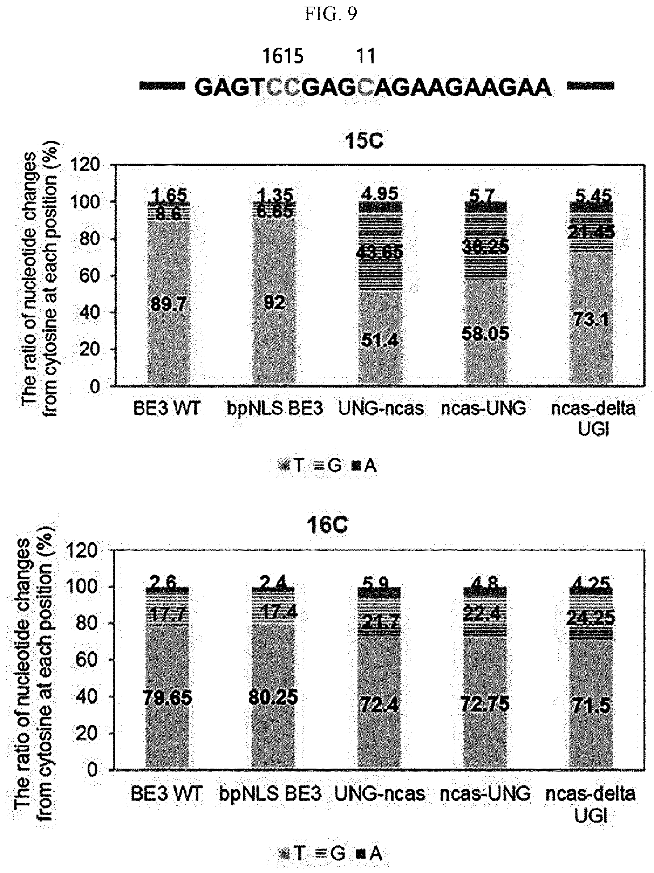

In addition, in a hEMX1 GX19 sgRNA sequence, a substitution rate of 15C or 16C was confirmed. As an experimental result, compared to BE3 WT or bpNLS BE3, it was confirmed that UNG-ncas or ncas-UNG had an increased probability of C to G or C to A substitution at 15C or 16C (see FIG. 9).

It was confirmed that, in the hEMX1 GX19 sgRNA sequence, C to G or C to A substitution more easily occurs at 15C than 16C, and in the single base substitution CRISPR protein having an UNG-ncas structure, the probability of C to G or C to A substitution is the highest (see FIG. 9).

### 2) Confirmation of C to N (A, T, G) efficiency in HEK cells

C to N substitution rate of the single base substitution CRISPR protein was confirmed using a hEMX1 GX20 sgRNA target in HEK cells.

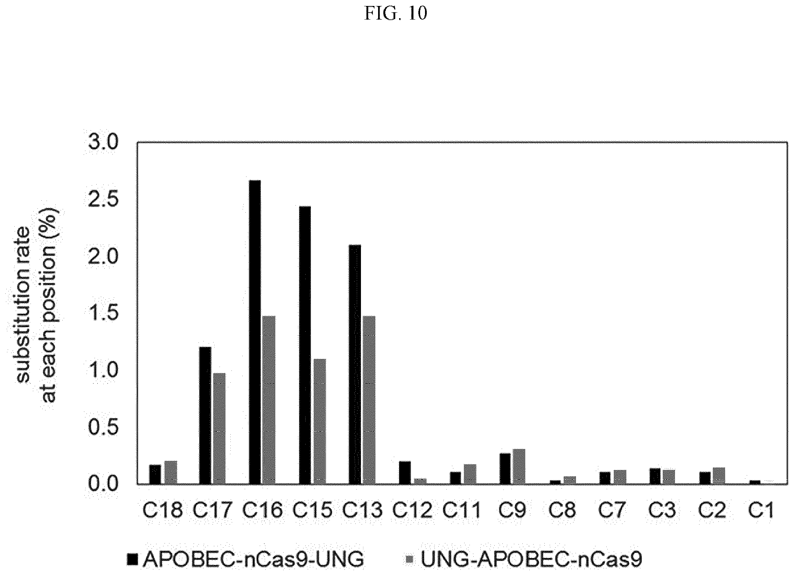

As an experimental result, it was confirmed that base substitution occurs at 13C, 15C, 16C and 17C in the hEMX1 GX19 sgRNA sequence (see FIG. 10).

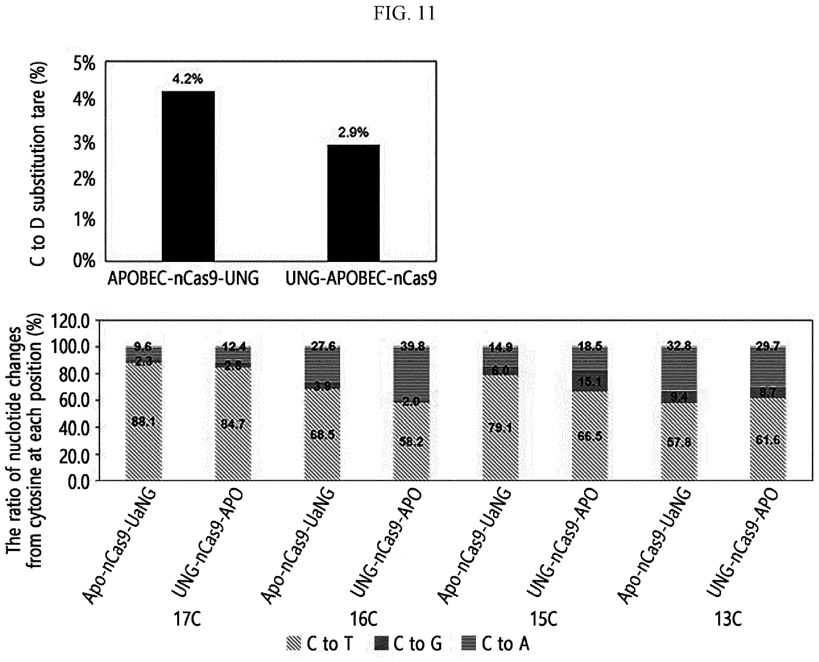

In addition, it was confirmed that ncas-UNG is increased in C to N substitution rate compared to UNG-ncas in HEK cells (see FIG. 11). Particularly, it was confirmed that C to G or C to A base substitution more easily occurs in UNG-ncas than ncas-UNG at 15C, 16C and 17C (see FIG. 11).

In addition, as a result of confirming the single base substitution efficiency in a hEMX1 target nucleic acid sequence using a single base substitution CRISPR protein complex, that is, a fused base substitution domain (scFv-APO-UNG or scFv-UNG-APO) having a single chain variable fragment (scFv), it was confirmed that base substitution from C to A more easily occurs at 11C, and base substitution from C to G more easily occurs at 15C and 16C (see FIGS. 22 to 24).

### (3) Nureki nCas9 verification

To widen a target site capable of giving a random error using a single base substitution CRISPR protein, an experiment was performed using Nureki nCas9 having an NG PAM sequence.

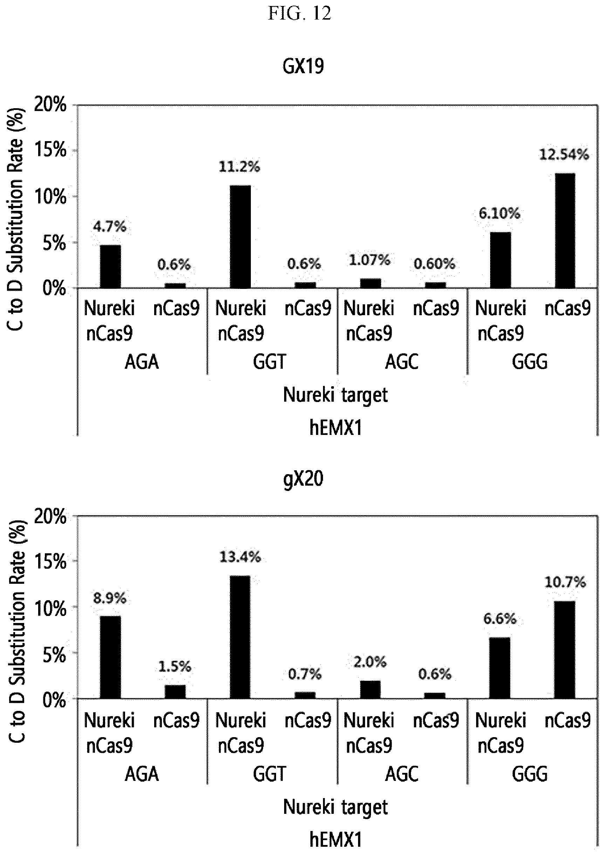

As a result of performing the experiment using hEMX1 GX17 sgRNA and hEMX1 GX20 sgRNA, it was confirmed that they work well in HEK cells. Particularly, it was confirmed that C to N substitution occurs in NG PAM (see FIG. 12).

### [Example 2]

### Example 2-1: Plasmid construction

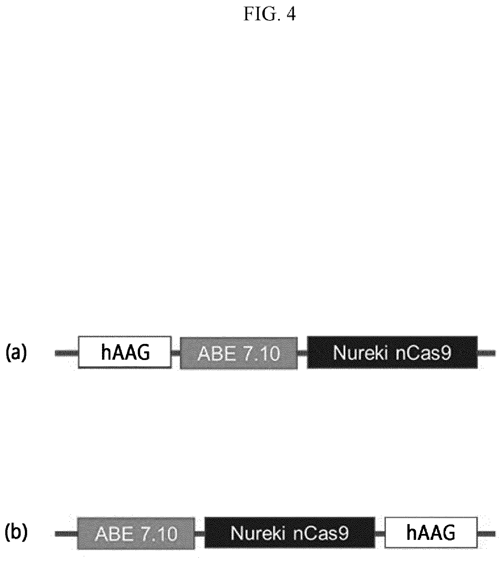

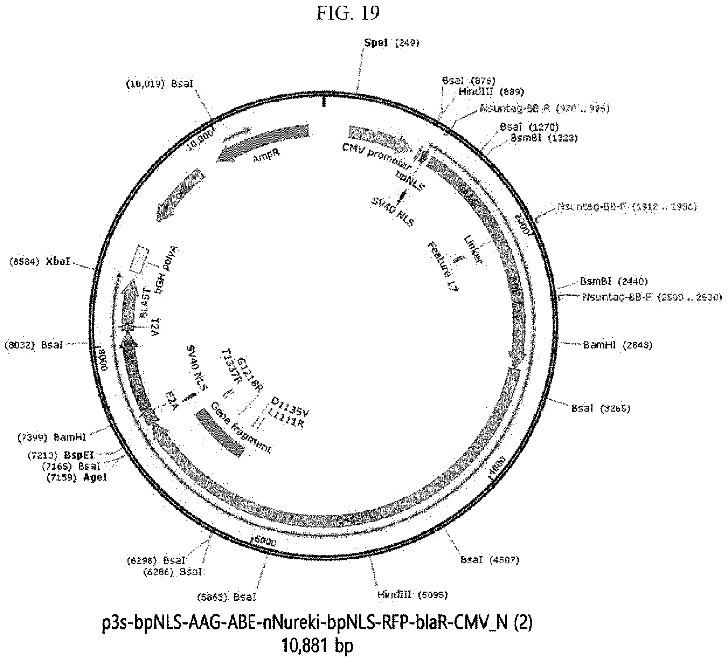

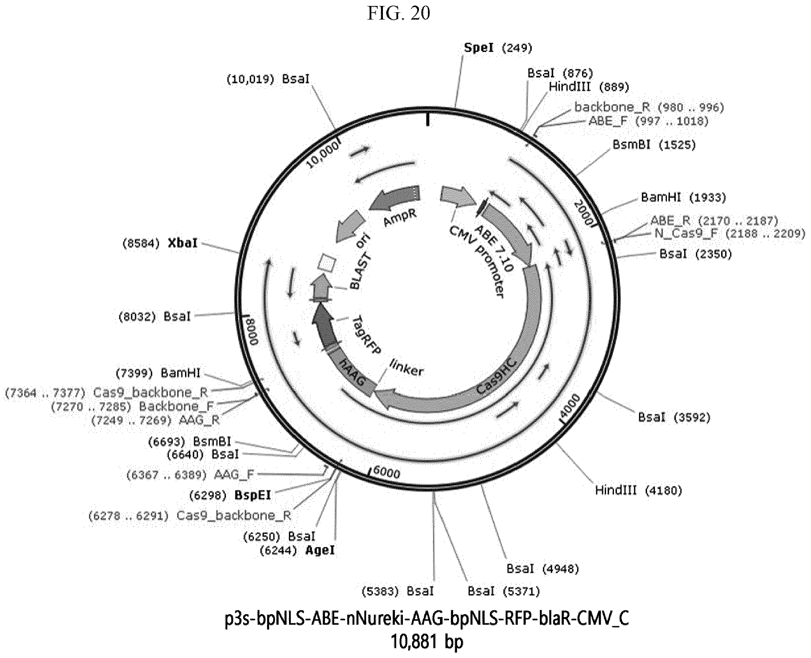

Plasmids were constructed using Gibson Assembly (NEBuilder HiFi DNA Assembly kit, NEB). After each fragment of FIG. 4 was amplified using PCR, the DNA fragment amplified by PCR was added to the Gibson Assembly Master mix, and incubated at 50 °C for 60 minutes. All plasmids include human codon-optimized WT-Cas9 (P3s-Cas9HC; Addgene plasmid #43945), a CMV promoter, a p15A replication origin and a selection marker for an ampicillin resistance gene (see FIGS. 19 and 20).

### Example 2-2: Design and synthesis of sgRNA

### (1) Design of sgRNA

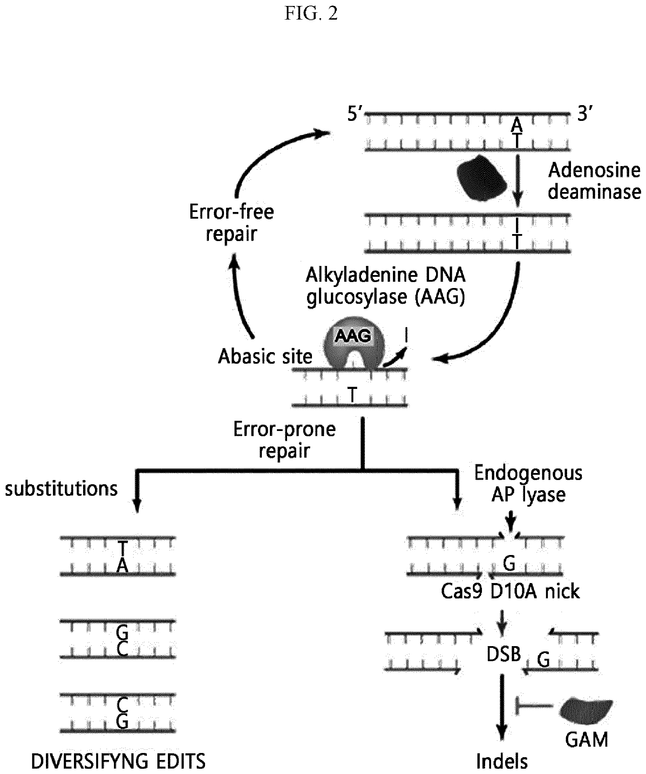

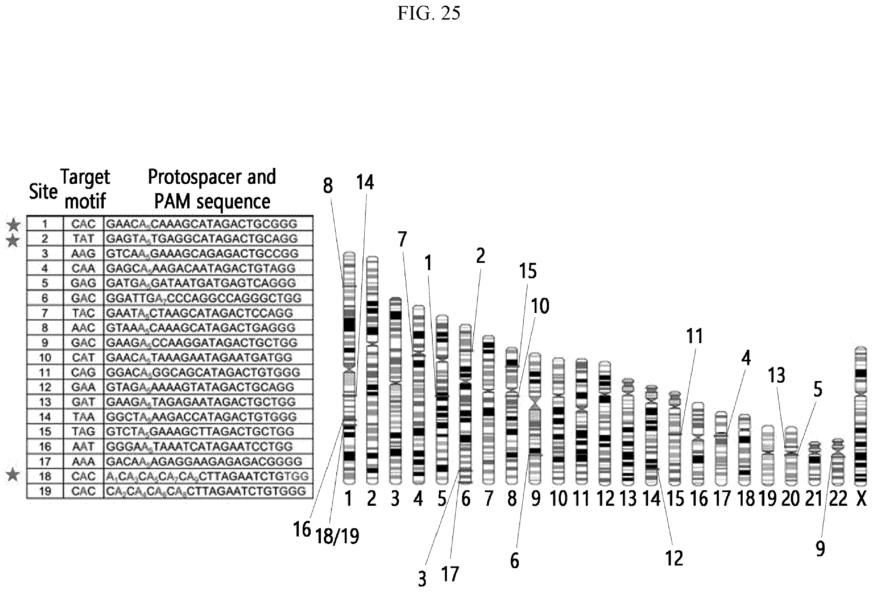

Three of sgRNAs shown in Extended Data FIG. 2 in the article, titled "Base editing of A, T to C, G in genomic DNA without DNA cleavage" disclosed in the science journal "Nature" were selected (see FIG. 25).

### (2) Synthesis of sgRNA

Two complementary oligonucleotides were annealed and extended to PCR-amplify templates for sgRNA synthesis.

*In vitro* transcription was performed using T7 RNA polymerase (New England Biolabs) for template DNA (excluding "NGG" of the 3' terminus in a target sequence), RNA was synthesized according to the manufacturer's protocol, and then the template DNA was removed using Turbo DNAse (Ambion). Transcribed RNA was purified using an Expin Combo kit (GeneAll) and isopropanol precipitation.

In this example, the chemically synthesized sgRNA used herein was modified with 2'OMe and phosphorothioate.

### Example 2-3: Cell culture and transfection

### (1) HEK293T cells: single base substitution CRISPR protein transfection

HEK293T cells were incubated in a Dulbecco's Modified Eagle's medium (DMEM, Welgene) supplemented with 10% FBS and 1% antibiotic in 5% CO₂ at 37 °C. Before transfection, the HEK293T cells were dispensed into a 24-well plate at a density of 5×10⁴ cells per well. Subsequently, 1 µg each of three different sgRNA expression plasmids was transfected with 3 µg of ABE (WT, N-AAG or C-AAG) in 200 µl of an Opti-MEM medium using 12 uL of a Fugene^{®} HD transfection reagent (Cat no. E231A, Promega).

### (2) Deep sequencing

On-target and off-target sites were PCR-amplified to a size of 200 to 300 bp using HiPi Plus DNA polymerase (Elpis-Bio). A PCR product obtained by the above method was sequenced using a MiSeq (Illumina) device and analyzed using a Cas analyzer provided by CRISPR RGEN Tools (www.rgenome.net). Substitution within 5 bp from a CRISPR/Cas9 cleavage site was considered a mutation induced from a single base substitution CRISPR protein.

### Example 2-4: Experimental results

An adenine base editor (ABE) refers to adenine-repairing genetic scissors, and is a technology for substituting adenine (A) with guanine (G). Alkyladenine DNA glycosylase (AAG) is an enzyme that removes an inosine base from DNA (FIG. 2). The inventors developed an adenine base substitution protein by inserting the AAG gene at each of the N-terminus and the C-terminus of an ABE WT plasmid to induce a random mutation of adenine (A). A fused protein was produced with Cas9 nickase, adenosine deaminase and DNA glycosylase in various orders (FIG. 4).

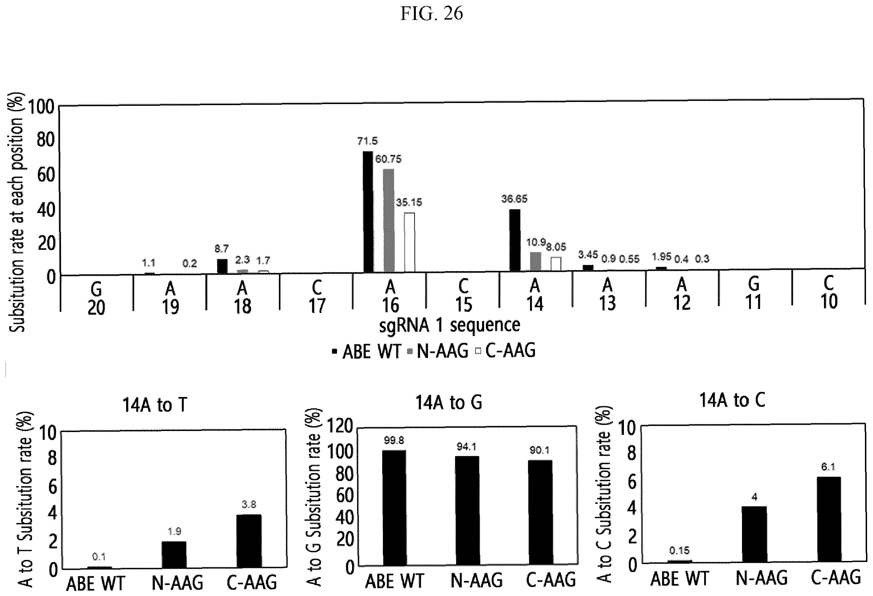

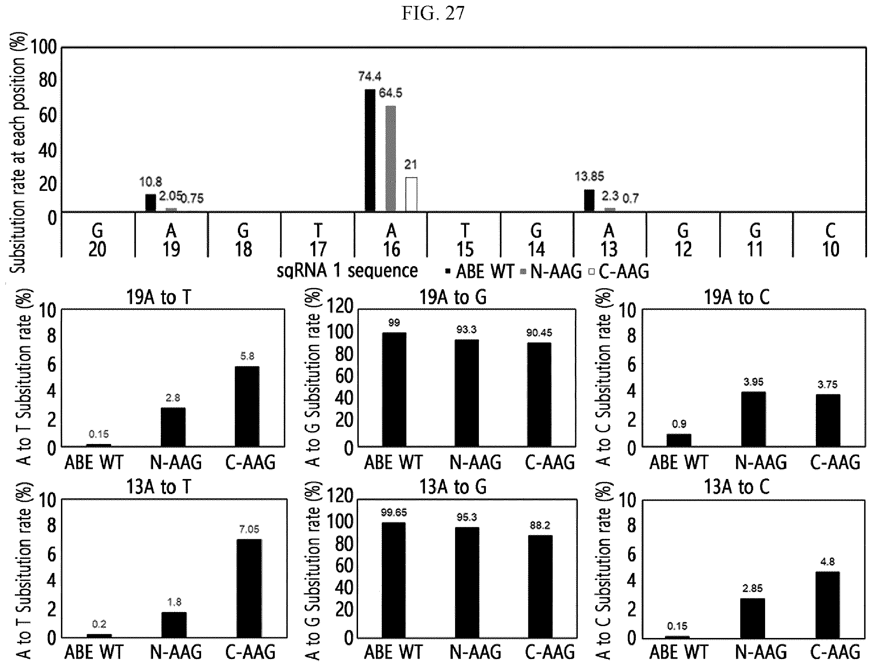

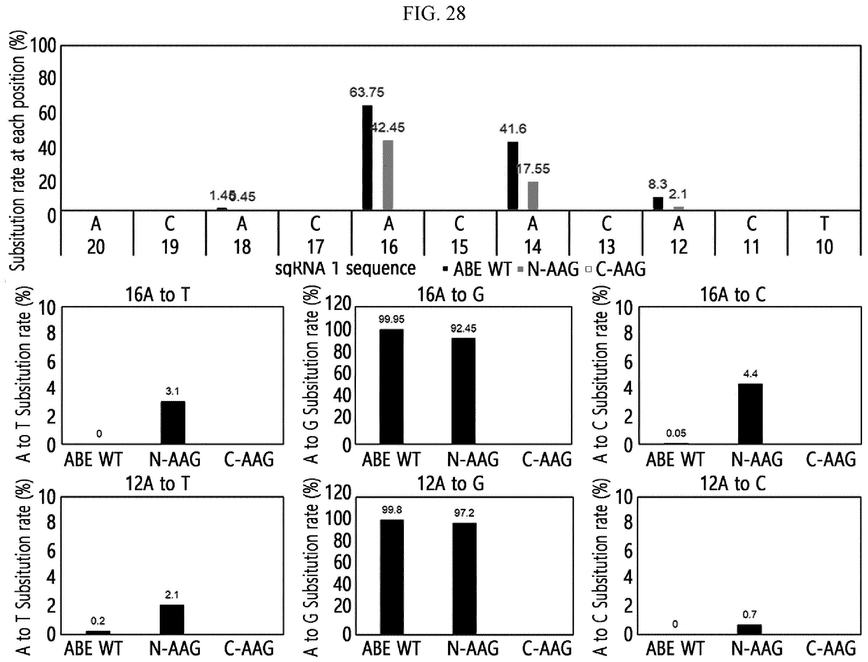

To confirm a random mutation of adenine (A), three sgRNAs (sgRNA1, sgRNA2 and sgRNA3) were transfected into HEK 293T cells along with a plasmid having a nucleic acid encoding a base substitution protein (i.e., a modified ABE plasmid). As a result of the experiment, compared to ABE WT, it was confirmed that adenine (A) 14 in the base sequence of sgRNA 1 is randomly substituted with a different base (thymine, T; cytosine, C; or guanine, G) in HEK293T cells transfected with modified ABE plasmids (N-AAG and C-AAG). It was confirmed that adenines (A) 19 and 13 in the base sequence of sgRNA 1 are substituted with different bases (FIG. 27), and adenines 16 and 12 are substituted in sgRNA 1 only in a plasmid in which AAG is inserted into the N-terminus (FIG. 28). Accordingly, it was confirmed that the random substitution of adenine (A) with a different base is induced by inserting AAG into ABE. Moreover, when an adenine substitution protein is used regardless of the order of Cas9 nickase, adenosine deaminase and DNA glycosylase, it was confirmed that random substitution of adenine (A) with a different base is induced (see FIGS. 26 to 28).

### [Example 3]

### Single base substitution using SunTag system

### Example 3-1: Plasmid construction

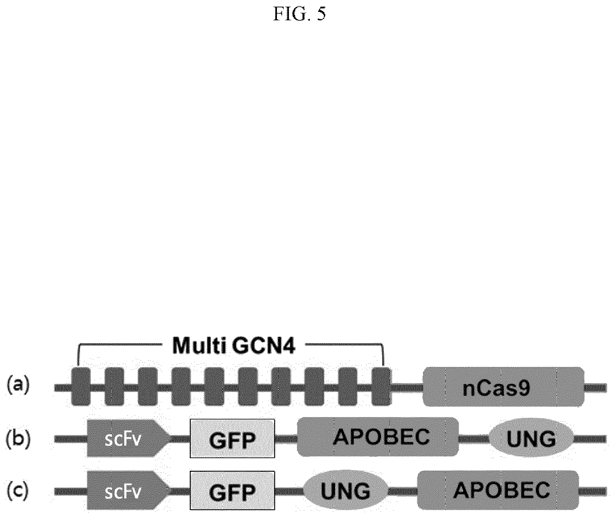

Plasmids were constructed using Gibson Assembly (NEBuilder HiFi DNA Assembly kit, NEB). After each of the fragments of FIGS. 5(a), (b) and (c) was amplified by PCR, the DNA fragment amplified by PCR was added to the Gibson Assembly Master mix, and incubated at 50 °C for 15 to 60 minutes. All plasmids include human codon-optimized WT-Cas9 (P3s-Cas9HC; Addgene plasmid #43945), a CMV promoter, a p15A replication origin and a selection marker for an ampicillin-resistant gene.

### Example 3-2: Cell culture and transfection

PC9 cells were incubated in a Rosewell Park Memorial Institute 1640 (RPMI 1640, Welgene) supplemented with 10% FBS and 1% antibiotic in 5% CO₂ at 37 °C. Before transfection, the PC9 cells were dispensed into a 24-well plate at a density of 2x10⁵ cells per well. Subsequently, 1500 ng each of base substitution plasmids (Apobec-nCas9-UGI and Apobec-nureki nCas9-UNG) and 500 ng of a sgRNA-expression plasmid (hEMX1 GX19); 1000 ng of a SunTag plasmid (GCN4-nCas9) and 1000 ng each of ScFv plasmids (ScFv-Apobec-UNG and ScFv-UNG-Apobec); or 500 g of a sgRNA-expression plasmid (hEMX1 GX19) was transfected in 200 µl of Opti-MEM medium using 4 µL of Lipofectamine^{™} 2000 (Thermo Fisher Scientific, 11668019).

### Example 3-3: Deep sequencing

Using HiPi Plus DNA polymerase (Elpis-Bio), on-target and off-target sites were amplified by PCR to a size of 200 to 300 bp. A PCR product obtained by the above method was sequenced using a MiSeq (Illumina) device and analyzed using a Cas analyzer provided from CRISPR RGEN Tools (www.rgenome.net). Substitution within 10 bp from a sgRNA sequence region was considered a mutation induced from a single base substitution CRISPR protein.

### Example 3-4: Experimental results

C to N substitution rate was confirmed using a single base substitution protein in PC9 cells.

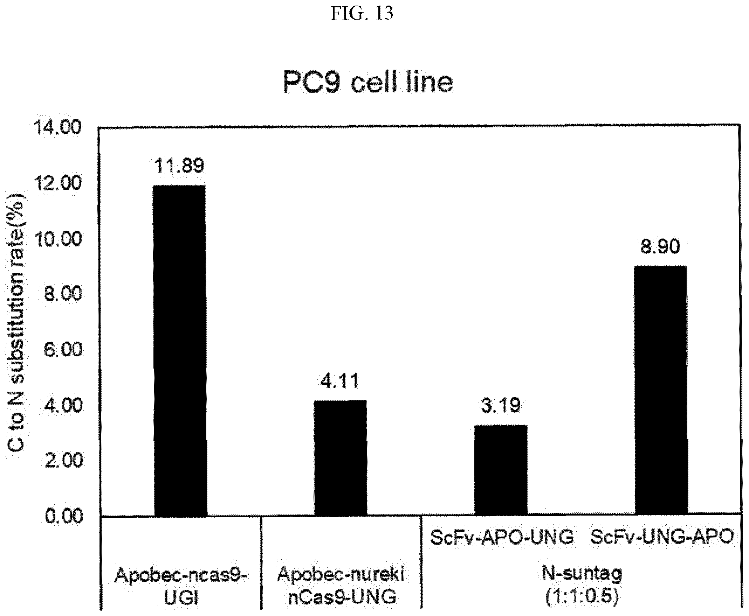

The induction of a random mutation was increased by maximizing a UNG effect only with one nCas9 using a SunTag system. As a result, it was confirmed that ScFv-UNG-Apobec can have similar single base substitution efficiency to WT and induce random base substitution (C to T or A or G) (see FIG. 13).

### [Example 4]

### Induction of EGFR C797S mutation using single base substitution CRISPR protein and confirmation of osimertinib resistance

### Example 4-1: PC9 cells: transduction of single base substitution CRISPR protein and drug culture

PC9 cells were incubated in Rosewell Park Memorial Institute 1640 (RPMI 1640, Welgene) supplemented with 10% FBS and 1% antibiotic in 5% CO₂ at 37 °C. Before transfection, the PC9 cells were dispensed in a 15-cm² dish at a density of 3×10⁶ cells per well. Subsequently, 5 µg each of two different sgRNA expression plasmids was transfected with 15 µg of N-UNG in 3 mL Opti-MEM medium, using 40 µL of Lipofectamine^{™} 2000 (Thermo Fisher Scientific, 11668019). Three days after transfection, the plasmid was treated with 4 µg/mL of blasticidin for 7 days. After a stabilized cell line was obtained through sufficient antibiotic culture, the cells were treated with 100 nM osimertinib (Selleckchem, S5078), which is a targeted therapeutic agent for non-small cell lung cancer, for 20 days. A positive control experiment was performed using sgRNA (C797S sgRNA 1 (SEQ ID NO: 21) and C797S sgRNA 2 (SEQ ID NO: 22)) capable of producing C797S mutants known to have osimertinib resistance. It was confirmed that the C797S mutants are enriched using a screening system.

### Example 4-2: Deep sequencing

On-target and off-target sites were PCR-amplified to a size of 200 to 300 bp using HiPi Plus DNA polymerase (Elpis-Bio). A PCR product obtained by the above method was sequenced using a MiSeq (Illumina) device and analyzed using a BE Analyzer provided by CRISPR RGEN Tools (www.rgenome.net). Substitution within 10 bp from a sgRNA sequence site was considered a mutation induced from a single base substitution CRISPR protein.

### Example 4-3: Experimental results

Osimertinib, which is a third-generation EGFR tyrosine kinase inhibitor (TKI), is being used as a therapeutic agent for patients with EGFR T790M-positive non-small cell lung cancer, who are resistant to a second-generation drug. Mutants resistant to a specific drug were screened by inducing random base substitution of cytosine in a target sgRNA sequence by N-UNG.

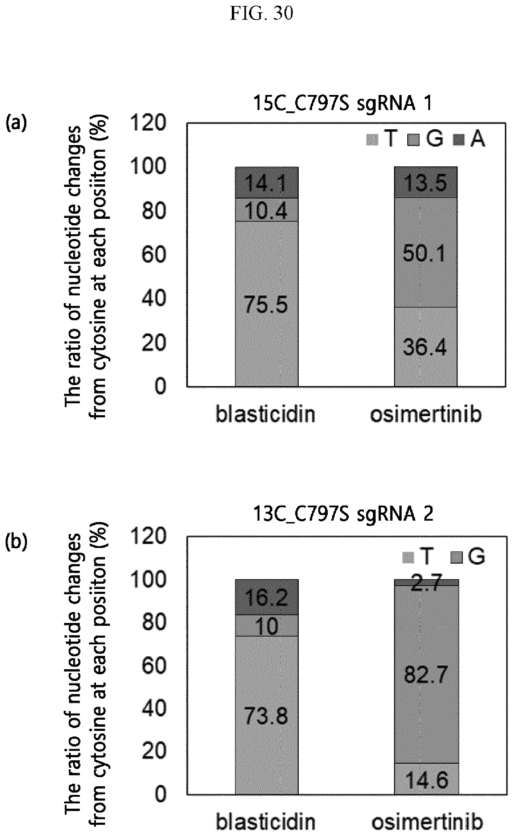

By using a known mutant resistant to osimertinib, C797S, as a positive control, it was confirmed that a corresponding tool works. When base substitution of C15 to G in C797S sgRNA1 or C13 to G in C797S sgRNA2 occurs, amino acid 797 of EGFR, cysteine, is changed to serine. As a result of the experiment, while only 10% of 15C and 13C were substituted with G by C797S sgRNA1 and divalent N-UNG in an only blastidine-treated group, it was confirmed that parts in which C is changed to G are increased 50% or 80% in an osimertinib-treated group (see FIG. 30).

### [Example 5]

### Preparation of transformed cells by introduction of EGFR sgRNA library and drug resistance mutant screening

### Example 5-1: Design and synthesis of EGFR sgRNA library

A total of 1803 sgRNAs from 27 exons of an epidermal growth factor receptor (EGFR) gene were designed using a CRISPR RGEN tool (www.rgenome.net). Twist Bioscience was commissioned for synthesis after adding CACCG to the 5' terminus in the forward oligo sequence of the designed 1803 sgRNA oligo pools, and adding AAAC to the 5' terminus and C to the 3' terminus in the reverse oligo sequence thereof.

### Example 5-2: Preparation of EGFR seRNA library plasmids

The synthesized EGFR sgRNA oligo pools were reacted at 95 °C for 5 minutes, and annealed by gradually lowering a temperature until 25 °C. Afterward, the EGFR sgRNA oligo pools and a PiggyBac transposon backbone vector cleaved with a Bsa1 restriction enzyme were ligated by T4 ligase. The ligated reaction solution was inserted into Endura^{™} DUOs electrocompetent cells (Lucigen, Cat no. 60242-2) by electroporation. The *E. coli* cells transformed as such were applied evenly on an LB medium supplemented with ampicillin, and incubated at 37 °C overnight. EGFR sgRNA library plasmids were obtained from *E. coli* colonies using NuceloBond Xtra Midi EF (Macherey-Nagel, cat No.740420.50).

### Example 5-3: Cell culture

PC9 cells were incubated in Rosewell Park Memorial Institute 1640 (RPMI 1640, Welgene) supplemented with 10% FBS and 1% antibiotic in 5% CO₂ at 37 °C.

### Example 5-4: Preparation of transformed cells using PiggyBac transposon

Cells enabling EGFR sgRNA expression were prepared by applying a gene delivery system, that is, a PiggyBac transposon, to the PC9 cells. Before transformation, the PC9 cells were dispensed in a T175 flask at a density of 4 × 10⁶ cells per flask. Afterward, a PiggyBac transposon vector and a transposase expression vector were transfected in a 3 mL Opti-MEM medium in a ratio of 1:5 using 40 uL of Lipofectamine^{™} 2000 (Thermo Fisher Scientific, 11668019). The next day, the cells were treated with 2 µg/mL of puromycin and incubated for 7 days. A stabilized cell line was obtained through sufficient antibiotic subculture.

### Example 5-5: Transfection of single base substitution CRISPR protein and screening of drug resistance mutants

About 18 to 24 hours before transfection using Lipofectamine^{™} 2000 (Thermo Fisher Scientific, 11668019), 4×10⁶ of the transformed PC9 cells were dispensed in a T175 flask. Afterward, 20 µg N-UNG was transfected. Three days after transfection, the cells were treated with 4 µg/mL of blasticidin as an antibiotic and incubated for 7 days. When stabilized cells were obtained by sufficient antibiotic culture, 4×10⁶ of the cells were dispensed in a T175 flask. Afterward, the cells were incubated with a 100 nM non-small cell lung cancer therapeutic agent, osimertinib (Selleckchem, S5078) for 20 days, thereby obtaining resistant mutant cells.

### Example 5-6: Deep sequencing

On-target and off-target sites were PCR-amplified to a size of 200 to 300 bp using HiPi Plus DNA polymerase (Elpis-Bio). A PCR product obtained by the above method was sequenced using a MiSeq (Illumina) device, and the analysis of the resulting 1803 EGFR sgRNA sequences was commissioned.

### Example 5-7: Experimental results

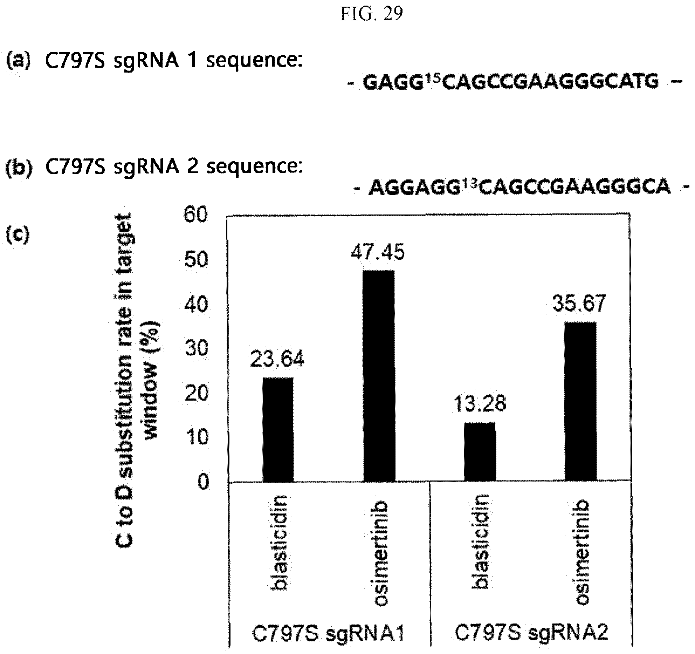

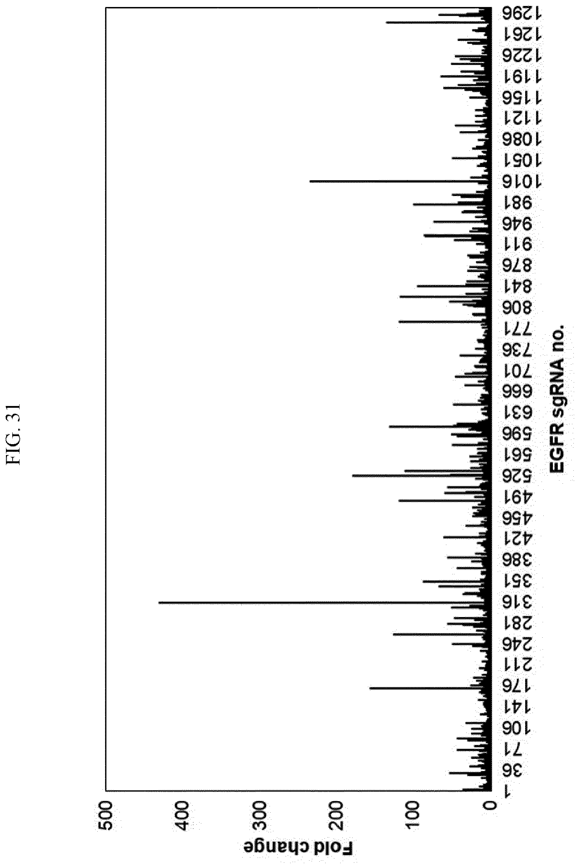

Cytosine in sgRNA was randomly substituted with N-UNG in the PC9 cells expressing EGFR sgRNA, and then the cells were incubated in an osimertinib-supplemented medium, followed by obtaining a result of analyzing viable cells (see FIGS. 29 and 30). FIG. 31 shows a result of analyzing viable cells by performing random substitution of cytosine in sgRNA with N-UNG in the PC9 cells capable of expressing EGFR sgRNA and incubating the cells in an osimertinib-supplemented medium.

## Claims

1. A fusion protein for single base substitution or a nucleic acid encoding thereof comprising,
(a) a CRISPR enzyme or variant thereof;
(b) a deaminase; and
(c) a DNA glycosylase or variant thereof,
wherein, the fusion protein for single base substitution is capable of inducing the substitution of a cytosine or an adenine with any base, and wherein the cytosine or the adenine is included in one or more nucleotide in a target nucleic acid sequence.

2. The fusion protein for single base substitution or the nucleic acid encoding thereof of claim 1,
wherein the fusion protein for single base substitution has any one component selected from
(i) N terminus-[CRISPR enzyme]-[deaminase]-[DNA glycosylase]-C terminus;
(ii) N terminus-[CRISPR enzyme]-[DNA glycosylase]-[deaminase]-C terminus;
(iii) N terminus-[deaminase]-[CRISPR enzyme]-[DNA glycosylase]-C terminus;
(iv) N terminus-[deaminase]-[DNA glycosylase]-[CRISPR enzyme]-C terminus;
(v) N terminus-[DNA glycosylase]-[CRISPR enzyme]-[deaminase]-C terminus; and
(vi) N terminus-[DNA glycosylase]-[deaminase]-[CRISPR enzyme]-C terminus.

3. The fusion protein for single base substitution or the nucleic acid encoding thereof of claim 1,
wherein the deaminase is a cytidine deaminase, and the DNA glycosylase is an uracil-DNA glycosylase or variant thereof, and
wherein, the fusion protein for single base substitution is capable of inducing the substitution of the cytosine with any base, and wherein the cytosine is included in one or more nucleotide in the target nucleic acid sequence.

4. The fusion protein for single base substitution or the nucleic acid encoding thereof of claim 3,
wherein the cytidine deaminase is any one of APOBEC, activation-induced cytidine deaminase (AID), and variant thereof.

5. The fusion protein for single base substitution or the nucleic acid encoding thereof of claim 1,
wherein the deaminase is an adenosine deaminase, and the DNA glycosylase is an alkyladenine DNA glycosylase or variant thereof,
wherein the fusion protein for single base substitution is capable of inducing the substitution of the adenine with any base, wherein the adenine is included in one or more nucleotide in the target nucleic acid sequence.

6. The fusion protein for single base substitution or the nucleic acid encoding thereof of claim 5,
wherein the adenosine deaminase is any one of TadA, Tad2p, ADA, ADA1, ADA2, ADAR2, ADAT2, ADAT3, and variant thereof.

7. The fusion protein for single base substitution or the nucleic acid encoding thereof of claim 1,
wherein the fusion protein for single base substitution further comprises one or more nuclear localization sequence (NLS).

8. The fusion protein for single base substitution or the nucleic acid encoding thereof of claim 1,
wherein the CRISPR enzyme or variant thereof comprises one or more selected from the group consisting of *Streptococcus pyogenes-drived* Cas9 protein, *Campylobacter jejuni-*drived Cas9 protein, *Streptococcus thermophilus-drived* Cas9 protein, *Streptococcus aureus-*drived Cas9 protein, *Neisseria meningitidis-drived* Cas9 protein, and Cpf1 protein.

9. The fusion protein for single base substitution or the nucleic acid encoding thereof of claim 8,
wherein the variant of CRISPR enzyme is **characterized in that** any one of a RuvC domain and a HNH is inactivated.

10. The fusion protein for single base substitution or the nucleic acid encoding thereof of claim 9,
wherein, the variant of CRISPR enzyme is nickase.

11. The fusion protein for single base substitution or the nucleic acid encoding thereof of claim 1,
wherein the fusion protein for single base substitution comprises a linking moiety which is interposed between one selected from (a), (b), and (c), and the other one selected from (a), (b), and (c).

12. A vector comprising a nucleic acid encoding a fusion protein for single base substitution of any one claims 1 to 11.

13. A complex for single base substitution comprising:
(a) a CRISPR enzyme or variant thereof;
(b) a deaminase;
(c) a DNA glycosylase; and
(d) two or more binding domain,
wherein the fusion protein for single base substitution is capable of inducing the substitution of a cytosine or an adenine with any base, and wherein the cytosine or the adenine is included in one or more nucleotide in a target nucleic acid sequence.

14. The complex for single base substitution of claim 13,
wherein each of the CRISPR enzyme, the deaminase, the DNA glycosylase is linked to one or more binding domain,
wherein the CRISPR enzyme, the deaminase, the DNA glycosylase form the complex through the interaction between the binding domains.

15. The complex for single base substitution of claim 14,
wherein any one of the CRISPR enzyme, the deaminase, and the DNA glycosylase is linked to a first binding domain and a second binding domain,
wherein, the first binding domain and a binding domain of another component is an interacting pair, and the second binding domain and a binding domain of the other component is an interacting pair,
wherein the complex is formed by the pairs.

16. The complex for single base substitution of claim 13,
wherein the deaminase is a cytidine deaminase, and
wherein the DNA glycosylase is an Uracil-DNA glycosylase or variant thereof,
wherein the fusion protein for single base substitution is capable of inducing the substitution of the cytosine with any base, and wherein the cytosine is included in one or more nucleotide in a target nucleic acid sequence.

17. The complex for single base substitution of claim 16,
wherein the cytidine deaminase is any one of APOBEC, activation-induced cytidine deaminase (AID), and variant thereof.

18. The complex for single base substitution of claim 13,
wherein the deaminase is a adenosine deaminase, and
wherein the DNA glycosylase is an Alkyladenine DNA glycosylase or variant thereof,
wherein the fusion protein for single base substitution is capable of inducing the substitution of the adenine with any base, and wherein the adenine is included in one or more nucleotide in a target nucleic acid sequence.

19. The complex for single base substitution of claim 18,
wherein the adenosine deaminase is any one of TadA, Tad2p, ADA, ADA1, ADA2, ADAR2, ADAT2, ADAT3, and variant thereof.

20. The complex for single base substitution of claim 13,
wherein the complex for single base substitution comprises:
(i) a first fusion protein comprising two components selected from the CRISPR enzyme, the deaminase, and the DNA glycosylase, and a first binding domain, and
(ii) a second fusion protein comprising one component selected from the CRISPR enzyme, the deaminase, and the DNA glycosylase, which is not selected in (i), and a second binding domain,
wherein the first binding domain and the second binding domain are interacting pair, and
wherein the complex is formed by the pair.

21. The complex for single base substitution of claim 20,
wherein the complex for single base substitution comprises:
(i) the first fusion protein comprising the deaminase, the DNA glycosylase, and the first binding domain, and
(ii) the second fusion protein comprising the CRISPR enzyme and the second binding domain.

22. The complex for single base substitution of claim 14,
wherein the binding domain is any one of a FRB domain, a FKBP dimerization domain, an intein, an ERT domains, a VPR domain, a GCN4 peptide, and a single chain variable fragment (scFv), or any one of a domain forming a heterodimer.

23. The complex for single base substitution of claim 15 or 20,
wherein the pair is any one selected from the following:
(i) a FRB and a FKBP dimerization domains;
(ii) a first intein and a second intein;
(iii) an ERT and a VPR domains;
(iv) a GCN4 peptide and a single chain variable fragment (scFv); and
(v) a first domain and a second domain forming a heterodimer.

24. The complex for single base substitution of claim 23,
wherein the pair is the GCN4 peptide and the single chain variable fragment (scFv).

25. The complex for single base substitution of claim 21,
wherein the first binding domain is a single chain variable fragment (scFv),
wherein the second fusion protein further comprises one or more a binding domain, wherein the binding domain which is further comprised in the second fusion protein is a GCN4 peptide, and
wherein two or more first fusion proteins form the complex, through interaction with any one of the GCN4 peptide.

26. A composition for single base substitution comprising,
(a) a guide RNA or a nucleic acid encoding thereof, and
(b) i) a fusion protein for single base substitution or a nucleic acid encoding thereof of claim 1, or ii) a complex for single base substitution of claim 13,
wherein, the guide RNA is complementarily binding to a target nucleic acid sequence,
wherein the target nucleic acid sequence bound to the guide RNA is 15 to 25bp,
wherein the fusion protein for single base substitution or the complex for single base substitution is capable of inducing the substitution of a cytosine or an adenine with any base, wherein the cytosine or the adenine is included in one or more nucleotide in a target region.

27. The composition for single base substitution of claim 26,
wherein the composition for single base substitution comprises one or more vector.

28. A method for single base substitution, the method comprising:
Contacting (i) and (ii) to a target region comprising a target nucleic acid sequence *in vitro* or *ex vivo,*
(i) a guide RNA,
(ii) a fusion protein for single base substitution of the claim 1, or a complex for single base substitution of the claim 12,
wherein, the guide RNA is complementarily binding to a target nucleic acid sequence,
wherein the target nucleic acid sequence bound to the guide RNA is 15 to 25bp,
wherein the fusion protein for single base substitution or the complex for single base substitution is capable of inducing the substitution of a cytosine or an adenine with any base, wherein the cytosine or the adenine is included in one or more nucleotide in a target region.

29. A method for SNP screening of a target gene, the method comprising:
inducing SNP artificially on the target gene, by introducing a composition for single base substitution of the claim 26 into a cell comprising the target gene;
selecting a cell comprising a desired SNP; and
obtaining an information on the desired SNP by analyzing the selected cell,
wherein the desired SNP is related to a structure or a function of a protein expressed from the target gene.

30. The method for SNP screening of the target gene of claim 29,
wherein the composition for single base substitution is introduced by one or more methods selected from the group consisting of an electroporation, a liposome, a plasmid, a viral vector, a nanoparticle, and a protein translocation domain (PTD) fusion protein method.

31. A method for screening a drug resistance mutation, the method comprising:
inducing SNP artificially on a target gene, by introducing a composition for single base substitution of claim 26 into one or more cells comprising the target gene;
treating a specific drug to the cell;
selecting a surviving cell; and
obtaining an information on a desired SNP by analyzing the selected cell,
wherein the desired SNP is related to a structure or a function of protein expressed from the target gene.

32. A method for obtaining an information on an Osimertinib resistance SNP, the method comprising:
a) introducing a protein for single base substitution or a nucleic acid encoding thereof, and any one or more guide RNA in guide RNA library or a nucleic acid encoding thereof into a cell comprising a target nucleic acid sequence;
b) treating the osimertinib to the cell of a);
c) isolating a surviving cell; and
d) obtaining an information on a desired SNP from the isolated cells,
wherein the desired SNP is related to a structure or a function of EGFR expressed from EGFR gene including the target nucleic acid sequence.

33. The method for screening SNP of the target gene of claim 31,
wherein the composition for single base substitution is introduced by one or more methods selected from the group consisting of an electroporation, a liposome, a plasmid, a viral vector, a nanoparticle, and a protein translocation domain (PTD) fusion protein method.


<110>     TOOLGEN INCORPORATED

<120>     SINGLE BASE EDITING PROTEINS AND COMPOSITION COMPRISING THE SAME

<130>     OPP19-003-PCT

<150>     US 62/851,372
<151>     2019-05-22

<150>     US 62/898,094
<151>     2019-09-10

<160>     92

<170>     KoPatentIn 3.0

<210>     1
<211>     20
<212>     DNA
<213>     homo sapiens

<400>     1
aagaagaaga cgagcctgag                                                   20


<210>     2
<211>     23
<212>     DNA
<213>     homo sapiens

<400>     2
gaacacaaag catagactgc ggg                                               23


<210>     3
<211>     23
<212>     DNA
<213>     homo sapiens

<400>     3
gagtatgagg catagactgc agg                                               23


<210>     4
<211>     23
<212>     DNA
<213>     homo sapiens

<400>     4
gtcaagaaag cagagactgc cgg                                               23


<210>     5
<211>     23
<212>     DNA
<213>     homo sapiens

<400>     5
gagcaaagac aatagactgt agg                                               23


<210>     6
<211>     23

<212>	DNA
<213>	homo sapiens

<400>	6
gatgagataa tgatgagtca ggg	23


<210>	7
<211>	23
<212>	DNA
<213>	homo sapiens

<400>	7
ggattgaccc aggccagggc tgg	23


<210>	8
<211>	23
<212>	DNA
<213>	homo sapiens

<400>	8
gaatactaag catagactcc agg	23


<210>	9
<211>	23
<212>	DNA
<213>	homo sapiens

<400>	9
gtaaacaaag catagactga ggg	23


<210>	10
<211>	23
<212>	DNA
<213>	homo sapiens

<400>	10
gaagaccaag gatagactgc tgg	23


<210>	11
<211>	23
<212>	DNA
<213>	homo sapiens

<400>	11
gaacataaag aatagaatga tgg	23


<210>	12
<211>	23
<212>	DNA
<213>	homo sapiens

<400>	12
ggacaggcag catagactgt ggg	23


<210>	13
<211>	23

<212>    DNA
<213>    homo sapiens

<400>    13
gtagaaaaag tatagactgc agg                                               23


<210>    14
<211>    23
<212>    DNA
<213>    homo sapiens

<400>    14
gaagatagag aatagactgc tgg                                               23


<210>    15
<211>    23
<212>    DNA
<213>    homo sapiens

<400>    15
ggctaaagac catagactgt ggg                                               23


<210>    16
<211>    23
<212>    DNA
<213>    homo sapiens

<400>    16
gggaataaat catagaatcc tgg                                               23


<210>    17
<211>    14
<212>    DNA
<213>    homo sapiens

<400>    17
kraatkkaga kkkk                                                         14


<210>    18
<211>    23
<212>    DNA
<213>    homo sapiens

<400>    18
gacaaagagg aagagagacg ggg                                               23


<210>    19
<211>    23
<212>    DNA
<213>    homo sapiens

<400>    19
acacacacac ttagaatctg tgg                                               23


<210>    20
<211>    23

<212>    DNA
<213>    homo sapiens

<400>    20
cacacacact tagaatctgt ggg                                                23


<210>    21
<211>    19
<212>    DNA
<213>    homo sapiens

<400>    21
gtacgggaag ccgacggag                                                     19


<210>    22
<211>    19
<212>    DNA
<213>    homo sapiens

<400>    22
acgggaagcc gacggagga                                                     19


<210>    23
<211>    7
<212>    PRT
<213>    Artificial Sequence

<220>
<223>    NLS sequence derived from SV40 virus


<400>    23
Pro Lys Lys Lys Arg Lys Val
1               5


<210>    24
<211>    16
<212>    PRT
<213>    Artificial Sequence

<220>
<223>    NLS sequence derived from nucleoplasmin


<400>    24
Lys Arg Pro Ala Ala Thr Lys Lys Ala Gly Gln Ala Lys Lys Lys Lys
1               5                   10                  15



<210>    25
<211>    9
<212>    PRT
<213>    Artificial Sequence

<220>
<223>    bipartite NLS sequence derived from c-myc

<400>    25
Pro Ala Ala Lys Arg Val Lys Leu Asp
1               5

<210>    26
<211>    11
<212>    PRT
<213>    Artificial Sequence

<220>
<223>    bipartite NLS of nucleoplasmin

<400>    26
Arg Gln Arg Arg Asn Glu Leu Lys Arg Ser Pro
1               5                   10

<210>    27
<211>    38
<212>    PRT
<213>    Artificial Sequence

<220>
<223>    hRNPA1 M9 NLS

<400>    27
Asn Gln Ser Ser Asn Phe Gly Pro Met Lys Gly Gly Asn Phe Gly Gly
1               5                   10                  15

Arg Ser Ser Gly Pro Tyr Gly Gly Gly Gly Gln Tyr Phe Ala Lys Pro
            20                  25                  30

Arg Asn Gln Gly Gly Tyr
        35

<210>    28
<211>    42
<212>    PRT
<213>    Artificial Sequence

<220>
<223>    importin alpha IBB domain sequence

<400>    28
Arg Met Arg Ile Glx Phe Lys Asn Lys Gly Lys Asp Thr Ala Glu Leu
1               5                   10                  15

Arg Arg Arg Arg Val Glu Val Ser Val Glu Leu Arg Lys Ala Lys Lys
            20                  25                  30

Asp Glu Gln Ile Leu Lys Arg Arg Asn Val
        35                  40

<210>    29
<211>    8
<212>    PRT
<213>    Artificial Sequence

<220>
<223>    myoma T protein sequence


<400>    29
Val Ser Arg Lys Arg Pro Arg Pro
1               5


<210>    30
<211>    8
<212>    PRT
<213>    Artificial Sequence

<220>
<223>    myoma T protein sequence


<400>    30
Pro Pro Lys Lys Ala Arg Glu Asp
1               5


<210>    31
<211>    7
<212>    PRT
<213>    Artificial Sequence

<220>
<223>    p53 sequence of human


<400>    31
Pro Pro Lys Lys Lys Pro Leu
1               5


<210>    32
<211>    12
<212>    PRT
<213>    Artificial Sequence

<220>
<223>    c-abl IV sequence of mouse


<400>    32
Ser Ala Leu Ile Lys Lys Lys Lys Lys Met Ala Pro
1               5                   10


<210>    33
<211>    5
<212>    PRT
<213>    Artificial Sequence

<220>
<223>    NS1 sequence of influenza virus


<400>    33
Asp Arg Leu Arg Arg

1               5


<210>   34
<211>   7
<212>   PRT
<213>   Artificial Sequence

<220>
<223>   NS1 sequence of influenza virus


<400>   34
Pro Lys Gln Lys Lys Arg Lys
1               5


<210>   35
<211>   10
<212>   PRT
<213>   Artificial Sequence

<220>
<223>   hepatitis virus


<400>   35
Arg Lys Leu Lys Lys Lys Ile Lys Lys Leu
1               5                   10


<210>   36
<211>   10
<212>   PRT
<213>   Artificial Sequence

<220>
<223>   sequence of Mx1 protein  of mouse


<400>   36
Arg Glu Lys Lys Lys Phe Leu Lys Arg Arg
1               5                   10


<210>   37
<211>   20
<212>   PRT
<213>   Artificial Sequence

<220>
<223>   ADP-ribose of human


<400>   37
Lys Arg Lys Gly Asp Glu Val Asp Gly Val Asp Glu Val Ala Lys Lys
1               5                   10                  15

Lys Ser Lys Lys
            20


<210>   38

<211>    17
<212>    PRT
<213>    homo sapiens

<400>    38
Arg Lys Cys Leu Gln Ala Gly Met Asn Leu Glu Ala Arg Lys Thr Lys
1               5                   10                  15

Lys

<210>    39
<211>    20
<212>    DNA
<213>    Artificial Sequence

<220>
<223>    GX19

<400>    39
gagtccgagc agaagaagaa                                                20

<210>    40
<211>    20
<212>    DNA
<213>    Artificial Sequence

<220>
<223>    GX20

<400>    40
tgcccctccc tccctggccc                                                20

<210>    41
<211>    20
<212>    DNA
<213>    Artificial Sequence

<220>
<223>    Nureki sgRNA 1

<400>    41
gaggacaaag tacaaacggc                                                20

<210>    42
<211>    20
<212>    DNA
<213>    Artificial Sequence

<220>
<223>    Nureki sgRNA 2

<400>    42
gggctcccat cacatcaacc                                                20

<210>    43
<211>    20
<212>    DNA
<213>    Artificial Sequence

<220>
<223>    Nureki sgRNA 3


<400>    43
ggccccagtg gctgctctgg                                                    20


<210>    44
<211>    20
<212>    DNA
<213>    Artificial Sequence

<220>
<223>    Nureki sgRNA 4


<400>    44
gctttaccca gttctctggg                                                    20


<210>    45
<211>    77
<212>    RNA
<213>    Artificial Sequence

<220>
<223>    sequence for forming a complex with spCas9 protein


<400>    45
guuuuagucc cugaaaaggg acuaaaauaa agaguuugcg ggacucugcg ggguuacaau        60

ccccuaaaac cgcuuuu                                                       77


<210>    46
<211>    76
<212>    RNA
<213>    Artificial Sequence

<220>
<223>    sequence for forming a complex with cjCas9 protein


<400>    46
guuuuagagc uagaaauagc aaguuaaaau aaggcuaguc cguuaucaac uugaaaaagu        60

ggcaccgagu cggugc                                                        76


<210>    47
<211>    8
<212>    PRT
<213>    Artificial Sequence

<220>

<223>     PAM of cjCas9 protein

<400>     47
Asn Asn Asn Asn Arg Tyr Ala Cys
1               5

<210>     48
<211>     20
<212>     RNA
<213>     Artificial Sequence

<220>
<223>     Sp20-viHBV-B

<400>     48
   guaacacgag caggggtccu                                                    20

<210>     49
<211>     20
<212>     RNA
<213>     Artificial Sequence

<220>
<223>     Sp20-viHBV-B

<400>     49
ccccgccugu aacacgagca                                                    20

<210>     50
<211>     20
<212>     RNA
<213>     Artificial Sequence

<220>
<223>     Sp20-viHBV-B

<400>     50
accccgccug uaacacgagc                                                    20

<210>     51
<211>     20
<212>     RNA
<213>     Artificial Sequence

<220>
<223>     Sp20-viHBV-B

<400>     51
aggaccccug cucguguuac                                                    20

<210>     52
<211>     20
<212>     RNA

<213>    Artificial Sequence

<220>
<223>    Sp20-viHBV-B


<400>    52
accccugcuc guguuacagg                                                    20


<210>    53
<211>    20
<212>    RNA
<213>    Artificial Sequence

<220>
<223>    Sp20-viHBV-B


<400>    53
caccacgagu cuagacucug                                                    20


<210>    54
<211>    20
<212>    RNA
<213>    Artificial Sequence

<220>
<223>    Sp20-viHBV-B


<400>    54
ggacuucucu caauuuucua                                                    20


<210>    55
<211>    20
<212>    RNA
<213>    Artificial Sequence

<220>
<223>    Sp20-viHBV-B


<400>    55
ccuacgaacc acugaacaaa                                                    20


<210>    56
<211>    20
<212>    RNA
<213>    Artificial Sequence

<220>
<223>    Sp20-viHBV-B


<400>    56
ccauuuguuc agugguucgu                                                    20


<210>    57

<211>    20
<212>    RNA
<213>    Artificial Sequence

<220>
<223>    Sp20-viHBV-B


<400>    57
cauuuguuca gugguucgua                                                    20


<210>    58
<211>    20
<212>    RNA
<213>    Artificial Sequence

<220>
<223>    Sp20-viHBV-B


<400>    58
ggguugcguc agcaaacacu                                                    20


<210>    59
<211>    20
<212>    RNA
<213>    Artificial Sequence

<220>
<223>    Sp20-viHBV-B


<400>    59
   uuugcugacg caacccccac                                                     20


<210>    60
<211>    20
<212>    RNA
<213>    Artificial Sequence

<220>
<223>    Sp20-viHBV-B


<400>    60
uccgcaguau ggaucggcag                                                    20


<210>    61
<211>    20
<212>    RNA
<213>    Artificial Sequence

<220>
<223>    Sp20-viHBV-B


<400>    61
aggaguuccg caguauggau                                                    20

<210>    62
<211>    20
<212>    RNA
<213>    Artificial Sequence

<220>
<223>    Sp20-viHBV-B


<400>    62
uccucugccg auccauacug                                                    20


<210>    63
<211>    20
<212>    RNA
<213>    Artificial Sequence

<220>
<223>    Sp20-viHBV-B


<400>    63
cgucccgcgc aggauccagu                                                    20


<210>    64
<211>    20
<212>    RNA
<213>    Artificial Sequence

<220>
<223>    Sp20-viHBV-B


<400>    64
ccgcgggauu cagcgccgac                                                    20


<210>    65
<211>    20
<212>    RNA
<213>    Artificial Sequence

<220>
<223>    Sp20-viHBV-B


<400>    65
uccgcgggau ucagcgccga                                                    20


<210>    66
<211>    20
<212>    RNA
<213>    Artificial Sequence

<220>
<223>    Sp20-viHBV-B


<400>    66

cccgucggcg cugaaucccg                                                    20

<210>    67
<211>    20
<212>    RNA
<213>    Artificial Sequence

<220>
<223>    Sp20-viHBV-B

<400>    67
guaaagagag gugcgccccg                                                    20

<210>    68
<211>    20
<212>    RNA
<213>    Artificial Sequence

<220>
<223>    Sp20-viHBV-B

<400>    68
ggggcgcacc ucucuuuacg                                                    20

<210>    69
<211>    20
<212>    RNA
<213>    Artificial Sequence

<220>
<223>    Sp20-viHBV-B

<400>    69
gaagcgaagu gcacacgguc                                                    20

<210>    70
<211>    20
<212>    RNA
<213>    Artificial Sequence

<220>
<223>    Sp20-viHBV-B

<400>    70
ggucuccaug cgacgugcag                                                    20

<210>    71
<211>    20
<212>    RNA
<213>    Artificial Sequence

<220>
<223>    Sp20-viHBV-B

<400>    71
aaugucaacg accgaccuug                                                    20


<210>    72
<211>    20
<212>    RNA
<213>    Artificial Sequence

<220>
<223>    Sp20-viHBV-B


<400>    72
aggaggcugu aggcauaaau                                                    20


<210>    73
<211>    20
<212>    RNA
<213>    Artificial Sequence

<220>
<223>    Sp20-viHBV-B


<400>    73
cggaaguguu gauaagauag                                                    20


<210>    74
<211>    20
<212>    RNA
<213>    Artificial Sequence

<220>
<223>    Sp20-viHBV-B


<400>    74
ccggaagugu ugauaagaua                                                    20


<210>    75
<211>    20
<212>    RNA
<213>    Artificial Sequence

<220>
<223>    Sp20-viHBV-B


<400>    75
gcgagggagu ucuucuucua                                                    20


<210>    76
<211>    20
<212>    RNA
<213>    Artificial Sequence

<220>

<223>    Sp20-viHBV-B

<400>    76
gaccuucguc ugcgaggcga                                                    20

<210>    77
<211>    20
<212>    RNA
<213>    Artificial Sequence

<220>
<223>    Sp20-viHBV-B

<400>    77
gauugagacc uucgucugcg                                                    20

<210>    78
<211>    20
<212>    RNA
<213>    Artificial Sequence

<220>
<223>    Sp20-viHBV-B

<400>    78
cucccucgcc ucgcagacga                                                    20

<210>    79
<211>    20
<212>    RNA
<213>    Artificial Sequence

<220>
<223>    Sp20-viHBV-B

<400>    79
gauugagauc uucugcgacg                                                    20

<210>    80
<211>    20
<212>    RNA
<213>    Artificial Sequence

<220>
<223>    Sp20-viHBV-B

<400>    80
gucgcagaag aucucaaucu                                                    20

<210>    81
<211>    20
<212>    RNA
<213>    Artificial Sequence

<220>
<223>    Sp20-viHBV-B


<400>    81
ucgcagaaga ucucaaucuc                                                    20


<210>    82
<211>    22
<212>    RNA
<213>    Artificial Sequence

<220>
<223>    Sp20-viHBV-B


<400>    82
ugucaacaag aaaaaccccg cc                                                 22


<210>    83
<211>    22
<212>    RNA
<213>    Artificial Sequence

<220>
<223>    Sp20-viHBV-B


<400>    83
aagcccuacg aaccacugaa ca                                                 22


<210>    84
<211>    22
<212>    RNA
<213>    Artificial Sequence

<220>
<223>    Sp20-viHBV-B


<400>    84
uuaccaauuu ucuuuugucu uu                                                 22


<210>    85
<211>    22
<212>    RNA
<213>    Artificial Sequence

<220>
<223>    Sp20-viHBV-B


<400>    85
acgucccgcg caggauccag uu                                                 22


<210>    86
<211>    22

<212>    RNA
<213>    Artificial Sequence

<220>
<223>    Sp20-viHBV-B


<400>    86
gugcacacgg uccggcagau ga                                                22


<210>    87
<211>    22
<212>    RNA
<213>    Artificial Sequence

<220>
<223>    Sp20-viHBV-B


<400>    87
gugccuucuc aucugccgga cc                                                22


<210>    88
<211>    22
<212>    RNA
<213>    Artificial Sequence

<220>
<223>    Sp20-viHBV-B


<400>    88
cgacgugcag aggugaagcg aa                                                22


<210>    89
<211>    22
<212>    RNA
<213>    Artificial Sequence

<220>
<223>    Sp20-viHBV-B


<400>    89
ugcgacgugc agaggugaag cg                                                22


<210>    90
<211>    22
<212>    RNA
<213>    Artificial Sequence

<220>
<223>    Sp20-viHBV-B


<400>    90
gaccgugugc acuucgcuuc ac                                                22

<210>    91
<211>    22
<212>    RNA
<213>    Artificial Sequence

<220>
<223>    Sp20-viHBV-B

<400>    91
auguccaugc cccaaagcca cc                                                22

<210>    92
<211>    22
<212>    RNA
<213>    Artificial Sequence

<220>
<223>    Sp20-viHBV-B

<400>    92
gaccaccaaa ugccccuauc uu                                                22